# EUROPÄISCHE PATENTSCHRIFT

(11) **EP 2 038 370 B1**
(45) Veröffentlichungstag und Bekanntmachung des Hinweises auf die Patenterteilung: **25.04.2012**
(21) Anmeldenummer: 07764736.0
(22) Anmeldetag: 20.06.2007
(51) Int. Cl.: C09K 11/06, H05B 33/14

(54) **NEUE MATERIALIEN FÜR ORGANISCHE ELEKTROLUMINESZENZVORRICHTUNGEN**
NOVEL MATERIALS FOR ORGANIC ELECTROLUMINESCENT DEVICES
NOUVEAUX MATÉRIAUX DESTINÉS À DES DISPOSITIFS ÉLECTROLUMINESCENTS ORGANIQUES

(30) Priorität: 11.07.2006 DE 102006031990
(43) Veröffentlichungstag der Anmeldung: 25.03.2009
(73) Patentinhaber: Merck Patent GmbH, 64293 Darmstadt (DE)
(72) Erfinder: BUESING, Arne, 65929 Frankfurt am Main (DE); STOESSEL, Philipp, 60487 Frankfurt am Main (DE); HEIL, Holger, 64295 Darmstadt (DE)
(86) Internationale Anmeldenummer: PCT/EP2007/005413
(87) Internationale Veröffentlichungsnummer: WO 2008/006449

(56) Entgegenhaltungen:
- WO-A-2004/020372
- WO-A-2007/061218
- JP-A- 11 162 642
- T. HADIZAD, J. ZHANG, Z. WANG, T. GORJANC, C PY: "A general synthetic route to indenofluorene derivatives as new organic semiconductors" ORGANIC LETTERS, Bd. 7, Nr. 5, 2005, Seiten 795-797, XP002446382
- C. PY ET.AL.: "Hole mobility and electroluminescence properties of a dithiophene indenofluorene" J. VAC.SCI. TECHNOL. A, Bd. 24, Nr. 3, Mai 2006 (2006-05), Seiten 654-656, XP002446383

## Beschreibung

Die vorliegende Erfindung betrifft organische Halbleiter und deren Verwendung in organischen elektronischen Vorrichtungen.

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings zeigen diese Vorrichtungen noch erhebliche Probleme, die einer dringenden Verbesserung bedürfen:
1. Die üblicherweise verwendeten Verbindungen weisen kein ausreichend tiefes LUMO (niedrigstes unbesetztes Molekülorbital) auf. Verbindungen mit niedrigerem LUMO werden für leichtere Elektroneninjektion und damit für eine Verringerung der Betriebsspannung benötigt.
2. Die Farbkoordinaten vieler blauer Emitter sind noch nicht zufriedenstellend.
3. Die thermische Stabilität insbesondere blauer Dotanden ist nicht ausreichend.
4. Die Lebensdauer und die Effizienz blau emittierender organischer Elektrolumineszenzvorrichtungen sollte für hochwertige Anwendungen noch weiter gesteigert werden.

Für fluoreszierende OLEDs werden gemäß dem Stand der Technik vor allem kondensierte Aromaten, insbesondere Anthracen- oder Pyrenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis-(1-naphthyl)anthracen-Derivate für die Verwendung in OLEDs offenbart.

Weitere Anthracenderivate sind in WO 01/076323, in WO 01/021729, in WO 04/013073, in WO 04/018588, in WO 03/087023 oder in WO 04/018587 offenbart. Host-Materialien, basierend auf Arylsubstituierten Pyrenen und Chrysenen, werden in WO 04/016575 offenbart. Es ist für hochwertige Anwendungen notwendig, verbesserte Host-Materialien zur Verfügung zu haben.

Als Stand der Technik bei blau emittierenden Verbindungen kann die Verwendung von Arylvinylaminen genannt werden (z. B. WO 04/013073, WO 04/016575, WO 04/018587). Diese Verbindungen sind jedoch thermisch instabil und lassen sich nicht unzersetzt verdampfen, was einen hohen technischen Aufwand für die OLED-Herstellung erfordert und somit einen technischen Nachteil darstellt. Einen weiteren Nachteil stellt die Emissionsfarbe dieser Verbindungen dar: Während im Stand der Technik mit diesen Verbindungen tiefblaue Emission (CIE-y-Koordinaten im Bereich von 0.15-0.18) beschrieben wird, konnten diese Farbkoordinaten nicht in einfachen Vorrichtungen gemäß dem Stand der Technik reproduziert werden. Hier erhält man grünblaue Emission. Es ist nicht offensichtlich, wie mit diesen Verbindungen blaue Emission erzeugt werden kann. Es ist für hochwertige Anwendungen notwendig, verbesserte Emitter besonders im Bezug auf Device- und Sublimationsstabilität zur Verfügung zu haben.

In T. Hadizad et al., Org. Lett. 2005, 795-797, wird die Synthese von bestimmten Indenofluorenderivaten und ihre Verwendung als Funktionsmaterialien in OLEDs offenbart.

In phosphoreszierenden OLEDs wird als Matrixmaterial häufig 4,4'-Bis-(N-carbazolyl)biphenyl (CBP) verwendet. Die Nachteile sind kurze Lebensdauern der mit ihnen hergestellten Devices und häufig hohe Betriebsspannungen, die zu geringen Leistungseffizienzen führen. Außerdem weist CBP eine nicht ausreichend hohe Glasübergangstemperatur auf. Des Weiteren hat sich gezeigt, dass CBP für blau emittierende Elektrolumineszenzvorrichtungen ungeeignet ist, was in einer schlechten Effizienz resultiert. Außerdem ist der Aufbau der Devices mit CBP komplex, da zusätzlich eine Lochblockierschicht und eine Elektronentransportschicht verwendet werden müssen. Verbesserte Triplett-Matrixmaterialien, basierend auf Ketoverbindungen, sind in WO 04/093207 beschrieben, jedoch ergeben auch diese noch nicht mit allen Triplettemittern zufriedenstellende Resultate.

Als Elektronentransportverbindung in organischen Elektrolumineszenzvorrichtungen wird meist AlQ₃ (Aluminium-trishydroxychinolinat) verwendet (US 4539507). Dieses lässt sich nicht rückstandsfrei aufdampfen, da es sich bei der Sublimationstemperatur teilweise zersetzt, was insbesondere für Produktionsanlagen ein großes Problem darstellt. Ein weiterer Nachteil ist die starke Hygroskopie von AlQ₃, ebenso wie die niedrige Elektronenbeweglichkeit, was zu höheren Spannungen und damit zu einer niedrigeren Leistungseffizienz führt. Um Kurzschlüsse im Display zu vermeiden, würde man gern die Schichtdicke erhöhen; dies ist mit AlQ₃ wegen der geringen Ladungsträgerbeweglichkeit und der daraus resultierenden Spannungserhöhung nicht möglich. Als ungünstig erweist sich weiterhin die Eigenfarbe von AlQ₃ (im Feststoff gelb), die gerade bei blauen OLEDs durch Reabsorption und schwache Reemission zu Farbverschiebungen führen kann. Hier sind blaue OLEDs nur mit starken Effizienz- bzw. Farborteinbußen darstellbar.

Es besteht also weiterhin Bedarf an verbesserten Materialien, insbesondere emittierenden Verbindungen, vor allem blau emittierenden Verbindungen, aber auch Host-Materialien für fluoreszierende und phosphoreszierende Emitter, Lochtransportmaterialien und Elektronentransportmaterialien, die thermisch stabil sind, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen und die synthetisch einfach zugänglich sind.

Überraschend wurde gefunden, dass Verbindungen, in denen eine Phenylengruppe mit einer Naphthyl-, Anthryl- oder Phenanthrenylgruppe und mit einer Phenylgruppe verknüpft ist und in denen noch zusätzlich jeweils mindestens eine Brücke zwischen der Phenylengruppe und der Phenylgruppe und zwischen der Phenylengruppe und der Naphthyl- bzw. Anthryl- bzw. Phenanthrenylgruppe existiert, und heterocyclische Derivate dieser Verbindungen sich sehr gut für die Verwendung in organischen Elektrolumineszenzvorrichtungen eignen. Diese Verbindungen weisen eine hohe thermische Stabilität auf. Mit diesen Materialien ist weiterhin eine Steigerung der Effizienz und der Lebensdauer der organischen elektronischen Vorrichtung im Vergleich zu Materialien gemäß dem Stand der Technik möglich. Weiterhin sind diese Materialien sehr gut für die Verwendung in organischen elektronischen Vorrichtungen geeignet, da sie eine hohe Glasübergangstemperatur aufweisen. Diese Materialien und deren Verwendung in organischen elektronischen Vorrichtungen sind daher der Gegenstand der vorliegenden Erfindung.

Gegenstand der Erfindung sind Verbindungen gemäß den Formeln (1), (2), (4) und (5), 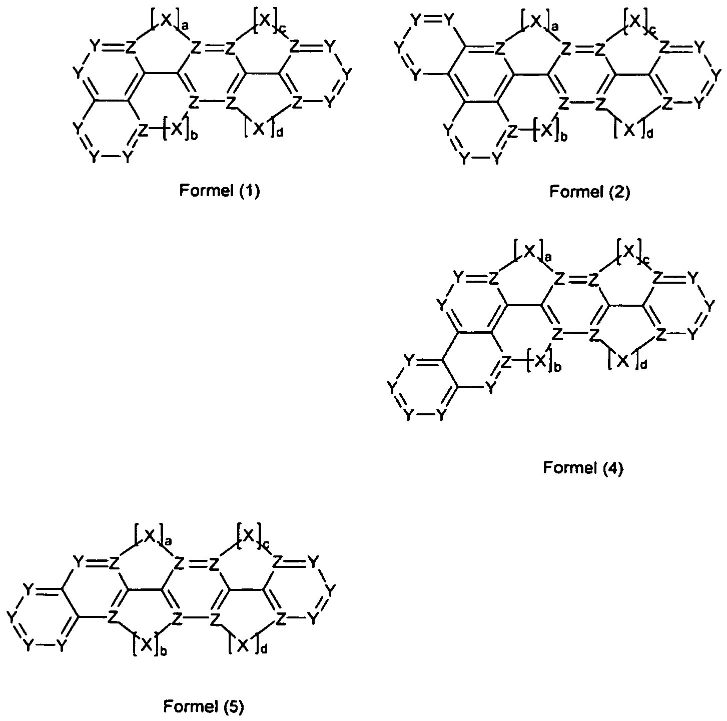 wobei für die Symbole und indizes gilt:
- Y: ist bei jedem Auftreten gleich oder verschieden CR¹;
- Z: ist gleich C, falls an die Gruppe Z eine Brücke X gebunden ist, und ist gleich Y, falls an die Gruppe Z keine Brücke X gebunden ist;
- X: ist bei jedem Auftreten gleich oder verschieden eine bivalente Brücke, ausgewählt aus C(R¹)₂, C=O, C=NR¹, O, S, S=O, SO₂ und N(R¹);
- R¹: ist bei jedem Auftreten gleich oder verschieden H, F, Br, C(=O)Ar, P(=O)Ar₂, CR²=CR²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²-, -C=C- oder -O- ersetzt sein können und wobei ein oder mehrere H-Atome durch F ersetzt sein können, oder eine Triarylamingruppe mit 18 bis 30 C-Atomen, die mit einem oder mehreren Resten R² substituiert sein kann, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei oder drei dieser Systeme,
und/oder mindestens ein Symbol R¹ steht für eine Gruppe N(Ar)₂ der Formel (29) oder (30), 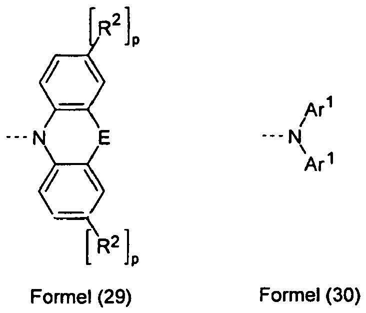 wobei R² die in Anspruch 1 aufgeführte Bedeutung hat und weiterhin gilt:
E steht für eine Einfachbindung, O, S, N(R²) oder C(R²)₂;
Ar¹ ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, welche jeweils mit einem oder mehreren Resten R² substituiert sein kann;
p ist bei jedem Auftreten gleich oder verschieden 0 oder 1;
- Ar: ist bei jedem Auftreten gleich oder verschieden ein aromatisches oder heteroaromatisches Ringsystem mit 5 bis 30 aromatischen Ringatomen, das mit einem oder mehreren nicht- aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
- R²: ist bei jedem Auftreten gleich oder verschieden H oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches aliphatisches oder aromatisches Ringsystem bilden;
- a, b, c, d: ist bei jedem Auftreten gleich oder verschieden 0 oder 1, mit der Maßgabe, dass a + b = 1 und c + d = 1 ist, wobei a = 0 bzw. b = 0 bzw. c = 0 bzw. d = 0 jeweils bedeutet, dass die entsprechende Brücke X nicht vorhanden ist; die Brücke X steht dann nicht für eine Einfachbindung.

Bevorzugt weisen die Verbindungen gemäß Formel (1), (2), (4) und (5) eine Glasübergangstemperatur T_{G} von größer als 70 °C auf, besonders bevorzugt größer als 100 °C, ganz besonders bevorzugt größer als 130 °C.

Unter benachbarten Resten R¹ bzw. R² im Sinne dieser Erfindung werden Reste verstanden, die entweder am selben Kohlenstoffatom bzw. am selben Heteroatom gebunden sind oder die an benachbarten Kohlenstoffatomen gebunden sind.

Eine Arylgruppe im Sinne dieser Erfindung enthält 6 bis 40 C-Atome; eine Heteroarylgruppe im Sinne dieser Erfindung enthält 2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen. Phenanthren, Pyren, Chrysen, Perylon, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugte Ausführungsformen der Strukturen gemäß Formel (1), (2), (4) und (5) sind die folgenden Strukturen 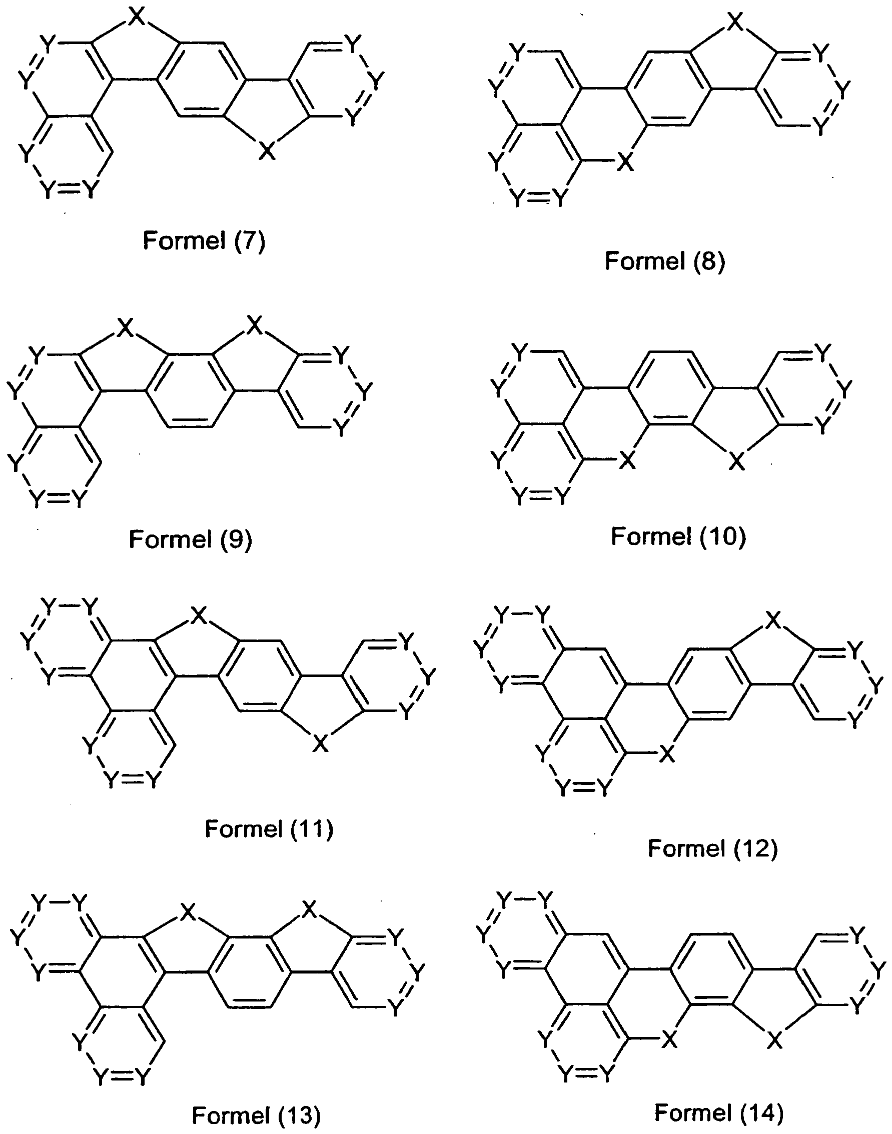 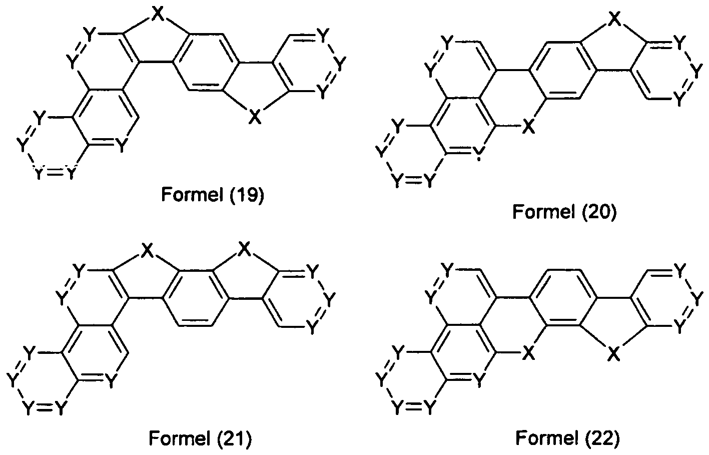 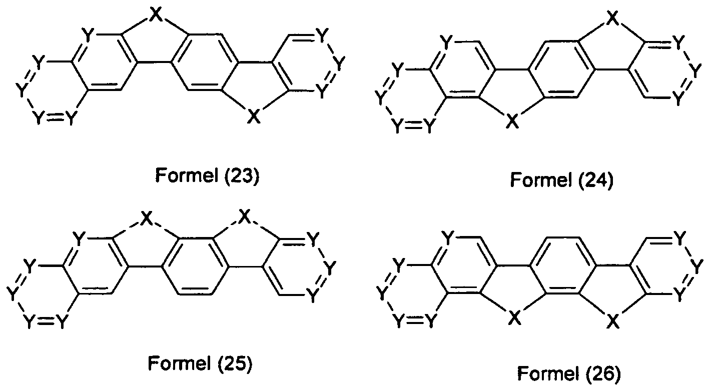 wobei die Symbole X und Y dieselbe Bedeutung haben, wie oben beschrieben.

In einer besonders bevorzugten Ausführungsform sind die Strukturen gemäß Formel (1), (2), (4) und (5) gewählt aus den folgenden Formeln 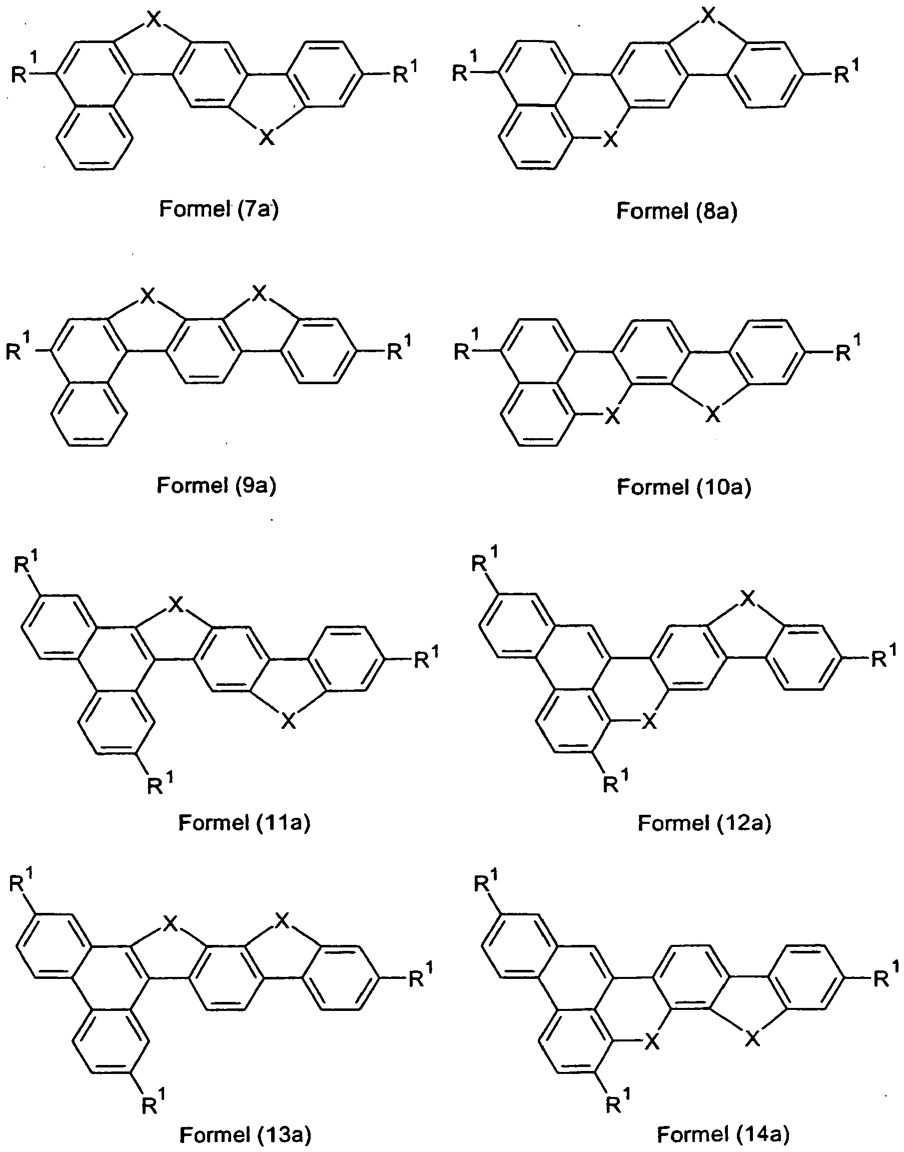 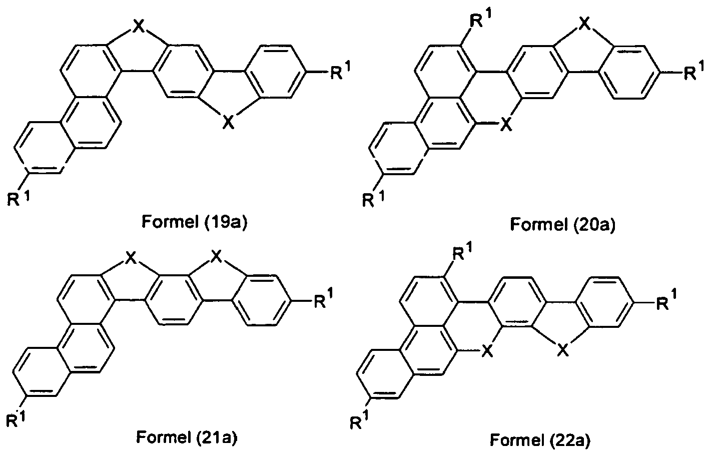 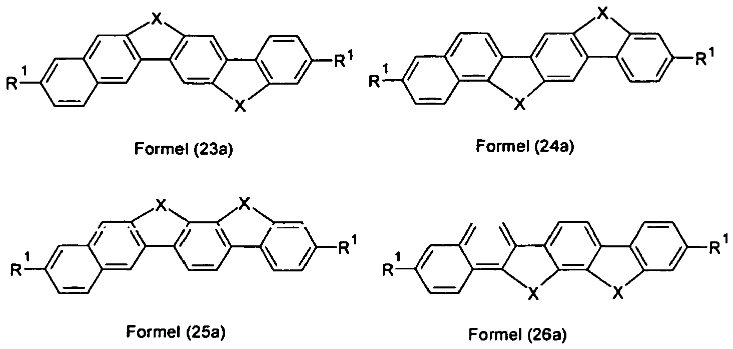 wobei die Symbole X und R¹ dieselbe Bedeutung haben, wie oben beschrieben.

Besonders bevorzugt ist in den Strukturen gemäß Formel (7a) bis (15a) und (19a) bis (26a) mindestens ein Rest R¹ ungleich Wasserstoff; besonders bevorzugt sind mindestens zwei Reste R¹ ungleich Wasserstoff. Dabei kann es auch bevorzugt sein, wenn die Reste R¹ verschieden sind. Wenn nur ein Rest R¹ vorhanden ist, ist es bevorzugt, wenn dieser an der kondensierten Arylgruppe des Systems gebunden ist. Er kann aber auch am nicht kondensierten Phenylring gebunden sein.

Besonders bevorzugte Reste R¹ sind gleich oder verschieden bei jedem Auftreten eine Gruppe NAr₂, wie unten aufgeführt, oder H, F, Br, C(=O)Ar, P(=O)Ar₂, Methyl, Ethyl, iso-Propyl, tert-Butyl, wobei ein oder mehrere H-Atome jeweils durch F ersetzt sein können, oder eine Triphenylamingruppe, die mit einem oder mehreren Resten R² substituiert sein kann, oder eine Arylgruppe mit 6 bis 14 C-Atomen oder eine Spirobifluorengruppe, die jeweils mit einem oder mehreren Resten R² substituiert sein kann, oder eine Kombination aus zwei dieser Systeme. Bei Verbindungen, die aus Lösung verarbeitet werden, sind auch lineare oder verzweigte Alkylketten mit bis zu 10 C-Atomen bevorzugt. Brom als Substituent ist vor allem für die Verwendung dieser Verbindung als Zwischenstufe zur Herstellung anderer erfindungsgemäßer Verbindungen bevorzugt.

Besonders bevorzugt steht Ar¹ gleich oder verschieden für Phenyl, 1-Naphthyl, 2-Naphthyl, 2-, 3- oder 4-Triphenylamin, 1- oder 2-Naphthyldiphenylamin, welches jeweils über die Naphthyl- oder die Phenylgruppe gebunden sein kann, oder 1- oder 2-Dinaphthylphenylamin, welches jeweils über die Naphthyl- oder die Phenylgruppe gebunden sein kann. Diese Gruppen können jeweils durch eine oder mehrere Alkylgruppen mit 1 bis 4 C-Atomen oder durch Fluor substituiert sein.

Bevorzugt sind weiterhin Verbindungen gemäß den obenstehenden Formeln, in denen die Symbole X bei jedem Auftreten gleich oder verschieden ausgewählt sind aus C(R¹)₂ oder N(R¹), insbesondere C(R¹)₂. Dabei sei hier nochmals explizit darauf hingewiesen, dass hier auch mehrere benachbarte Reste R¹ an der Gruppe X miteinander ein aromatisches oder aliphatisches Ringsystem bilden können. Wenn mehrere Reste R¹ an einer Gruppe C(R¹)₂ miteinander ein Ringsystem bilden, führt dies zu Spirostrukturen. Die Ausbildung derartiger Spirostrukturen durch Bildung von Ringsystemen zwischen zwei Gruppen R¹ an C(R¹)₂ ist eine weitere bevorzugte Ausführungsform der Erfindung. Dies gilt insbesondere, wenn R¹ für eine substituierte oder unsubstituierte Phenylgruppe steht und die beiden Phenylgruppen zusammen mit dem C-Atom der Brücke ein Ringsystem bilden.

Bevorzugte Reste R¹, die an die Brücken X gebunden sind, sind gleich oder verschieden und sind ausgewählt aus H, geradkettigen Alkylgruppen mit 1 bis 5 C-Atomen oder verzweigten Alkylgruppen mit 3 bis 5 C-Atomen, wobei eine oder mehrere nicht benachbarte CH₂-Gruppen durch -R²C=CR2 -, -C=C- oder -O- ersetzt sein können und wobei ein oder mehrere H-Atome durch F ersetzt sein können, oder Arylgruppen mit 6 bis 16 C-Atomen oder Heteroarylgruppen mit 2 bis 16 C-Atomen, die jeweils mit einem oder mehreren Resten R² substituiert sein können, oder eine Kombination aus zwei oder drei dieser Systeme; dabei können zwei der Reste R¹, die an dasselbe Brückenatom gebunden sind, auch miteinander ein Ringsystem bilden. Besonders bevorzugte Reste R¹, die an die Brücken X gebunden sind, sind gleich oder verschieden und sind ausgewählt aus Methyl, Ethyl, iso-Propyl, tert-Butyl, wobei jeweils ein oder mehrere H-Atome durch F ersetzt sein können, oder Arylgruppen mit 6 bis 14 C-Atomen, die mit einem oder mehreren Resten R² substituiert sein können, oder eine Kombination aus zwei dieser Systeme; dabei können zwei der Reste R¹, die an dasselbe Brückenatom gebunden sind, auch miteinander ein Ringsystem bilden. Bei Verbindungen, die aus Lösung verarbeitet werden, sind auch lineare oder verzweigte Alkylketten mit bis zu 10 C-Atomen bevorzugt.

Weiterhin bevorzugt sind symmetrisch substituierte Verbindungen, also Verbindungen, in denen die Substituenten R¹ in den Formeln (7a) bis (14a) und (19a) bis (26a) gleich gewählt.

Beispiele für bevorzugte erfindungsgemäße Verbindungen sind die im Folgenden abgebildeten Strukturen.

| | |
|---|---|
| 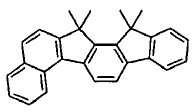 | 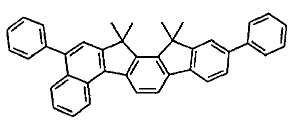 |
| (1) | (2) |
| 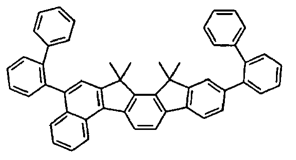 | 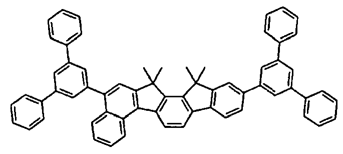 |
| (3) | (4) |
| 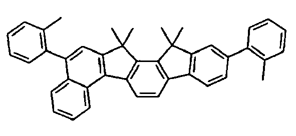 | 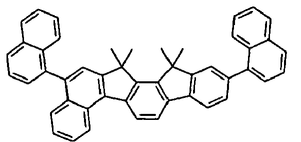 |
| (5) | (6) |
| 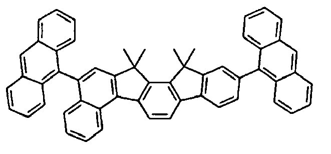 | 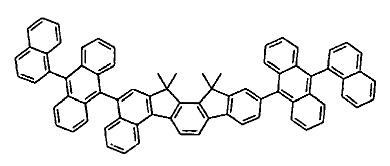 |
| (7) | (8) |
| 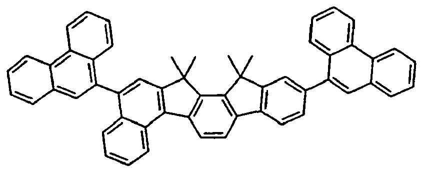 | 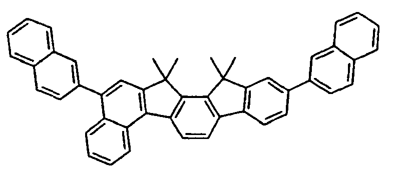 |
| (9) | (10) |
| 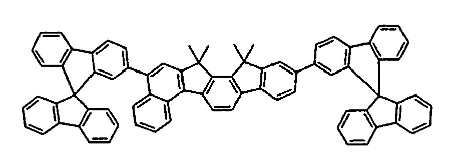 | 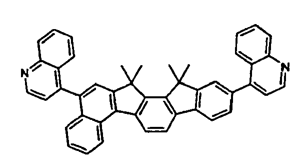 |
| (11) | (12) |
| 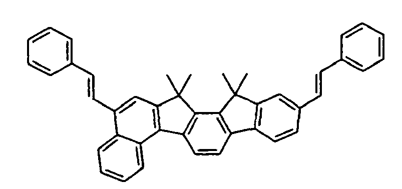 | 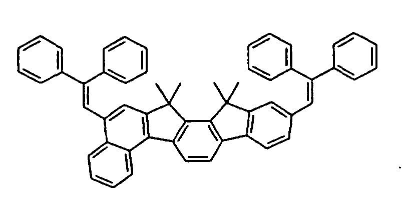 |
| (13) | (14) |
| 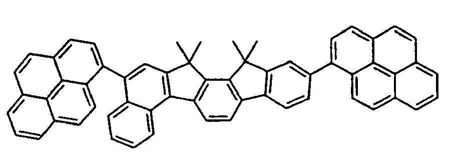 (13) | 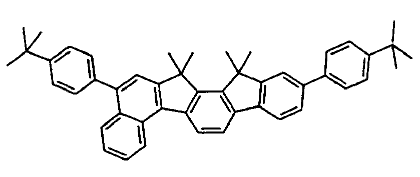 |
| (15) | (16) |
| 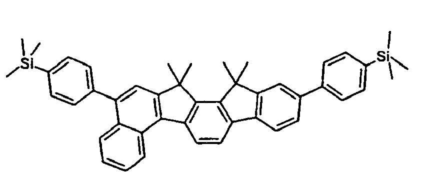 | 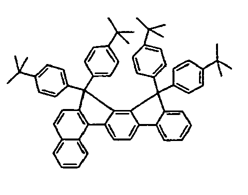 |
| (17) | (18) |
| 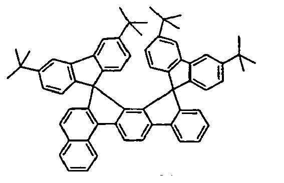 | 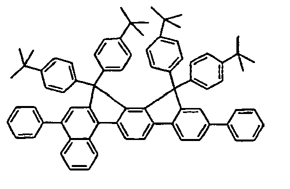 |
| (19) | (20) |
| 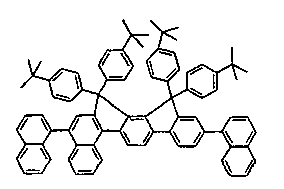 | 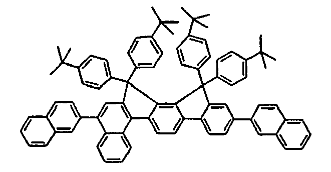 |
| (21) | (22) |
| 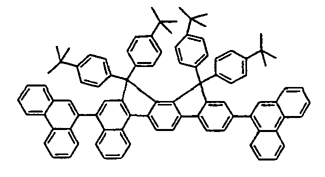 | 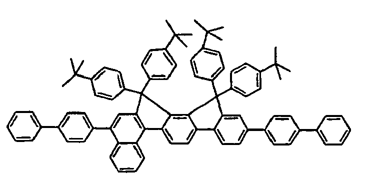 |
| (23) | (24) |
| 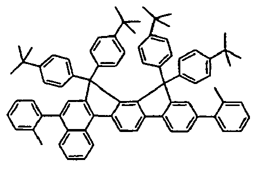 | 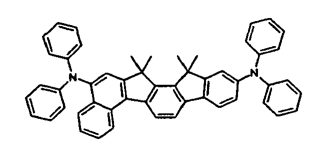 |
| (25) | (26) |
| 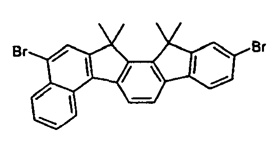 | 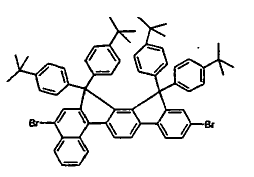 |
| (27) | (28) |
| 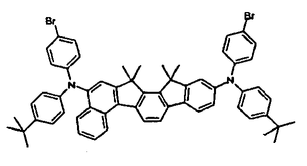 | 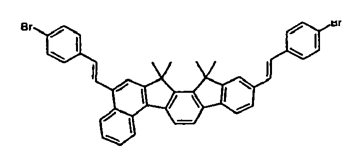 |
| (29) | (30) |
| 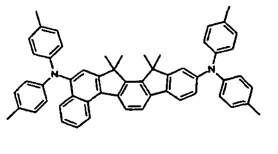 | 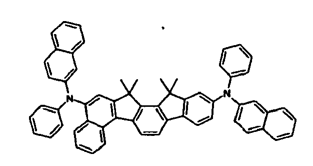 |
| (31) | (32) |
| 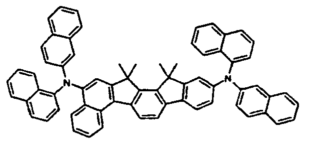 | 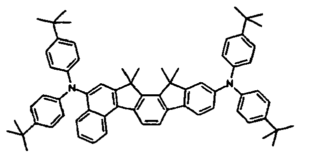 |
| (33) | (34) |
| 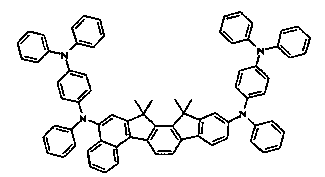 | 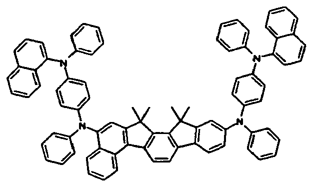 |
| (35) | (36) |
| 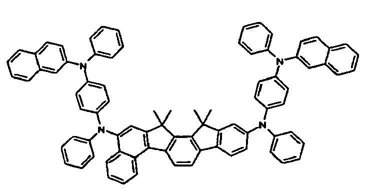 | 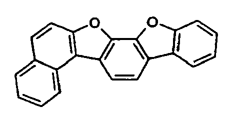 |
| (37) | (38) |
| 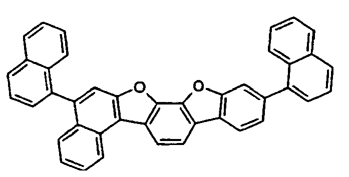 | 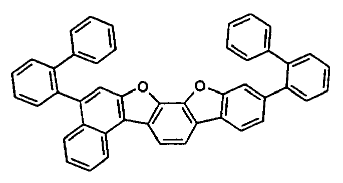 |
| (39) | (40) |
| 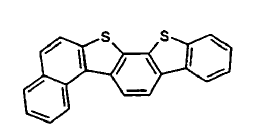 | 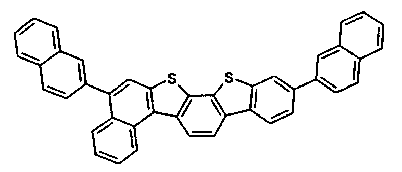 |
| (41) | (42) |
| 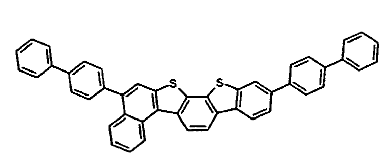 | 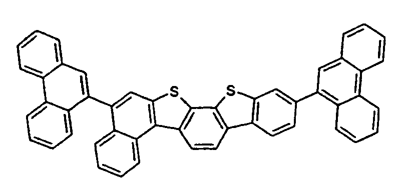 |
| (43) | (44) |
| 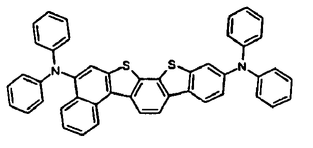 | 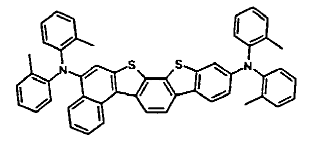 |
| (45) | (46) |
| 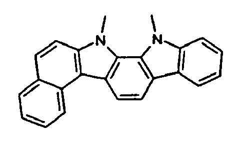 | 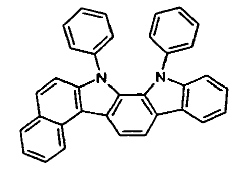 |
| (47) | (48) |
| 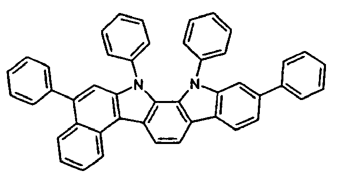 | 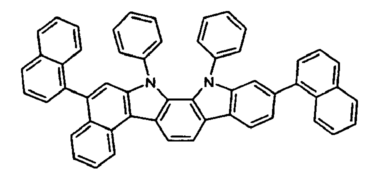 |
| (49) | (50) |
| 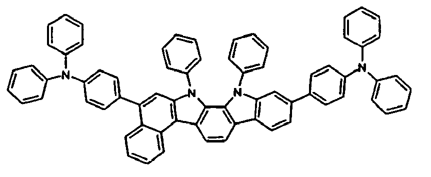 | 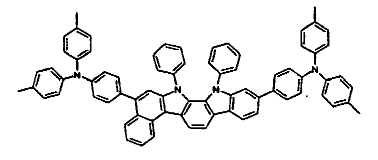 |
| (51) | (52) |
| 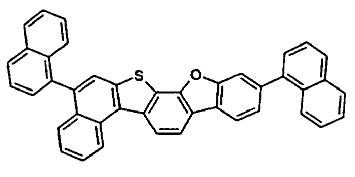 | 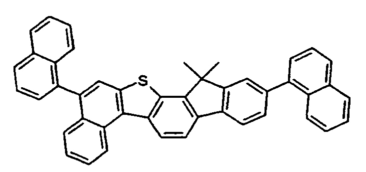 |
| (53) | (54) |
| 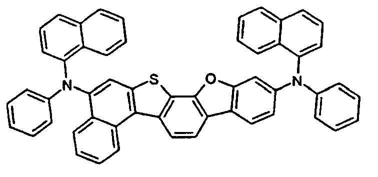 | 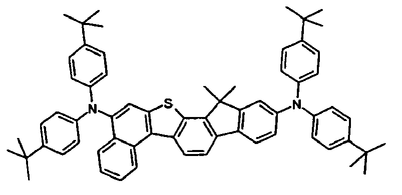 |
| (55) | (56) |
| 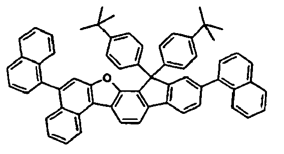 | 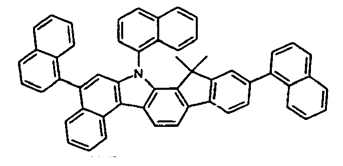 |
| (57) | (58) |
| 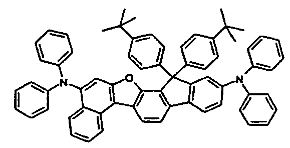 | 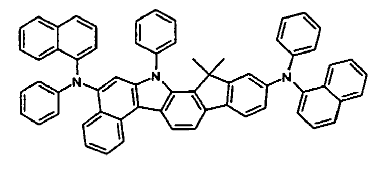 |
| (59) | (60) |
| 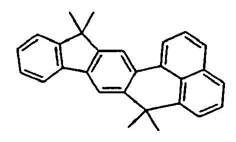 | 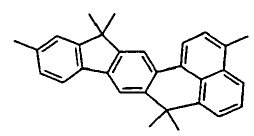 |
| (61) | (62) |
| 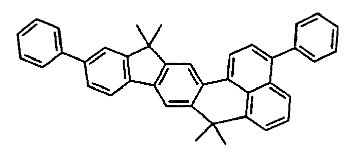 | 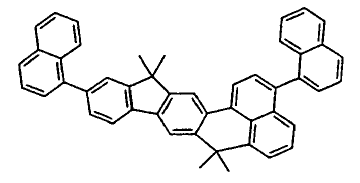 |
| (63) | (64) |
| 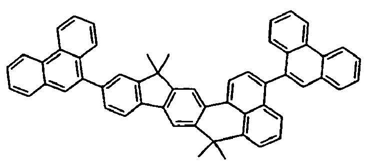 | 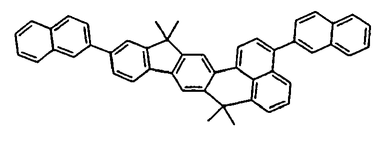 |
| (65) | (66) |
| 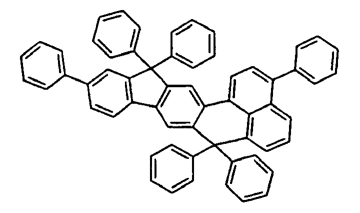 | 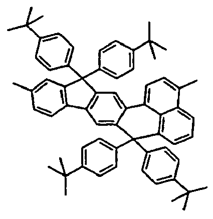 |
| (67) | (68) |
| 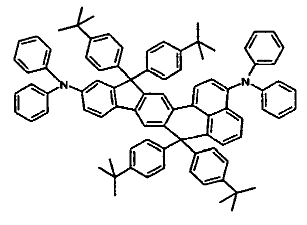 | 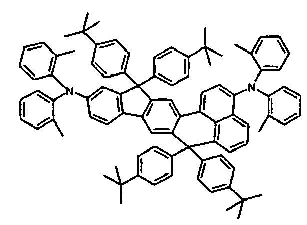 |
| (69) | (70) |
| 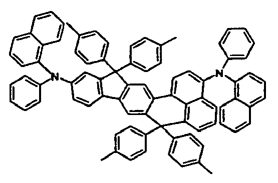 | 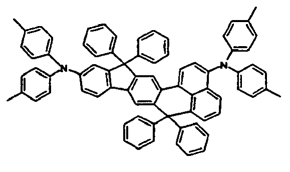 |
| (71) | (72) |
| 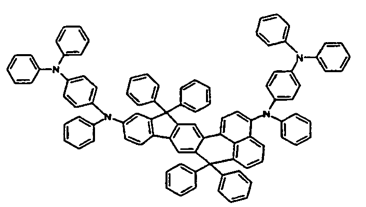 | 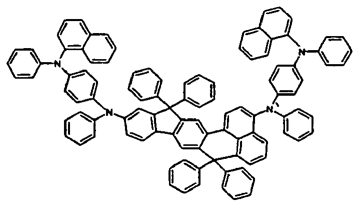 |
| (73) | (74) |
| 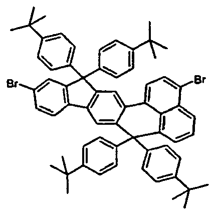 | 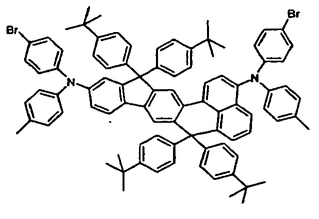 |
| (75) | (76) |
| 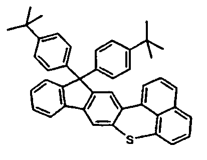 | 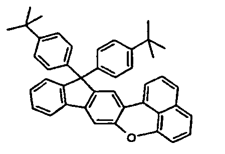 |
| (77) | (78) |
| 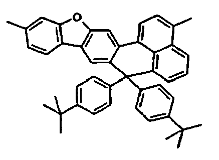 | 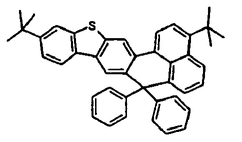 |
| (79) | (80) |
| 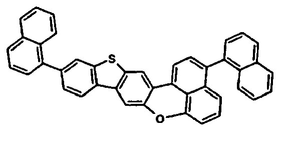 | 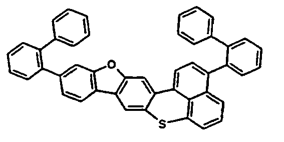 |
| (81) | (82) |
| 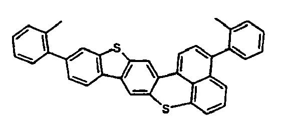 | 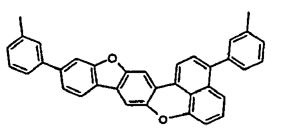 |
| (83) | (84) |
| 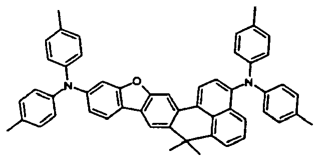 | 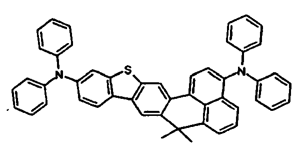 |
| (85) | (86) |
| 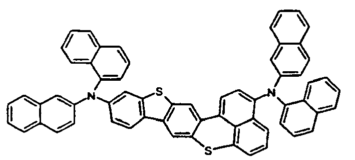 | 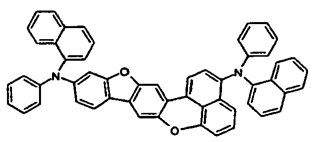 |
| (87) | (88) |
| 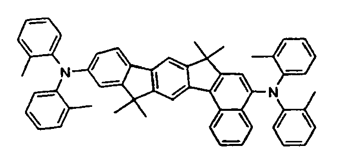 | 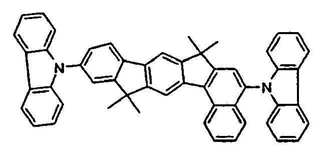 |
| (89) | (90) |
| 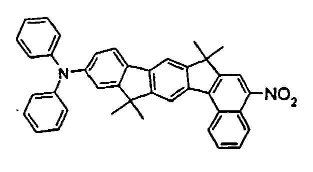 | 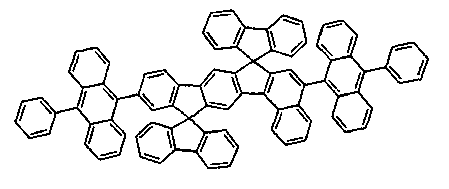 |
| (91) | (92) |
| 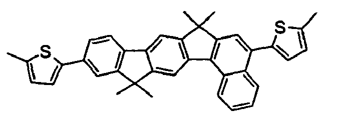 | 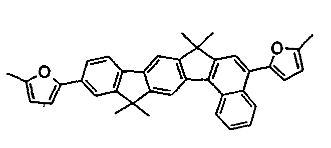 |
| (93) | (94) |
| 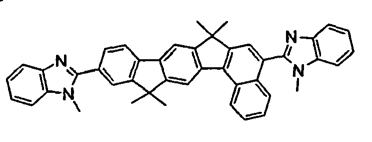 | 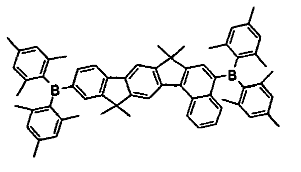 |
| (95) | (96) |
| 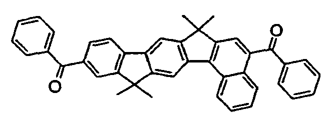 | 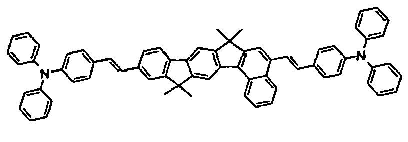 |
| (97) | (98) |
| 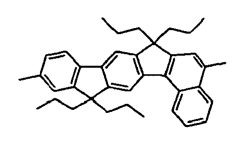 | 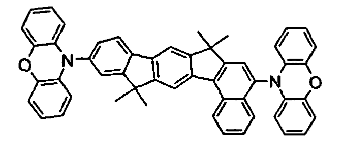 |
| (99) | (100) |
| 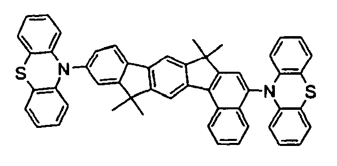 | 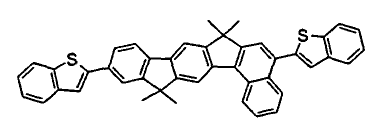 |
| (101) | (102) |
| 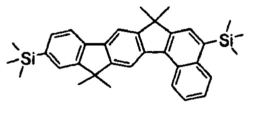 | 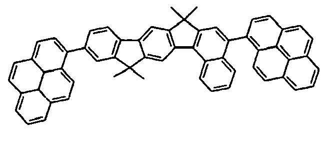 |
| (103) | (104) |
| 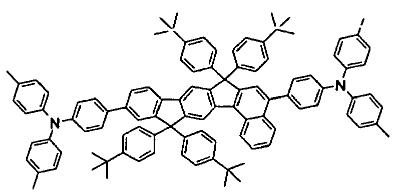 | 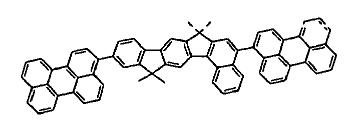 |
| (105) | (106) |
| 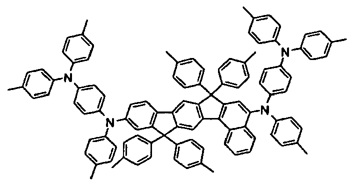 | 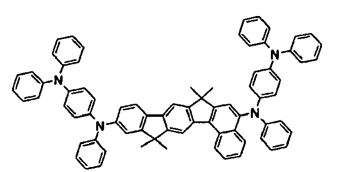 |
| (107) | (108) |
| 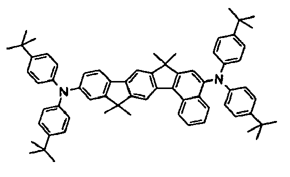 | |
| (109) | |
| 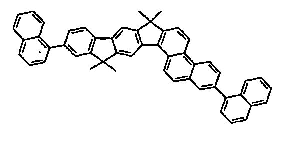 | |
| (111) | |
| 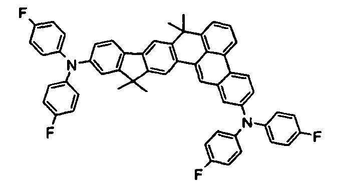 | 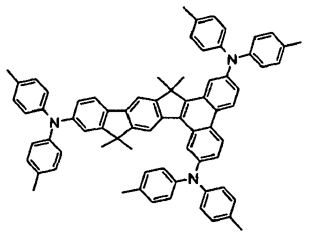 |
| (113) | (114) |
| 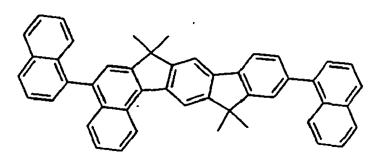 | 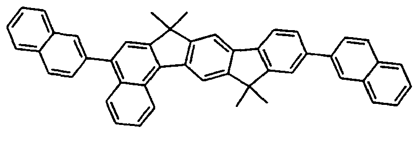 |
| (115) | (116) |
| 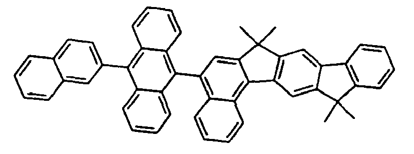 | 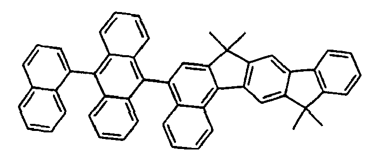 |
| (117) | (118) |
| 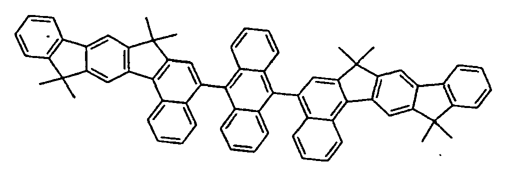 | 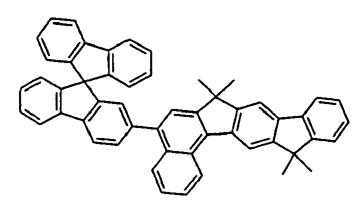 |
| (119) | (120) |
| 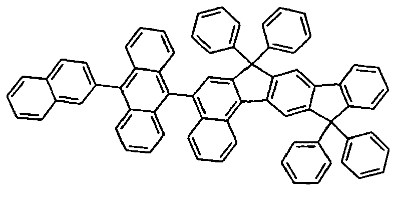 | 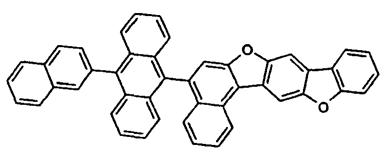 |
| (121) | (122) |
| 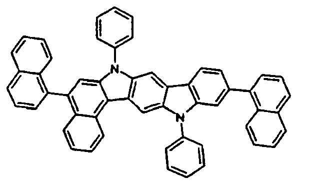 | 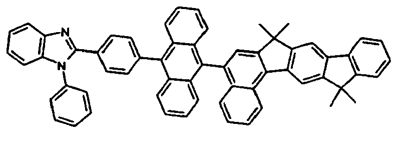 |
| (123) | (124) |
| 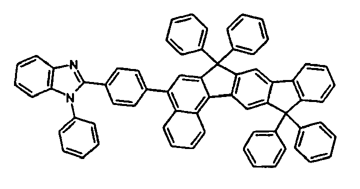 | 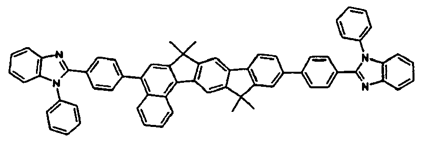 |
| (125) | (126) |
| 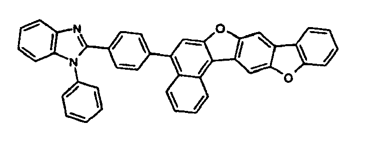 | 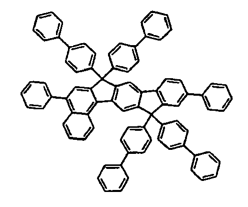 |
| (127) | (128) |
| 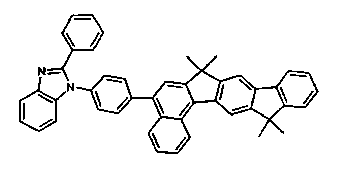 | 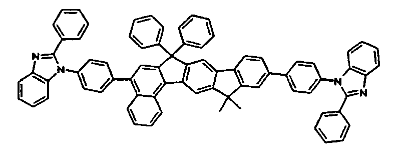 |
| (129) | (130) |
| 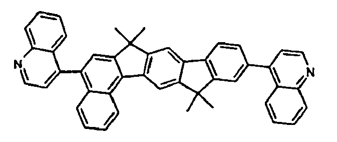 | 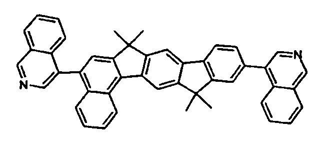 |
| (131) | (132) |
| 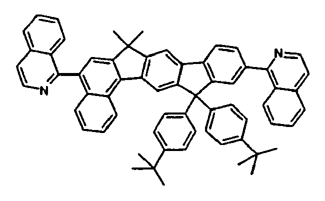 | 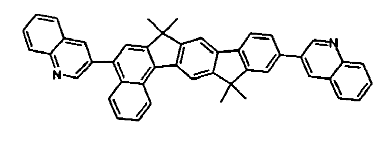 |
| (133) | (134) |
| 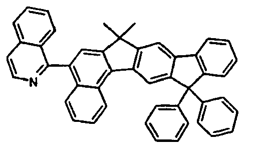 | 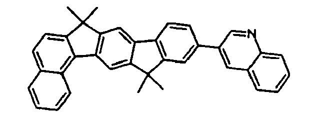 |
| (135) | (136) |
| 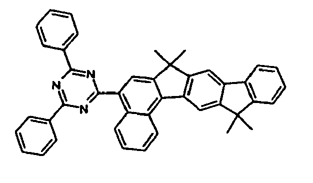 | 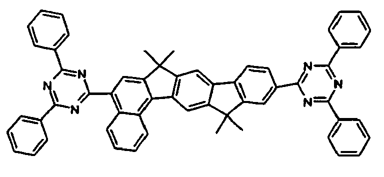 |
| (137) | (138) |
| 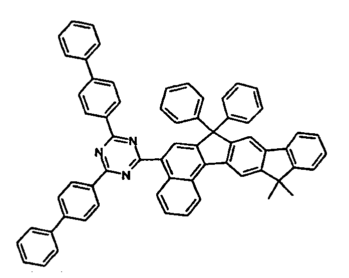 | 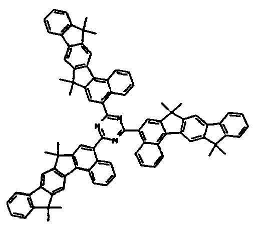 |
| (139) | (140) |
| 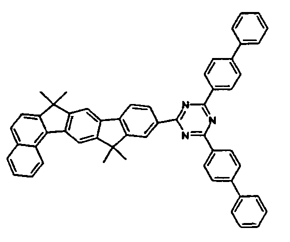 | 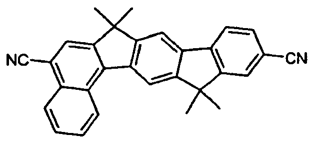 |
| (141) | (142) |
| 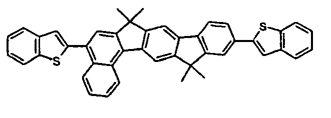 | 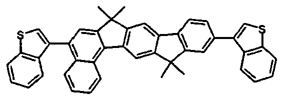 |
| | (144) |
| 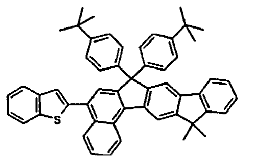 | 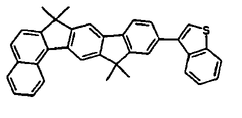 |
| (145) | (146) |
| 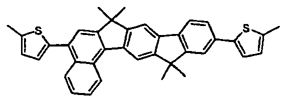 | 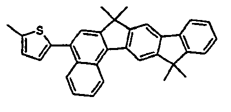 |
| (147) | (148) |
| 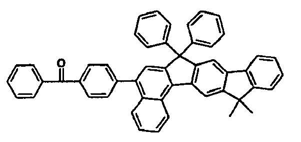 | 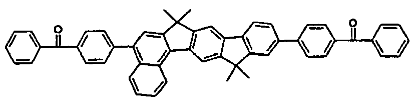 |
| (149) | (150) |
| 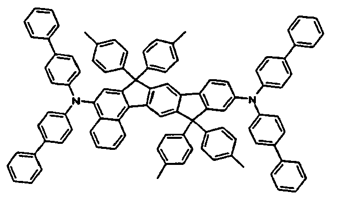 | 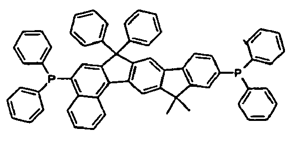 |
| (151) | (152) |
| 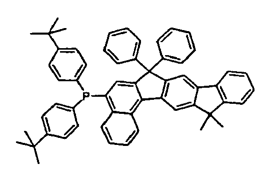 | 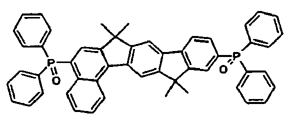 |
| (153) | (154) |
| 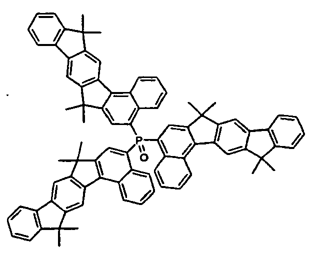 | 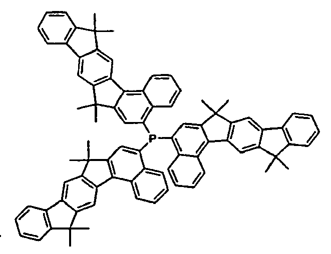 |
| (155) | (156) |
| 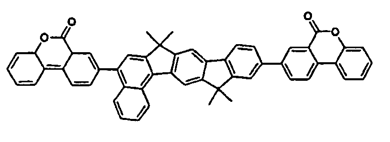 | 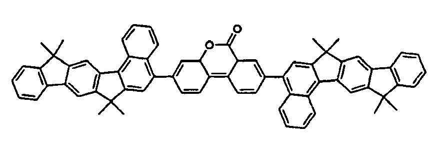 |
| (157) | (158) |
| 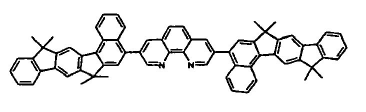 | 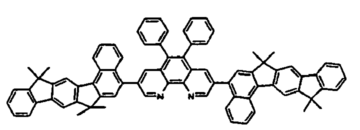 |
| (159) | (160) |
| 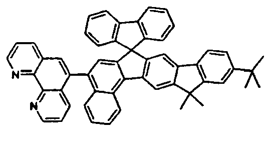 | 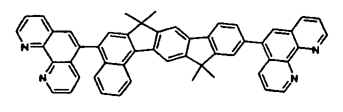 |
| (161) | (162) |
| 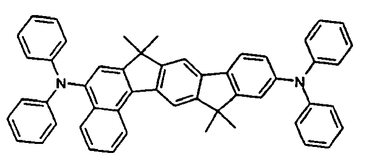 | 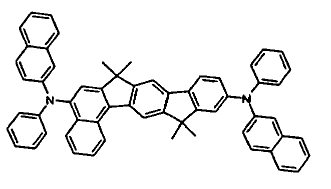 |
| (163) | (164) |
| 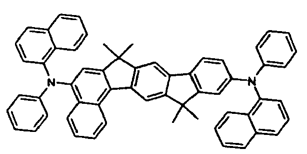 | 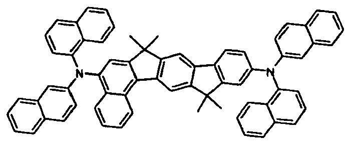 |
| (165) | (166) |
| 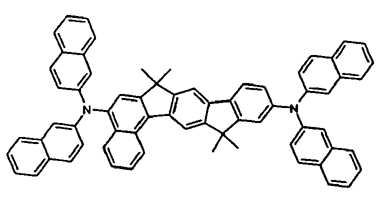 | 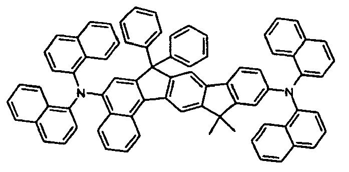 |
| (167) | (168) |
| 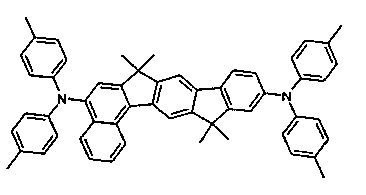 | 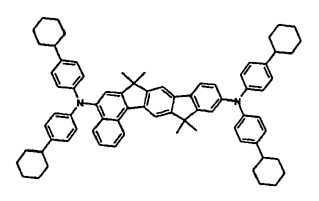 |
| (169) | (170) |
| 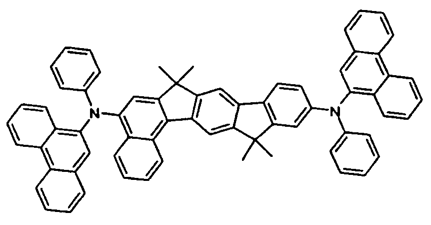 | 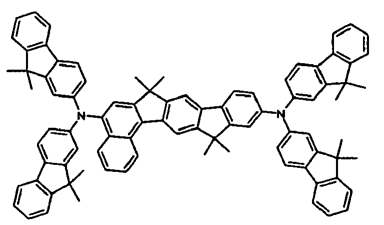 |
| (171) | (172) |
| 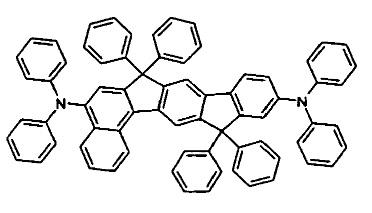 | 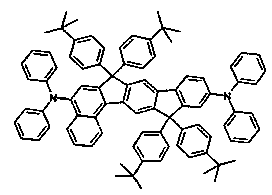 |
| (173) | (174) |
| 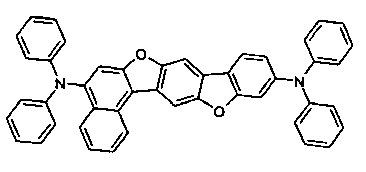 | 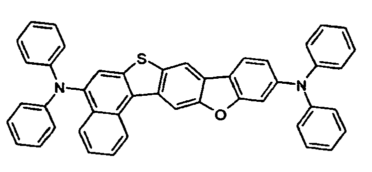 |
| (175) | (176) |
| 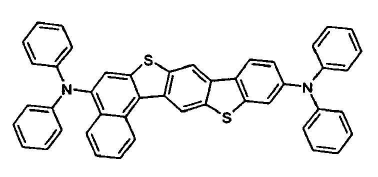 | 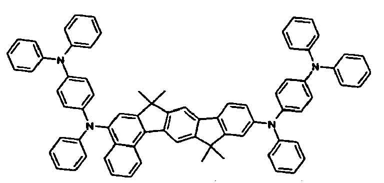 |
| (177) | (178) |
| 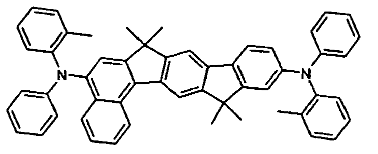 | 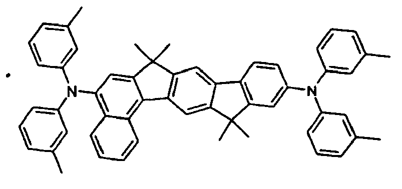 |
| (179) | (180) |
| 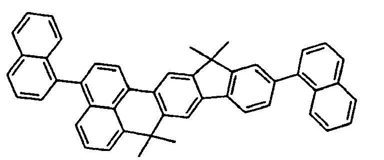 | 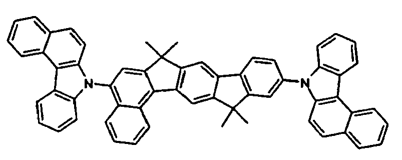 |
| (181) | (182) |
| 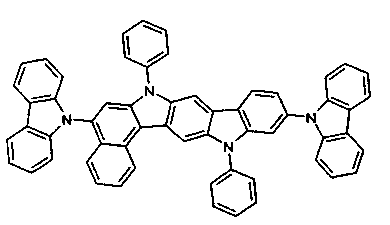 | 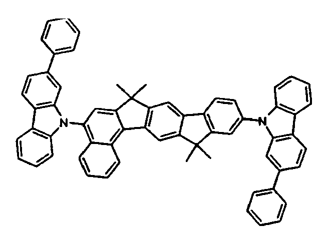 |
| (183) | (184) |
| 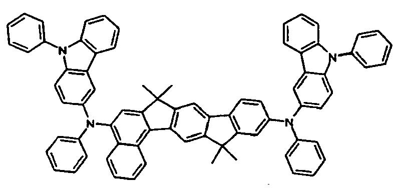 | 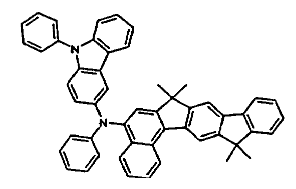 |
| (185) | (186) |
| 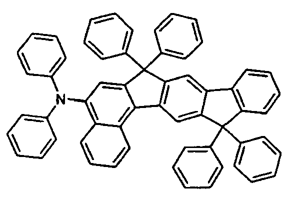 | 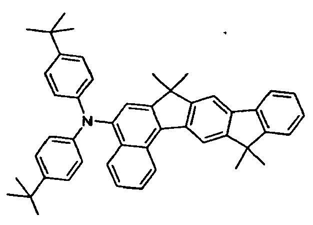 |
| (187) | (188) |
| 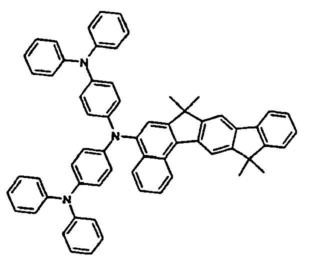 | 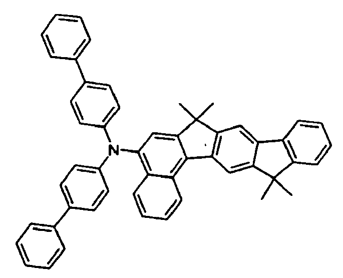 |
| (189) | (190) |
| 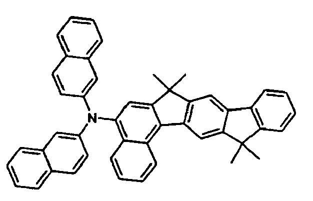 | 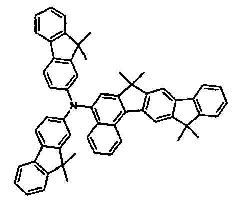 |
| (191) | (192) |
| 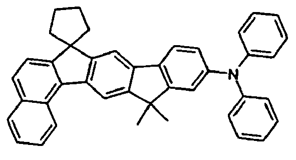 | 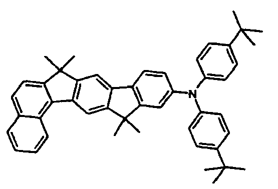 |
| (193) | (194) |
| 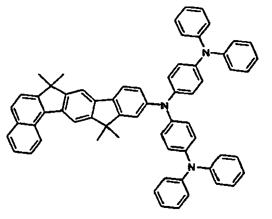 | 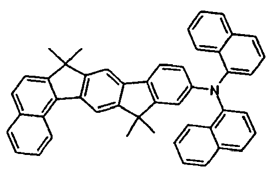 |
| (195) | (196) |
| 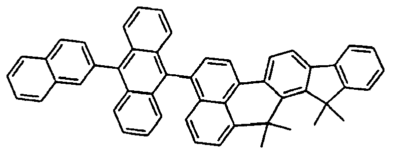 | 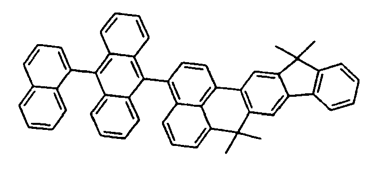 |
| (197) | (198) |
| 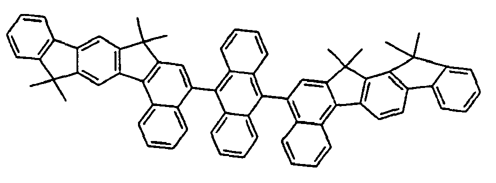 | 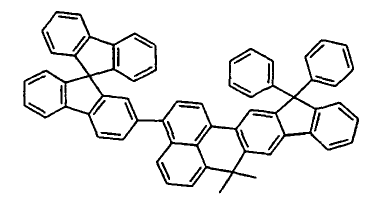 |
| (199) | (200) |
| 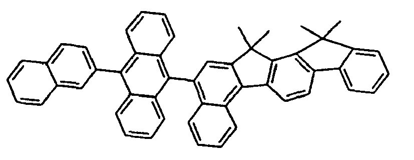 | 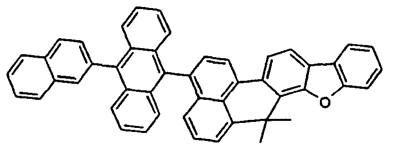 |
| (201) | (202) |
| 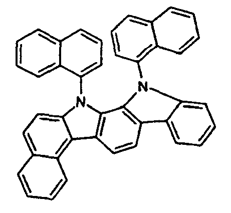 | 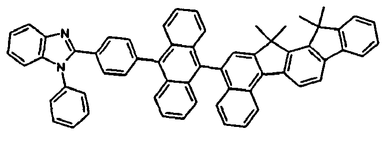 |
| (203) | (204) |
| 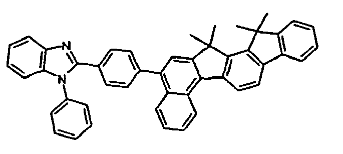 | 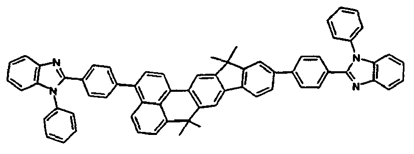 |
| (205) | (206) |
| 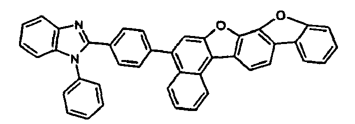 | 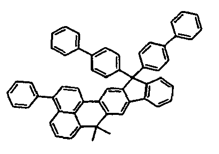 |
| (207) | (208) |
| 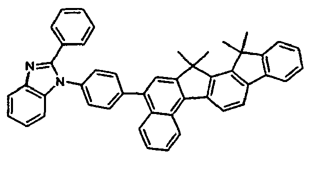 | 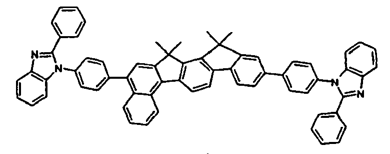 |
| (209) | (210) |
| 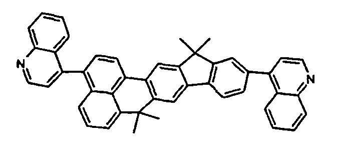 | 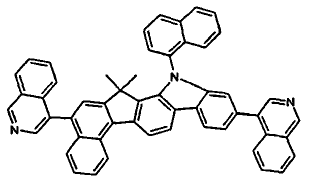 |
| (211) | (212) |
| 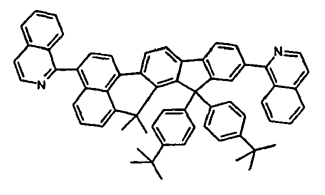 | 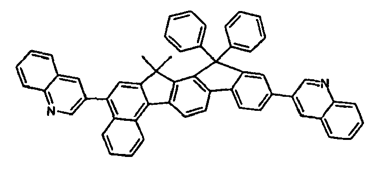 |
| (213) | (214) |
| 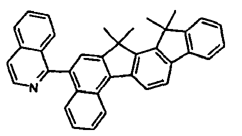 | 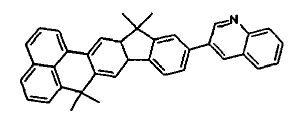 |
| (215) | (216) |
| 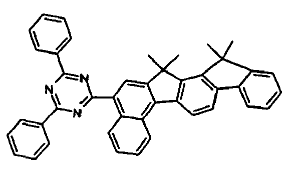 | 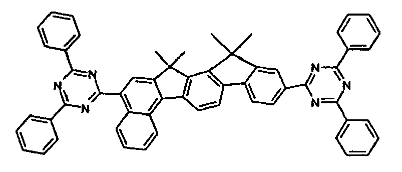 |
| (217) | (218) |
| 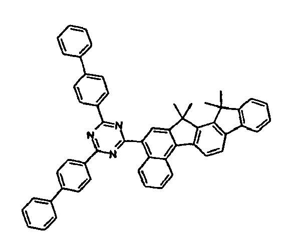 | 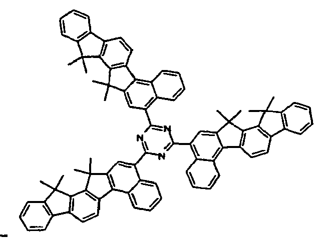 |
| (219) | (220) |
| 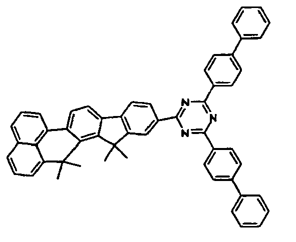 | 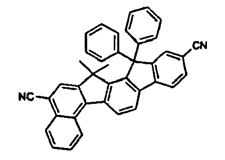 |
| (221) | (222) |
| 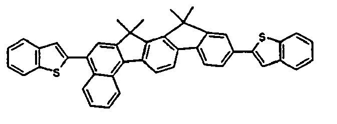 | 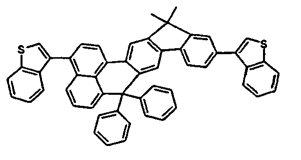 |
| (223) | (224) |
| 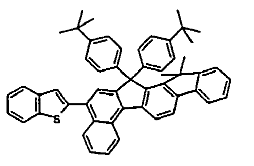 | 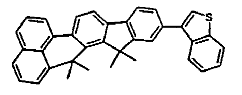 |
| (225) | (226) |
| 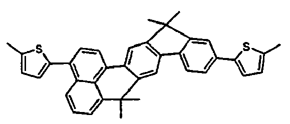 | 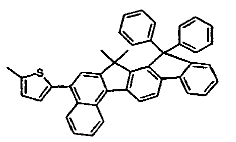 |
| (227) | (228) |
| 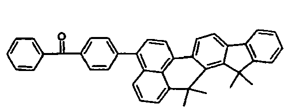 | 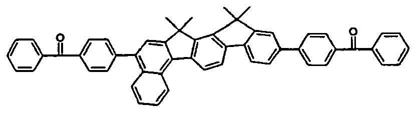 |
| (229) | (230) |
| 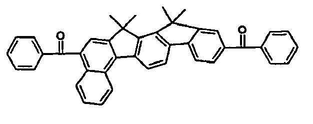 | 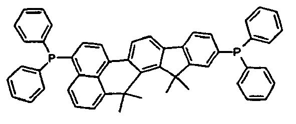 |
| (231) | (232) |
| 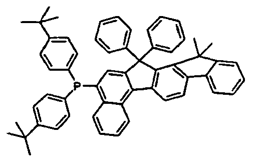 | 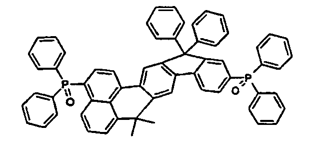 |
| (233) | (234) |
| 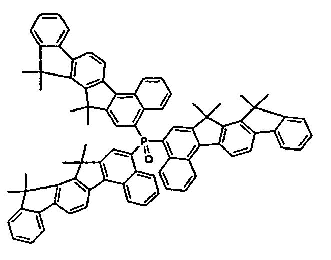 | 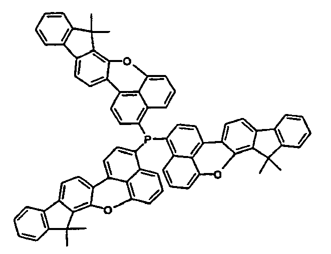 |
| (235) | (236) |
| 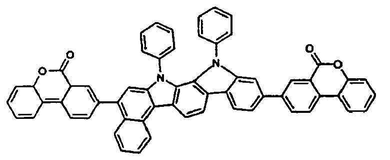 | 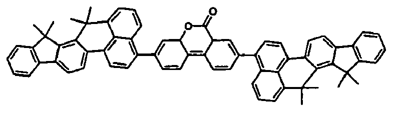 |
| (237) | (238) |
| 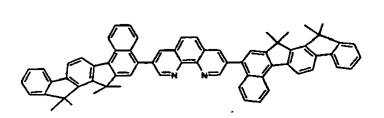 | 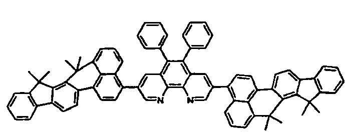 |
| (239) | (240) |
| 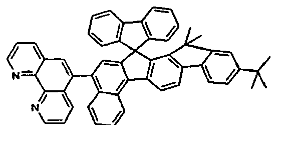 | 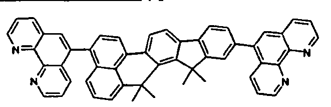 |
| (241) | (242) |
| 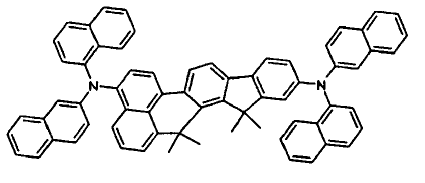 | 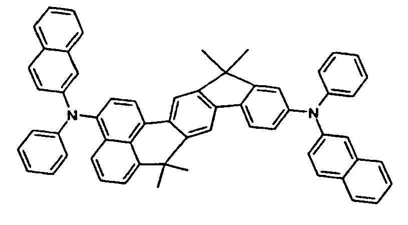 |
| (243) | (244) |
| 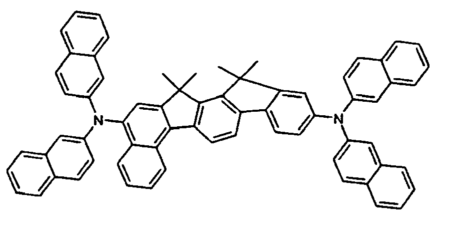 | 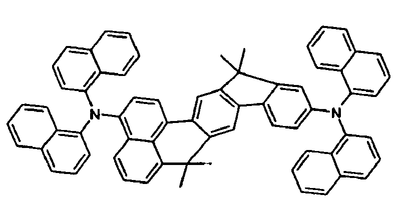 |
| (245) | (246) |
| 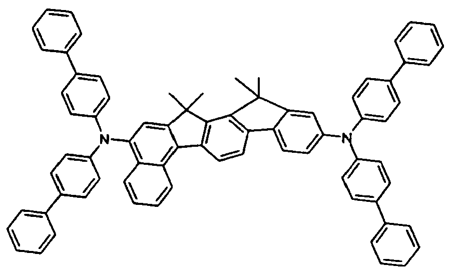 | 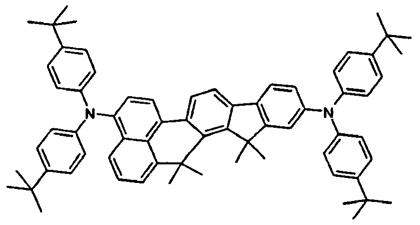 |
| (247) | (248) |
| 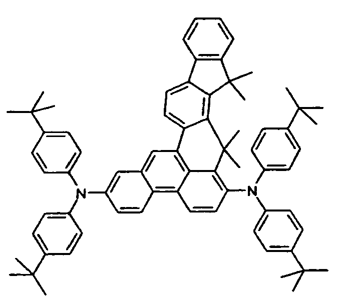 | 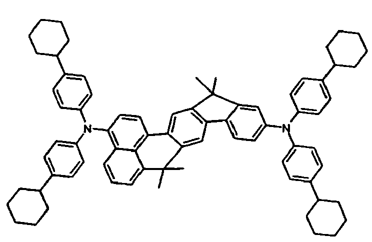 |
| (249) | (250) |
| 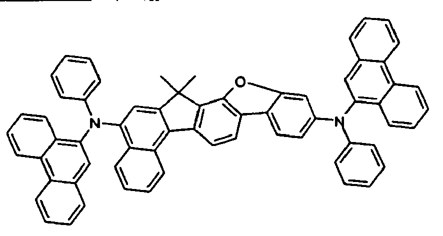 | 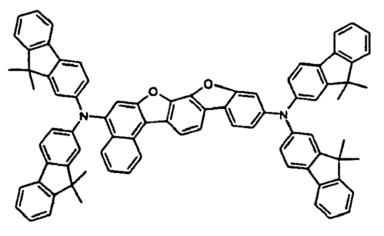 |
| (251) | (252) |
| 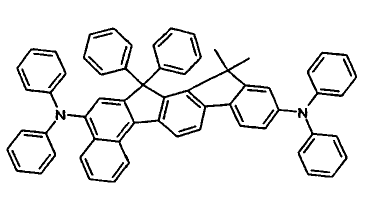 | 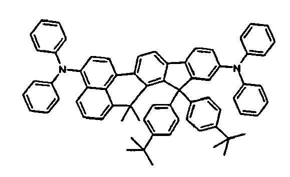 |
| (253) | (254) |
| 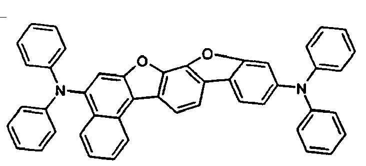 | 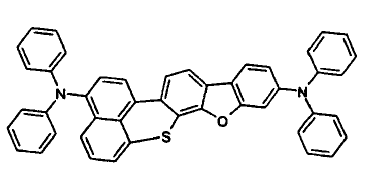 |
| (255) | (256) |
| 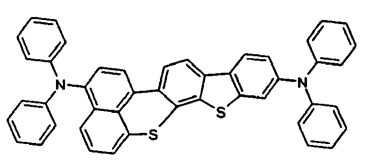 | 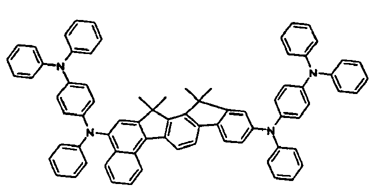 |
| (257) | (258) |
| 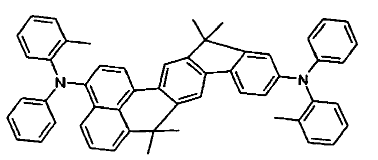 | 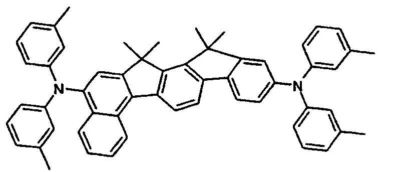 |
| (259) | (260) |
| 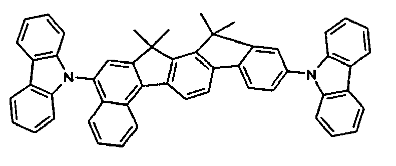 | 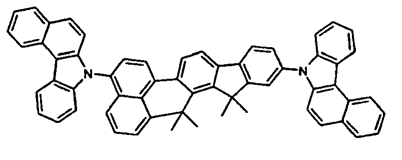 |
| (261) | (262) |
| 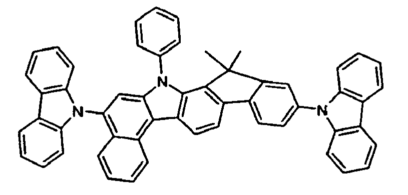 | 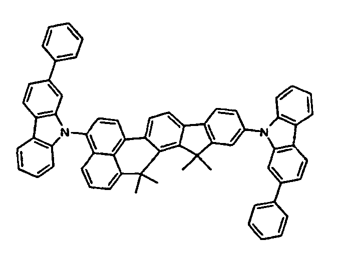 |
| (263) | (264) |
| 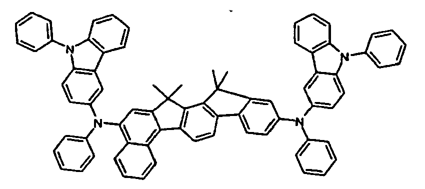 | 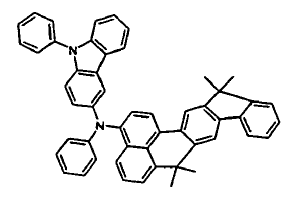 |
| (265) | (266) |
| 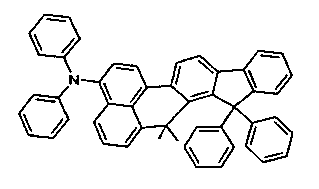 | 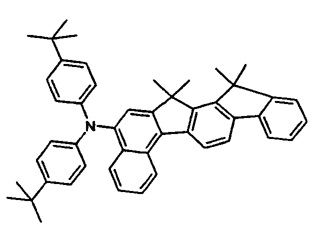 |
| (267) | (268) |
| 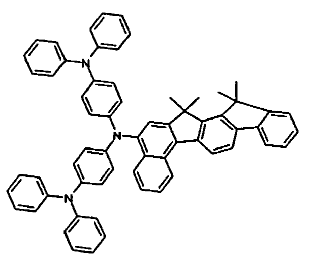 | 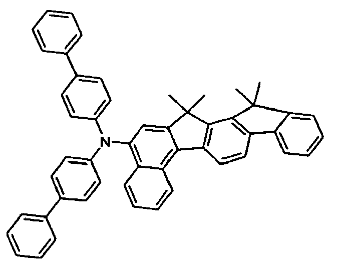 |
| (269) | (270) |
| 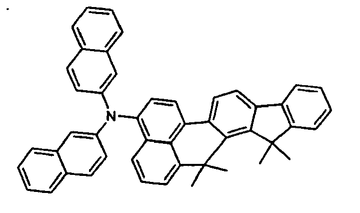 | 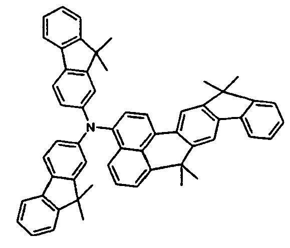 |
| (271) | (272) |
| 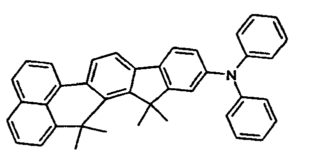 | 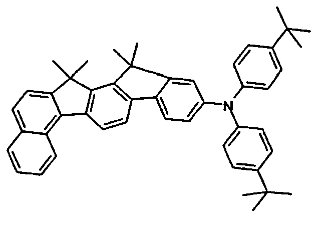 |
| (273) | (274) |
| 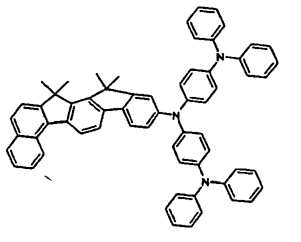 | 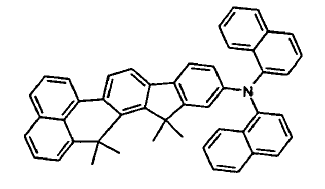 |
| (275) | (276) |
| 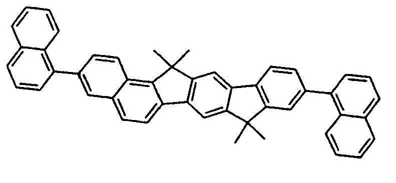 | 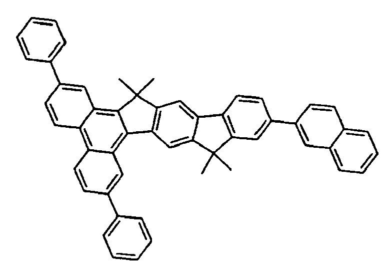 |
| (277) | (278) |
| 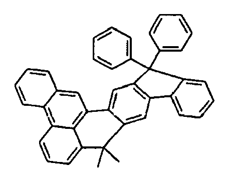 | |
| (279) | |
| | |
| | |
| 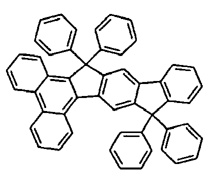 | 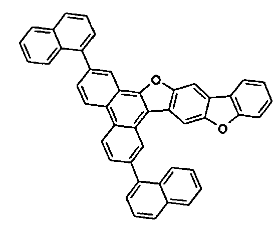 |
| (283) | (284) |
| 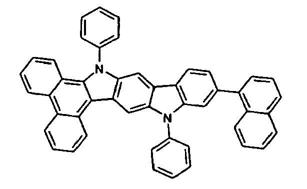 | 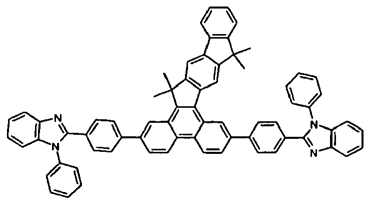 |
| (285) | (286) |
| | |
| | |
| 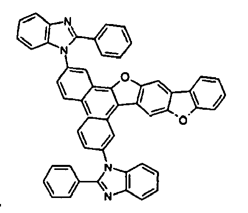 | |
| (289) | |
| 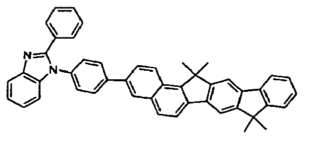 | 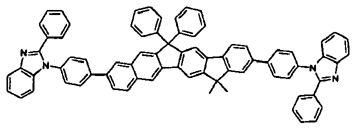 |
| (291) | (292) |
| 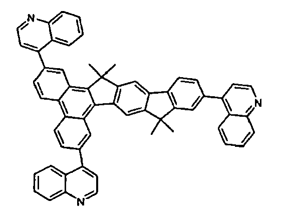 | |
| (293) | |
| 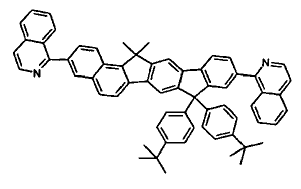 | 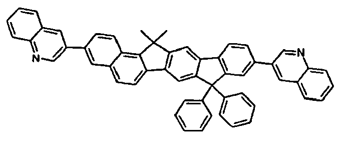 |
| (295) | (296) |
| | 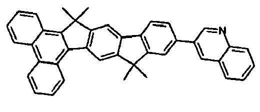 |
| | (298) |
| 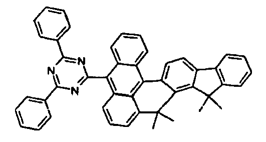 | 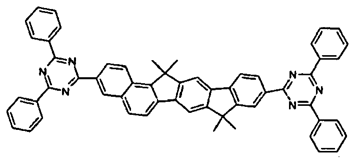 |
| (299) | (300) |
| 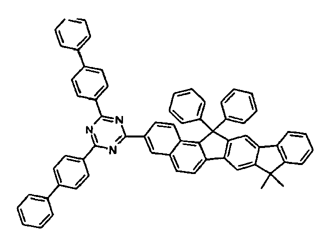 | 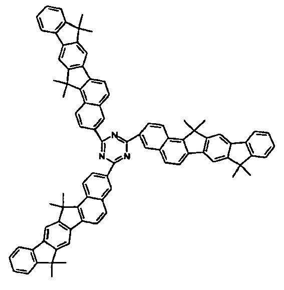 |
| (301) | (302) |
| 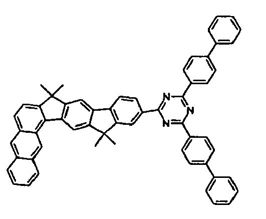 | |
| (303) | |
| 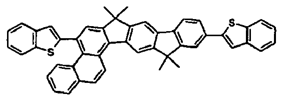 | 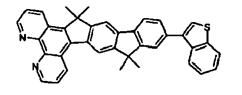 |
| (305) | (306) |
| 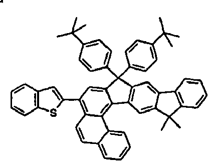 | 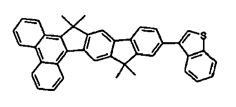 |
| (307) | (308) |
| | 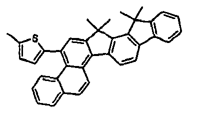 |
| | (310) |
| | 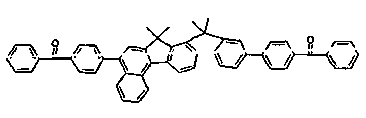 |
| | (312) |
| 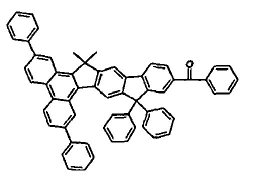 | |
| (313) | |
| 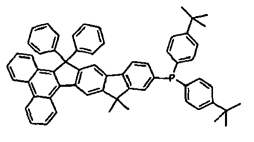 | |
| (315) | |
| | |
| | |
| | 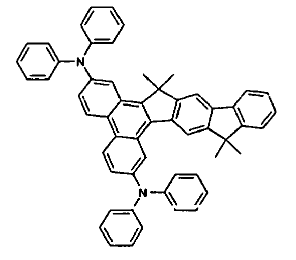 |
| | (320) |
| 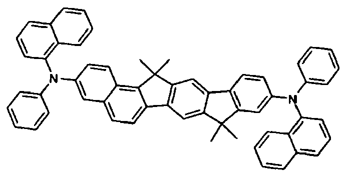 | |
| (321) | |
| 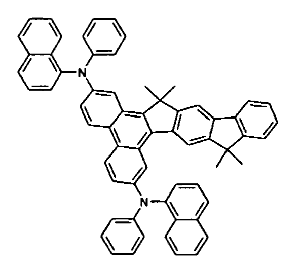 | 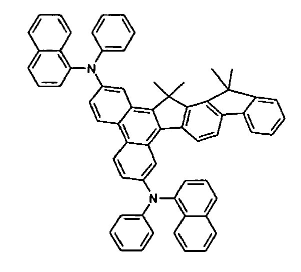 |
| (323) | (324) |
| 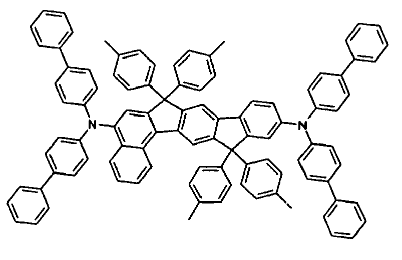 | 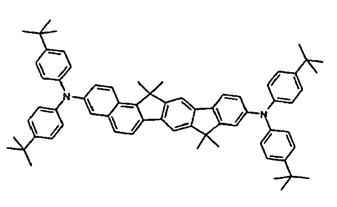 |
| (325) | (326) |
| 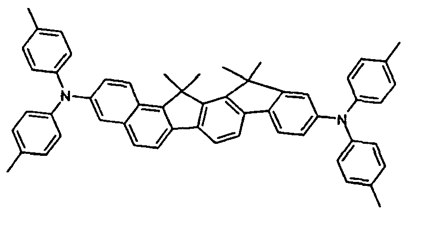 | 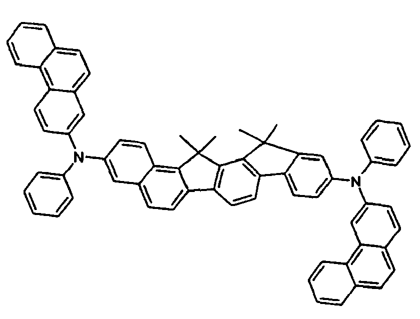 |
| (327) | (328) |
| 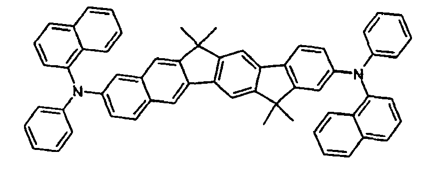 | 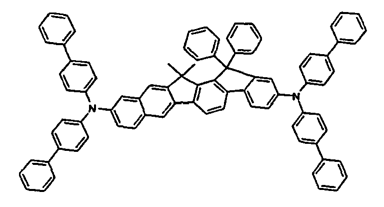 |
| (329) | (330) |
| 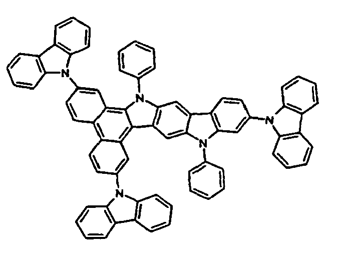 | 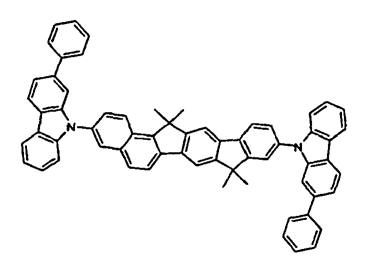 |
| (331) | (332) |
| 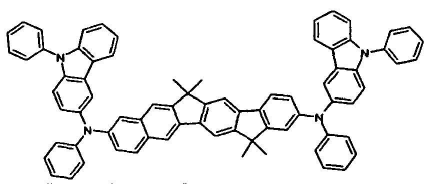 | 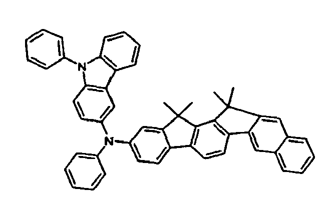 |
| (333) | (334) |
| 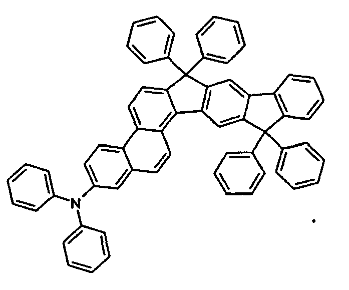 | 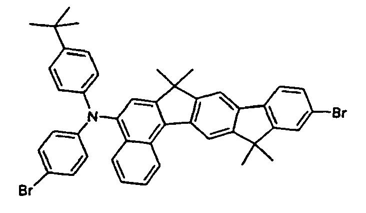 |
| (335) | (336) |
| 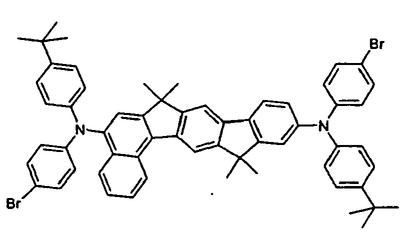 | 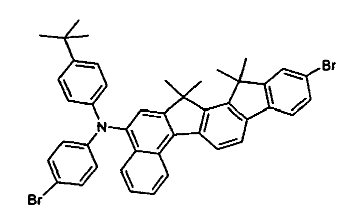 |
| (337) | (338) |

Weitere besonders bevorzugte Verbindungen können der folgenden Tabelle 1 entnommen werden, wobei sich die Reste auf die folgende Struktur beziehen:

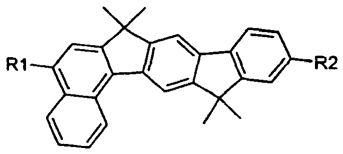

Dabei steht in Tabelle 1 Phenanthrenyl insbesondere für eine 9-Phenanthrenylgruppe. tBuPhenyl steht insbesondere für eine para-tert-Butylphenylgruppe. Ebenso bevorzugt wie die Strukturen in der Tabelle 1 sind die entsprechenden Strukturen, in denen statt der Methylgruppen an der Brücke X Phenylgruppen gebunden sind oder in denen statt der Methylgruppen an der Brücke X para-tert-Butylphenylgruppen gebunden sind. Weiterhin sind ebenso bevorzugt die Strukturen, in denen statt meta-Tolylgruppen als Gruppen Ar1, Ar2, Ar3 bzw. Ar4 ortho-Tolylgruppen bzw. para-Tolylgruppen gebunden sind.

**Tabelle 1: Bevorzugte Strukturen**

| Nr. | R1 | R2 | R1 | | R2 | |
|---|---|---|---|---|---|---|
| | | | Ar1 | Ar2 | Ar3 | Ar4 |
| 1 | H | H | - | - | - | - |
| 2 | H | Phenyl | - | - | - | - |
| 3 | H | 1-Naphthyl- | - | - | - | - |
| 4 | H | 2-Naphthyl | - | - | - | - |
| 5 | H | NAr3Ar4 | - | - | Phenyl | Phenyl |
| 6 | H | NAr3Ar4 | - | - | Phenyl | 1-Naphthyl |
| 7 | H | NAr3Ar4 | - | - | Phenyl | 2-Naphthyl |
| 8 | H | NAr3Ar4 | - | - | Phenyl | m-Tolyl |
| 9 | H | NAr3Ar4 | - | - | Phenyl | tBuPhenyl |
| 10 | H | NAr3Ar4 | - | - | Phenyl | Phenanthrenyl |
| 11 | H | NAr3Ar4 | - | - | 1-Naphthyl | 1-Naphthyl |
| 12 | H | NAr3Ar4 | - | - | 2-Naphthyl | 2-Naphthyl |
| 13 | H | NAr3Ar4 | - | - | m-Tolyl | m-Tolyl |
| 14 | H | NAr3Ar4 | - | - | tBuPhenyl | tBuPhenyl |
| 15 | H | Ph-NAr3Ar4 | - | - | Phenyl | Phenyl, |
| 16 | H | Ph-NAr3Ar4 | - | - | Phenyl, | 1-Naphthyl |
| 17 | H | Ph-NAr3Ar4 | - | - | Phenyl | 2-Naphthyl |
| 18 | H | Ph-NAr3Ar4 | - | - | Phenyl | m-Tolyl |
| 19 | H | Ph-NAr3Ar4 | - | - | Phenyl | tBuPhenyl |
| 20 | H | Ph-NAr3Ar4 | - | - | Phenyl | Phenanthrenyl |
| 21 | H | Ph-NAr3Ar4 | - | - | 1-Naphthyl | 1-Naphthyl |
| 22 | H | Ph-NAr3Ar4 | - | - | 2-Naphthyl | 2-Naphthyl |
| 23 | H | Ph-NAr3Ar4 | - | - | m-Tolyl | m-Tolyl |
| 24 | H | Ph-NAr3Ar4 | - | - | tBuPhenyl | tBuPhenyl |
| 25 | Phenyl | H | - | - | - | - |
| 26 | Phenyl | Phenyl | - | - | - | - |
| 27 | Phenyl | 1-Naphthyl | - | - | - | - |
| 28 | Phenyl | 2-Naphthyl | - | - | - | - |
| 29 | Phenyl | NAr3Ar4 | - | - | Phenyl | Phenyl |
| 30 | Phenyl | NAr3Ar4 | - | - | Phenyl | 1-Naphthyl |
| 31 | Phenyl | NAr3Ar4 | - | - | Phenyl | 2-Naphthyl |
| 32 | Phenyl | NAr3Ar4 | - | - | Phenyl | m-Tolyl |
| 33 | Phenyl | NAr3Ar4 | - | - | Phenyl | tBuPhenyl |
| 34 | Phenyl | NAr3Ar4 | - | - | Phenyl | Phenanthrenyl |
| 35 | Phenyl | NAr3Ar4 | - | - | 1-Naphthyl | 1-Naphthyl |
| 36 | Phenyl | NAr3Ar4 | - | - | 2-Naphthyl | 2-Naphthyl |
| 37 | Phenyl | NAr3Ar4 | - | - | m-Tolyl | m-Tolyl |
| 38 | Phenyl | NAr3Ar4 | - | - | tBuPhenyl | tBuPhenyl |
| 39 | Phenyl | Ph-NAr3Ar4 | - | - | Phenyl | Phenyl |
| 40 | Phenyl | Ph-NAr3Ar4 | - | - | Phenyl | 1-Naphthyl |
| 41 | Phenyl | Ph-NAr3Ar4 | - | - | Phenyl | 2-Naphthyl |
| 42 | Phenyl | Ph-NAr3Ar4 | - | - | Phenyl | m-Tolyl |
| 43 | Phenyl | Ph-NAr3Ar4 | - | - | Phenyl | tBuPhenyl |
| 44 | Phenyl | Ph-NAr3Ar4 | - | - | Phenyl | Phenanthrenyl |
| 45 | Phenyl | Ph-NAr3Ar4 | - | - | 1-Naphthyl | 1-Naphthyl |
| 46 | Phenyl | Ph-NAr3Ar4 | - | - | 2-Naphthyl | 2-Naphthyl |
| 47 | Phenyl | Ph-NAr3Ar4 | - | - | m-Tolyl | m-Tolyl |
| 48 | Phenyl | Ph-NAr3Ar4 | - | - | tBuPhenyl | tBuPhenyl |
| 49 | 1-Naphthyl | H | - | - | - | - |
| 50 | 1-Naphthyl | Phenyl | - | - | - | - |
| 51 | 1-Naphthyl | 1-Naphthyl | - | - | - | - |
| 52 | 1-Naphthyl | 2-Naphthyl | - | - | - | - |
| 53 | 1-Naphthyl | NAr3Ar4 | - | - | Phenyl | Phenyl |
| 54 | 1-Naphthyl | NAr3Ar4 | - | - | Phenyl | 1-Naphthyl |
| 55 | 1-Naphthyl | NAr3Ar4 | - | - | Phenyl | 2-Naphthyl |
| 56 | 1-Naphthyl | NAr3Ar4 | - | - | Phenyl | m-Tolyl |
| 57 | 1-Naphthyl | NAr3Ar4 | - | - | Phenyl | tBuPhenyl |
| 58 | 1-Naphthyl | NAr3Ar4 | - | - | Phenyl | Phenanthrenyl |
| 59 | 1-Naphthyl | NAr3Ar4 | - | - | 1-Naphthyl | 1-Naphthyl |
| 60 | 1-Naphthyl | NAr3Ar4 | - | - | 2-Naphthyl | 2-Naphthyl |
| 61 | 1-Naphthyl | NAr3Ar4 | - | - | m-Tolyl | m-Tolyl |
| 62 | 1-Naphthyl | NAr3Ar4 | - | - | tBuPhenyl | tBuPhenyl |
| 63 | 1-Naphthyl | Ph-NAr3Ar4 | - | - | Phenyl | Phenyl |
| 64 | 1-Naphthyl | Ph-NAr3Ar4 | - | - | Phenyl | 1-Naphthyl- |
| 65 | 1-Naphthyl | Ph-NAr3Ar4 | - | - | Phenyl | 2-Naphthyl |
| 66 | 1-Naphthyl | Ph-NAr3Ar4 | - | - | Phenyl | m-Tolyl |
| 67 | 1-Naphthyl | Ph-NAr3Ar4 | - | - | Phenyl | tBuPhenyl |
| 68 | 1-Naphthyl | Ph-NAr3Ar4 | - | - | Phenyl | Phenanthrenyl |
| 69 | 1-Naphthyl | Ph-NAr3Ar4 | - | - | 1-Naphthyl | 1-Naphthyl |
| 70 | 1-Naphthyl | Ph-NAr3Ar4 | - | - | 2-Naphthyl | 2-Naphthyl |
| 71 | 1-Naphthyl | Ph-NAr3Ar4 | - | - | m-Tolyl | m-Tolyl |
| 72 | 1-Naphthyl | Ph-NAr3Ar4 | - | - | tBuPhenyl | tBuPhenyl |
| 73 | 2-Naphthyl | H | - | - | - | - |
| 74 | 2-Naphthyl | Phenyl | - | - | - | - |
| 75 | 2-Naphthyl | 1-Naphthyl | - | - | - | - |
| 76 | 2-Naphthyl | 2-Naphthyl | - | - | - | - |
| 77 | 2-Naphthyl | NAr3Ar4 | - | - | Phenyl | Phenyl |
| 78 | 2-Naphthyl | NAr3Ar4 | - | - | Phenyl | 1-Naphthyl |
| 79 | 2-Naphthyl | NAr3Ar4 | - | - | Phenyl | 2-Naphthyl |
| 80 | 2-Naphthyl | NAr3Ar4 | - | - | Phenyl | m-Tolyl |
| 81 | 2-Naphthyl | NAr3Ar4 | - | - | Phenyl | tBuPhenyl |
| 82 | 2-Naphthyl | NAr3Ar4 | - | - | Phenyl | Phenanthrenyl |
| 83 | 2-Naphthyl | NAr3Ar4 | - | - | 1-Naphthyl | 1-Naphthyl |
| 84 | 2-Naphthyl | NAr3Ar4 | - | - | 2-Naphthyl | 2-Naphthyl |
| 85 | 2-Naphthyl | NAr3Ar4 | - | - | m-Tolyl | m-Tolyl |
| 86 | 2-Naphthyl | NAr3Ar4 | - | - | tBuPhenyl | tBuPhenyl |
| 87 | 2-Naphthyl | Ph-NAr3Ar4 | - | - | Phenyl | Phenyl |
| 88 | 2-Naphthyl | Ph-NAr3Ar4 | - | - | Phenyl | 1-Naphthyl |
| 89 | 2-Naphthyl | Ph-NAr3Ar4 | - | - | Phenyl | 2-Naphthyl |
| 90 | 2-Naphthyl | Ph-NAr3Ar4 | - | - | Phenyl | m-Tolyl |
| 91 | 2-Naphthyl | Ph-NAr3Ar4 | - | - | Phenyl | tBuPhenyl |
| 92 | 2-Naphthyl | Ph-NAr3Ar4 | - | - | Phenyl | Phenanthrenyl |
| 93 | 2-Naphthyl | Ph-NAr3Ar4 | - | - | 1-Naphthyl | 1-Naphthyl- |
| 94 | 2-Naphthyl | Ph-NAr3Ar4 | - | - | 2-Naphthyl | 2-Naphthyl |
| 95 | 2-Naphthyl | Ph-NAr3Ar4 | - | - | m-Tolyl | m-Tolyl |
| 96 | 2-Naphthyl | Ph-NAr3Ar4 | - | - | tBuPhenyl | tBuPhenyl |
| 97 | NAr1Ar2 | H | Phenyl | Phenyl | - | - |
| 98 | NAr1Ar2 | Phenyl | Phenyl | Phenyl | - | - |
| 99 | NAr1Ar2 | 1-Naphthyl | Phenyl | Phenyl | - | - |
| 100 | NAr1Ar2 | 2-Naphthyl | Phenyl | Phenyl | - | - |
| 101 | NAr1Ar2 | NAr3Ar4 | Phenyl | Phenyl | Phenyl | Phenyl |
| 102 | NAr1Ar2 | NAr3Ar4 | Phenyl | Phenyl | Phenyl | 1-Naphthyl |
| 103 | NAr1Ar2 | NAr3Ar4 | Phenyl | Phenyl | Phenyl | 2-Naphthyl |
| 104 | NAr1Ar2 | NAr3Ar4 | Phenyl | Phenyl | Phenyl | m-Tolyl |
| 105 | NAr1Ar2 | NAr3Ar4 | Phenyl | Phenyl | Phenyl | tBuPhenyl |
| 106 | NAr1Ar2 | NAr3Ar4 | Phenyl | Phenyl | Phenyl | Phenanthrenyl |
| 107 | NAr1Ar2 | NAr3Ar4 | Phenyl | Phenyl | 1-Naphthyl | 1-Naphthyl |
| 108 | NAr1Ar2 | NAr3Ar4 | Phenyl | Phenyl | 2-Naphthyl | 2-Naphthyl |
| 109 | NAr1Ar2 | NAr3Ar4 | Phenyl | Phenyl | m-Tolyl | m-Tolyl |
| 110 | NAr1Ar2 | NAr3Ar4 | Phenyl | Phenyl | tBuPhenyl | tBuPhenyl |
| 111 | NAr1Ar2 | Ph-NAr3Ar4 | Phenyl | Phenyl | Phenyl | Phenyl |
| 112 | NAr1Ar2 | Ph-NAr3Ar4 | Phenyl | Phenyl | Phenyl | 1-Naphthyl |
| 113 | NAr1Ar2 | Ph-NAr3Ar4 | Phenyl | Phenyl | Phenyl | 2-Naphthyl |
| 114 | NAr1Ar2 | Ph-NAr3Ar4 | Phenyl | Phenyl | Phenyl | m-Tolyl |
| 115 | NAr1Ar2 | Ph-NAr3Ar4 | Phenyl | Phenyl | Phenyl | tBuPhenyl |
| 116 | NAr1Ar2 | Ph-NAr3Ar4 | Phenyl | Phenyl | Phenyl | Phenanthrenyl |
| 117 | NAr1Ar2 | Ph-NAr3Ar4 | Phenyl | Phenyl | 1-Naphthyl | 1-Naphthyl |
| 118 | NAr1Ar2 | Ph-NAr3Ar4 | Phenyl | Phenyl | 2-Naphthyl | 2-Naphthyl |
| 119 | NAr1Ar2 | Ph-NAr3Ar4 | Phenyl | Phenyl | m-Tolyl | m-Tolyl |
| 120 | NAr1Ar2 | Ph-NAr3Ar4 | Phenyl | Phenyl | tBuPhenyl | tBuPhenyl |
| 121 | NAr1Ar2 | H | Phenyl | 1-Naphthyl | - | - |
| 122 | NAr1Ar2 | Phenyl | Phenyl | 1-Naphthyl | - | - |
| 123 | NAr1Ar2 | 1-Naphthyl | Phenyl | 1-Naphthyl | - | - |
| 124 | NAr1Ar2 | 2-Naphthyl | Phenyl | 1-Naphthyl | - | - |
| 125 | NAr1Ar2 | NAr3Ar4 | Phenyl | 1-Naphthyl | Phenyl | Phenyl |
| 126 | NAr1Ar2 | NAr3Ar4 | Phenyl | 1-Naphthyl | Phenyl | 1-Naphthyl |
| 127 | NAr1Ar2 | NAr3Ar4 | Phenyl | 1-Naphthyl | Phenyl | 2-Naphthyl |
| 128 | NAr1Ar2 | NAr3Ar4 | Phenyl | 1-Naphthyl | Phenyl | m-Tolyl |
| 129 | NAr1Ar2 | NAr3Ar4 | Phenyl | 1-Naphthyl | Phenyl | tBuPhenyl |
| 130 | NAr1Ar2 | NAr3Ar4 | Phenyl | 1-Naphthyl | Phenyl | Phenanthrenyl |
| 131 | NAr1Ar2 | NAr3Ar4 | Phenyl | 1-Naphthyl | 1-Naphthyl | 1-Naphthyl |
| 132 | NAr1Ar2 | NAr3Ar4 | Phenyl | 1-Naphthyl | 2-Naphthyl | 2-Naphthyl |
| 133 | NAr1Ar2 | NAr3Ar4 | Phenyl | 1-Naphthyl | m-Tolyl | m-Tolyl |
| 134 | NAr1Ar2 | NAr3Ar4 | Phenyl | 1-Naphthyl | tBuPhenyl | tBuPhenyl |
| 135 | NAr1Ar2 | Ph-NAr3Ar4 | Phenyl | 1-Naphthyl | Phenyl | Phenyl |
| 136 | NAr1Ar2 | Ph-NAr3Ar4 | Phenyl | 1-Naphthyl | Phenyl | 1-Naphthyl |
| 137 | NAr1Ar2 | Ph-NAr3Ar4 | Phenyl | 1-Naphthyl | Phenyl | 2-Naphthyl |
| 138 | NAr1Ar2 | Ph-NAr3Ar4 | Phenyl | 1-Naphthyl | Phenyl | m-Tolyl |
| 139 | NAr1Ar2 | Ph-NAr3Ar4 | Phenyl | 1-Naphthyl | Phenyl | tBuPhenyl |
| 140 | NAr1Ar2 | Ph-NAr3Ar4 | Phenyl | 1-Naphthyl | Phenyl | Phenanthrenyl |
| 141 | NAr1Ar2 | Ph-NAr3Ar4 | Phenyl | 1-Naphthyl | 1-Naphthyl | 1-Naphthyl |
| 142 | NAr1Ar2 | Ph-NAr3Ar4 | Phenyl | 1-Naphthyl | 2-Naphthyl | 2-Naphthyl |
| 143 | NAr1Ar2 | Ph-NAr3Ar4 | Phenyl | 1-Naphthyl | m-Tolyl | m-Tolyl |
| 144 | NAr1Ar2 | Ph-NAr3Ar4 | Phenyl | 1-Naphthyl | tBuPhenyl | tBuPhenyl |
| 145 | NAr1Ar2 | H | Phenyl | 2-Naphthyl | - | - |
| 146 | NAr1Ar2 | Phenyl | Phenyl | 2-Naphthyl | - | - |
| 147 | NAr1Ar2 | 1-Naphthyl | Phenyl | 2-Naphthyl | - | - |
| 148 | NAr1Ar2 | 2-Naphthyl | Phenyl | 2-Naphthyl | - | - |
| 149 | NAr1Ar2 | NAr3Ar4 | Phenyl | 2-Naphthyl | Phenyl | Phenyl |
| 150 | NAr1Ar2 | NAr3Ar4 | Phenyl | 2-Naphthyl | Phenyl | 1-Naphthyl |
| 151 | NAr1Ar2 | NAr3Ar4 | Phenyl | 2-Naphthyl | Phenyl | 2-Naphthyl |
| 152 | NAr1Ar2 | NAr3Ar4 | Phenyl | 2-Naphthyl | Phenyl | m-Tolyl |
| 153 | NAr1Ar2 | NAr3Ar4 | Phenyl | 2-Naphthyl | Phenyl | tBuPhenyl |
| 154 | NAr1Ar2 | NAr3Ar4 | Phenyl | 2-Naphthyl | Phenyl | Phenanthrenyl |
| 155 | NAr1Ar2 | NAr3Ar4 | Phenyl | 2-Naphthyl | 1-Naphthyl | 1-Naphthyl |
| 156 | NAr1Ar2 | NAr3Ar4 | Phenyl | 2-Naphthyl | 2-Naphthyl | 2-naphthyl |
| 157 | NAr1Ar2 | NAr3Ar4 | Phenyl | 2-Naphthyl | m-Tolyl | m-Tolyl |
| 158 | NAr1Ar2 | NAr3Ar4 | Phenyl | 2-Naphthyl | tBuPhenyl | tBuPhenyl |
| 159 | NAr1Ar2 | Ph-NAr3Ar4 | Phenyl | 2-Naphthyl | Phenyl | Phenyl |
| 160 | NAr1Ar2 | Ph-NAr3Ar4 | Phenyl | 2-Naphthyl | Phenyl | 1-Naphthyl |
| 161 | NAr1Ar2 | Ph-NAr3Ar4 | Phenyl | 2-Naphthyl | Phenyl | 2-Naphthyl |
| 162 | NAr1Ar2 | Ph-NAr3Ar4 | Phenyl | 2-Naphthyl | Phenyl | m-Tolyl |
| 163 | NAr1Ar2 | Ph-NAr3Ar4 | Phenyl | 2-Naphthyl | Phenyl | tBuPhenyl |
| 164 | NAr1Ar2 | Ph-NAr3Ar4 | Phenyl | 2-Naphthyl | Phenyl | Phenanthrenyl |
| 165 | NAr1Ar2 | Ph-NAr3Ar4 | Phenyl | 2-Naphthyl | 1-Naphthyl- | 1-Naphthyl |
| 166 | NAr1Ar2 | Ph-NAr3Ar4 | Phenyl | 2-Naphthyl | 2-Naphthyl | 2-Naphthyl |
| 167 | NAr1Ar2 | Ph-NAr3Ar4 | Phenyl | 2-Naphthyl | m-Tolyl | m-Tolyl |
| 168 | NAr1Ar2 | Ph-NAr3Ar4 | Phenyl | 2-Naphthyl | tBuPhenyl | tBuPhenyl |
| 169 | NAr1Ar2 | H | Phenyl | m-Tolyl | - | - |
| 170 | NAr1Ar2 | Phenyl | Phenyl | m-Tolyl | - | - |
| 171 | NAr1Ar2 | 1-Naphthyl | Phenyl | m-Tolyl | - | - |
| 172 | NAr1Ar2 | 2-Naphthyl | Phenyl | m-Tolyl | - | - |
| 173 | NAr1Ar2 | NAr3Ar4 | Phenyl | m-Tolyl | Phenyl | Phenyl |
| 174 | NAr1Ar2 | NAr3Ar4 | Phenyl | m-Tolyl | Phenyl | 1-Naphthyl |
| 175 | NAr1Ar2 | NAr3Ar4 | Phenyl | m-Tolyl | Phenyl | 2-Naphthyl |
| 176 | NAr1Ar2 | NAr3Ar4 | Phenyl | m-Tolyl | Phenyl | m-Tolyl |
| 177 | NAr1Ar2 | NAr3Ar4 | Phenyl | m-Tolyl | Phenyl | tBuPhenyl |
| 178 | NAr1Ar2 | NAr3Ar4 | Phenyl | m-Tolyl | Phenyl | Phenanthrenyl |
| 179 | NAr1Ar2 | NAr3Ar4 | Phenyl | m-Tolyl | 1-Naphthyl | 1-Naphthyl |
| 180 | NAr1Ar2 | NAr3Ar4 | Phenyl | m-Tolyl | 2-Naphthyl | 2-Naphthyl |
| 181 | NAr1Ar2 | NAr3Ar4 | Phenyl | m-Tolyl | m-Tolyl | m-Tolyl |
| 182 | NAr1Ar2 | NAr3Ar4 | Phenyl | m-Tolyl | tBuPhenyl | tBuPhenyl |
| 183 | NAr1Ar2 | Ph-NAr3Ar4 | Phenyl | m-Tolyl | Phenyl | Phenyl |
| 184 | NAr1Ar2 | Ph-NAr3Ar4 | Phenyl | m-Tolyl | Phenyl | 1-Naphthyl |
| 185 | NAr1Ar2 | Ph-NAr3Ar4 | Phenyl | m-Tolyl | Phenyl | 2-Naphthyl |
| 186 | NAr1Ar2 | Ph-NAr3Ar4 | Phenyl | m-Tolyl | Phenyl | m-Tolyl |
| 187 | NAr1Ar2 | Ph-NAr3Ar4 | Phenyl | m-Tolyl | Phenyl | tBuPhenyl |
| 188 | NAr1Ar2 | Ph-NAr3Ar4 | Phenyl | m-Tolyl | Phenyl | Phenanthrenyl |
| 189 | NAr1Ar2 | Ph-NAr3Ar4 | Phenyl | m-Tolyl | 1-Naphthyl | 1-Naphthyl |
| 190 | NAr1Ar2 | Ph-NAr3Ar4 | Phenyl | m-Tolyl | 2-Naphthyl | 2-Naphthyl |
| 191 | NAr1Ar2 | Ph-NAr3Ar4 | Phenyl | m-Tolyl | m-Tolyl | m-Tolyl |
| 192 | NAr1Ar2 | Ph-NAr3Ar4 | Phenyl | m-Tolyl | tBuPhenyl | tBuPhenyl |
| 193 | NAr1Ar2 | H | Phenyl | tBuPhenyl | - | - |
| 194 | NAr1Ar2 | Phenyl, | Phenyl | tBuPhenyl | - | - |
| 195 | NAr1 Ar2 | 1-Naphthyl | Phenyl | tBuPhenyl | - | - |
| 196 | NAr1 Ar2 | 2-Naphthyl | Phenyl | tBuPhenyl | - | - |
| 197 | NAr1Ar2 | NAr3Ar4 | Phenyl | tBuPhenyl | Phenyl, | Phenyl |
| 198 | NAr1Ar2 | NAr3Ar4 | Phenyl | tBuPhenyl | Phenyl | 1-Naphthyl |
| 199 | NAr1Ar2 | NAr3Ar4 | Phenyl, | tBuPhenyl | Phenyl | 2-Naphthvt |
| 200 | NAr1Ar2 | NAr3Ar4 | Phenyl | tBuPhenyl | Phenyl | m-Tolyl |
| 201 | NAr1Ar2 | NAr3Ar4 | Phenyl, | tBuPhenyl | Phenyl, | tBuPhenyl |
| 202 | NAr1Ar2 | NAr3Ar4 | Phenyl | tBuPhenyl | Phenyl | Phenanthrenyl |
| 203 | NAr1Ar2 | NAr3Ar4 | Phenyl | tBuPhenyl | 1-Naphthyl | 1-Naphthyl |
| 204 | NAr1Ar2 | NAr3Ar4 | Phenyl | tBuPhenyl | 2-Naphthyl | 2-Naphthyl |
| 205 | NAr1Ar2 | NAr3Ar4 | Phenyl | tBuPhenyl | m-Tolyl | m-Tolyl |
| 206 | NAr1Ar2 | NAr3Ar4 | Phenyl | tBuPhenyl | tBuPhenyl | tBuPhenyl |
| 207 | NAr1Ar2 | Ph-NAr3Ar4 | Phenyl, | tBuPhenyl | Phenyl | Phenyl |
| 208 | NAr1Ar2 | Ph-NAr3Ar4 | Phenyl | tBuPhenyl | Phenyl | 1-Naphthyl |
| 209 | NAr1Ar2 | Ph-NAr3Ar4 | Phenyl | tBuPhenyl | Phenyl | 2-Naphthyl |
| 210 | NAr1Ar2 | Ph-NAr3Ar4 | Phenyl | tBuPhenyl | Phenyl | m-Tolyl |
| 211 | NAr1Ar2 | Ph-NAr3Ar4 | Phenyl | tBuPhenyl | Phenyl | tBuPhenyl |
| 212 | NAr1Ar2 | Ph-NAr3Ar4 | Phenyl | tBuPhenyl | Phenyl | Phenanthrenyl |
| 213 | NAr1Ar2 | Ph-NAr3Ar4 | Phenyl | tBuPhenyl | 1-Naphthyl | 1-Naphthyl |
| 214 | NAr1Ar2 | Ph-NAr3Ar4 | Phenyl | tBuPhenyl | 2-Naphthyl | 2-Naphthyl |
| 215 | NAr1Ar2 | Ph-NAr3Ar4 | Phenyl | tBuPhenyl | m-Tolyl | m-Tolyl |
| 216 | NAr1Ar2 | Ph-NAr3Ar4 | Phenyl | tBuPhenyl | tBuPhenyl | tBuPhenyl |
| 217 | NAr1Ar2 | H | Phenyl | Phenanthrenyl | - | - |
| 218 | NAr1Ar2 | Phenyl | Phenyl | Phenanthrenyl | - | - |
| 219 | NAr1Ar2 | 1-Naphthyl | Phenyl | Phenanthrenyl | - | - |
| 220 | NAr1Ar2 | 2-Naphthyl | Phenyl | Phenanthrenyl | - | - |
| 221 | NAr1Ar2 | NAr3Ar4 | Phenyl | Phenanthrenyl | Phenyl | Phenyl |
| 222 | NAr1Ar2 | NAr3Ar4 | Phenyl | Phenanthrenyl | Phenyl | 1-Naphthyl |
| 223 | NAr1Ar2 | NAr3Ar4 | Phenyl | Phenanthrenyl | Phenyl | 2-Naphthyl |
| 224 | NAr1Ar2 | NAr3Ar4 | Phenyl | Phenanthrenyl | Phenyl | m-Tolyl |
| 225 | NAr1Ar2 | NAr3Ar4 | Phenyl | Phenanthrenyl | Phenyl | tBuPhenyl |
| 226 | NAr1Ar2 | NAr3Ar4 | Phenyl | Phenanthrenyl | Phenyl | Phenanthrenyl |
| 227 | NAr1Ar2 | NAr3Ar4 | Phenyl | Phenanthrenyl | 1-Naphthyl | 1-Naphthyl |
| 228 | NAr1Ar2 | NAr3Ar4 | Phenyl | Phenanthrenyl | 2-Naphthyl | 2-Naphthyl |
| 229 | NAr1Ar2 | NAr3Ar4 | Phenyl | Phenanthrenyl | m-Tolyl | m-Tolyl |
| 230 | NAr1Ar2 | NAr3Ar4 | Phenyl | Phenanthrenyl | tBuPhenyl | tBuPhenyl |
| 231 | NAr1Ar2 | Ph-NAr3Ar4 | Phenyl | Phenanthrenyl | Phenyl | Phenyl |
| 232 | NAr1Ar2 | Ph-NAr3Ar4 | Phenyl | Phenanthrenyl | Phenyl | 1-Naphthyl |
| 233 | NAr1Ar2 | Ph-NAr3Ar4 | Phenyl | Phenanthrenyl | Phenyl | 2-Naphthyl |
| 234 | NAr1Ar2 | Ph-NAr3Ar4 | Phenyl | Phenanthrenyl | Phenyl | M-Tolyl |
| 235 | NAr1Ar2 | Ph-NAr3Ar4 | Phenyl | Phenanthrenyl | Phenyl | tBuPhenyl |
| 236 | NAr1Ar2 | Ph-NAr3Ar4 | Phenyl | Phenanthrenyl | Phenyl | Phenanthrenyl |
| 237 | NAr1Ar2 | Ph-NAr3Ar4 | Phenyl | Phenanthrenyl | 1-Naphthyl | 1-Naphthyl |
| 238 | NAr1Ar2 | Ph-NAr3Ar4 | Phenyl | Phenanthrenyl | 2-Naphthyl | 2-Naphthyl |
| 239 | NAr1Ar2 | Ph-NAr3Ar4 | Phenyl | Phenanthrenyl | m-Tolyl | m-Tolyl |
| 240 | NAr1Ar2 | Ph-NAr3Ar4 | Phenyl | Phenanthrenyl | tBuPhenyl | tBuPhenyl |
| 241 | NAr1Ar2 | H | 1-Naphthyl | 1-Naphthyl | - | - |
| 242 | NAr1Ar2 | Phenyl | 1-Naphthyl | 1-Naphthyl | - | - |
| 243 | NAr1Ar2 | 1-Naphthyl | 1-Naphthyl | 1-Naphthyl | - | - |
| 244 | NAr1Ar2 | 2-Naphthyl | 1-Naphthyl | 1-Naphthyl | - | - |
| 245 | NAr1Ar2 | NAr3Ar4 | 1-Naphthyl | 1-Naphthyl | Phenyl | Phenyl |
| 246 | NAr1Ar2 | NAr3Ar4 | 1-Naphthyl | 1-Naphthyl | Phenyl | 1-Naphthyl |
| 247 | NAr1Ar2 | NAr3Ar4 | 1-Naphthyl | 1-Naphthyl | Phenyl | 2-Naphthyl |
| 248 | NAr1Ar2 | NAr3Ar4 | 1-Naphthyl | 1-Naphthyl | Phenyl | m-Tolyl |
| 249 | NAr1Ar2 | NAr3Ar4 | 1-Naphthyl | 1-Naphthyl | Phenyl | tBuPhenyl |
| 250 | NAr1Ar2 | NAr3Ar4 | 1-Naphthyl | 1-Naphthyl | Phenyl | Phenanthrenyl |
| 251 | NAr1Ar2 | NAr3Ar4 | 1-Naphthyl | 1-Naphthyl | 1-Naphthyl | 1-Naphthyl |
| 252 | NAr1Ar2 | NAr3Ar4 | 1-Naphthyl | 1-Naphthvl | 2-Naphthyl | 2-Naphthyl |
| 253 | NAr1Ar2 | NAr3Ar4 | 1-Naphthyl | 1-Naphthyl | m-Tolyl | m-Tolyl |
| 254 | NAr1Ar2 | NAr3Ar4 | 1-Naphthyl | 1-Naphthyl | tBuPhenyl | tBuPhenyl |
| 255 | NAr1Ar2 | Ph-NAr3Ar4 | 1-Naphthyl | 1-Naphthyl | Phenyl | Phenyl |
| 256 | NAr1Ar2 | Ph-NAr3Ar4 | 1-Naphthyl | 1-Naphthyl | Phenyl | 1-Naphthyl |
| 257 | NAr1Ar2 | Ph-NAr3Ar4 | 1-Naphthyl | 1-Naphthyl | Phenyl | 2-Naphthyl |
| 258 | NAr1Ar2 | Ph-NAr3Ar4 | 1-Naphthyl | 1-Naphthyl | Phenyl | m-Tolyl |
| 259 | NAr1Ar2 | Ph-NAr3Ar4 | 1-Naphthyl | 1-Naphthyl | Phenyl | tBuPhenyl |
| 260 | NAr1Ar2 | Ph-NAr3Ar4 | 1-Naphthyl | 1-Naphthyl | Phenyl | Phenanthrenyl |
| 261 | NAr1Ar2 | Ph-NAr3Ar4 | 1-Naphthyl | 1-Naphthyl | 1-Naphthyl | 1-Naphthyl |
| 262 | NAr1Ar2 | Ph-NAr3Ar4 | 1-Naphthyl | 1-Naphthyl | 2-Naphthyl | 2-Naphthyl |
| 263 | NAr1Ar2 | Ph-NAr3Ar4 | 1-Naphthyl | 1-Naphthyl | m-Tolyl | m-Tolyl |
| 264 | NAr1Ar2 | Ph-NAr3Ar4 | 1-Naphthyl | 1-Naphthyl | tBuPhenyl | tBuPhenyl |
| 265 | NAr1Ar2 | H | 2-Naphthyl | 2-Naphthyl | - | - |
| 266 | NAr1Ar2 | Phenyl | 2-Naphthyl | 2-Naphthyl | - | - |
| 267 | NAr1Ar2 | 1-Naphthyl | 2-Naphthyl | 2-Naphthyl | - | - |
| 268 | NAr1Ar2 | 2-Naphthyl | 2-Naphthyl | 2-Naphthyl | - | - |
| 269 | NAr1Ar2 | NAr3Ar4 | 2-Naphthyl | 2-Naphthyl | Phenyl | Phenyl |
| 270 | NAr1Ar2 | NAr3Ar4 | 2-Naphthyl | 2-Naphthyl | Phenyl | 1-Naphthyl |
| 271 | NAr1Ar2 | NAr3Ar4 | 2-Naphthyl | 2-Naphthyl | Phenyl | 2-Naphthyl |
| 272 | NAr1Ar2 | NAr3Ar4 | 2-Naphthyl | 2-Naphthyl | Phenyl | m-Tolyl |
| 273 | NAr1Ar2 | NAr3Ar4 | 2-Naphthyl | 2-Naphthyl | Phenyl | tBuPhenyl |
| 274 | NAr1Ar2 | NAr3Ar4 | 2-Naphthyl | 2-Naphthyl | Phenyl | Phenanthrenyl |
| 275 | NAr1Ar2 | NAr3Ar4 | 2-Naphthyl | 2-Naphthyl | 1-Naphthyl | 1-Naphthyl |
| 276 | NAr1Ar2 | NAr3Ar4 | 2-Naphthyl | 2-Naphthyl | 2-Naphthyl | 2-Naphthyl |
| 277 | NAr1Ar2 | NAr3Ar4 | 2-Naphthyl | 2-Naphthyl | m-Tolyl | m-Tolyl |
| 278 | NAr1Ar2 | NAr3Ar4 | 2-Naphthyl | 2-Naphthyl | tBuPhenyl | tBuPhenyl |
| 279 | NAr1Ar2 | Ph-NAr3Ar4 | 2-Naphthyl | 2-Naphthyl | Phenyl | Phenyl |
| 280 | NAr1Ar2 | Ph-NAr3Ar4 | 2-Naphthyl | 2-Naphthyl | Phenyl | 1-Naphthyl |
| 281 | NAr1Ar2 | Ph-NAr3Ar4 | 2-Naphthyl | 2-Naphthyl | Phenyl | 2-Naphthyl |
| 282 | NAr1Ar2 | Ph-NAr3Ar4 | 2-Naphthyl | 2-Naphthyl | Phenyl | m-Tolyl |
| 283 | NAr1Ar2 | Ph-NAr3Ar4 | 2-Naphthyl | 2-Naphthyl | Phenyl | tBuPhenyl |
| 284 | NAr1Ar2 | Ph-NAr3Ar4 | 2-Naphthyl | 2-Naphthyl | Phenyl | Phenanthrenyl |
| 285 | NAr1Ar2 | Ph-NAr3Ar4 | 2-Naphthyl | 2-Naphthyl | 1-Naphthyl | 1-Naphthyl |
| 286 | NAr1Ar2 | Ph-NAr3Ar4 | 2-Naphthyl | 2-Naphthyl | 2-Naphthyl | 2-Naphthyl |
| 287 | NAr1Ar2 | Ph-NAr3Ar4 | 2-Naphthyl | 2-Naphthyl | m-Tolyl | m-Tolyl |
| 288 | NAr1Ar2 | Ph-NAr3Ar4 | 2-Naphthyl | 2-Naphthyl | tBuPhenyl | tBuPhenyl |
| 289 | NAr1Ar2 | H | m-Tolyl | m-Tolyl | - | - |
| 290 | NAr1Ar2 | Phenyl | m-Tolyl | m-Tolyl | - | - |
| 291 | NAr1Ar2 | 1-Naphthyl | m-Tolyl | m-Tolyl | - | - |
| 292 | NAr1Ar2 | 2-Naphthyl | m-Tolyl | m-Tolyl | - | - |
| 293 | NAr1Ar2 | NAr3Ar4 | m-Tolyl | m-Tolyl | Phenyl | Phenyl |
| 294 | NAr1A2 | NAr3Ar4 | m-Tolyl | m-Tolyl | Phenyl | 1-Naphthyl |
| 295 | NAr1Ar2 | NAr3Ar4 | m-Tolyl | m-Tolyl | Phenyl | 2-Naphthyl |
| 296 | NAr1Ar2 | NAr3Ar4 | m-Tolyl | m-Tolyl | Phenyl | m-Tolyl |
| 297 | NAr1Ar2 | NAr3Ar4 | m-Tolyl | m-Tolyl | Phenyl | tBuPhenyl |
| 298 | NAr1Ar2 | NAr3Ar4 | m-Tolyl | m-Tolyl | Phenyl | Phenanthrenyl |
| 299 | NAr1Ar2 | NAr3Ar4 | m-Tolyl | m-Tolyl | 1-Naphthyl | 1-Naphthyl |
| 300 | NAr1Ar2 | NAr3Ar4 | m-Tolyl | m-Tolyl | 2-Naphthyl | 2-Naphthyl |
| 301 | NAr1Ar2 | NAr3Ar4 | m-Tolyl | m-Tolyl | m-Tolyl | m-Tolyl |
| 302 | NAr1Ar2 | NAr3Ar4 | m-Tolyl | m-Tolyl | tBuPhenyl | tBuPhenyl |
| 303 | NAr1Ar2 | Ph-NAr3Ar4 | m-Tolyl | m-Tolyl | Phenyl | Phenyl |
| 304 | NAr1 Ar2 | Ph-NAr3Ar4 | m-Tolyl | m-Tolyl | Phenyl | 1-Naphthyl |
| 305 | NAr1Ar2 | Ph-NAr3Ar4 | m-Tolyl | m-Tolyl | Phenyl | 2-Naphthyl |
| 306 | NAr1Ar2 | Ph-NAr3Ar4 | m-Tolyl | m-Tolyl | Phenyl | m-Tolyl |
| 307 | NAr1Ar2 | Ph-NAr3Ar4 | m-Tolyl | m-Tolyl | Phenyl | tBuPhenyl |
| 308 | NAr1Ar2 | Ph-NAr3Ar4 | m-Tolyl | m-Tolyl | Phenyl | Phenanthrenyl |
| 309 | NAr1Ar2 | Ph-NAr3Ar4 | m-Tolyl | m-Tolyl | 1-Naphthyl | 1-Naphthyl |
| 310 | NAr1Ar2 | Ph-NAr3Ar4 | m-Tolyl | m-Tolyl | 2-Naphthyl | 2-Naphthyl |
| 311 | NAr1Ar2 | Ph-NAr3Ar4 | m-Tolyl | m-Tolyl | m-Tolyl | m-Tolyl |
| 312 | NAr1Ar2 | Ph-NAr3Ar4 | m-Tolyl | m-Tolyl | tBuPhenyl | tBuPhenyl |
| 313 | NAr1Ar2 | H | tBuPhenyl | tBuPhenyl | - | - |
| 314 | NAr1Ar2 | Phenyl | tBuPhenyl | tBuPhenyl | - | - |
| 315 | NAr1Ar2 | 1-Naphthyl | tBuPhenyl | tBuPhenyl | - | - |
| 316 | NAr1Ar2 | 2-Naphthyl | tBuPhenyl | tBuPhenyl | - | - |
| 317 | NAr1Ar2 | NAr3Ar4 | tBuPhenyl | tBuPhenyl | Phenyl | Phenyl |
| 318 | NAr1Ar2 | NAr3Ar4 | tBuPhenyl | tBuPhenyl | Phenyl | 1-Naphthyl |
| 319 | NAr1Ar2 | NAr3Ar4 | tBuPhenyl | tBuPhenyl | Phenyl | 2-Naphthyl |
| 320 | NAr1Ar2 | NAr3Ar4 | tBuPhenyl | tBuPhenyl | Phenyl | m-Tolyl |
| 321 | NAr1Ar2 | NAr3Ar4 | tBuPhenyl | tBuPhenyl | Phenyl | tBuPhenyl |
| 322 | NAr1Ar2 | NAr3Ar4 | tBuPhenyl | tBuPhenyl | Phenyl | Phenanthrenyl |
| 323 | NAr1Ar2 | NAr3Ar4 | tBuPhenyl | tBuPhenyl | 1-Naphthyl | 1-naphthyl |
| 324 | NAr1Ar2 | NAr3Ar4 | tBuPhenyl | tBuPhenyl | 2-Naphthyl | 2-Naphthyl |
| 325 | NAr1Ar2 | NAr3Ar4 | tBuPhenyl | tBuPhenyl | m-Tolyl | m-Tolyl |
| 326 | NAr1Ar2 | NAr3Ar4 | tBuPhenyl | tBuPhenyl | tBuPhenyl | tBuPhenyl |
| 327 | NAr1Ar2 | Ph-NAr3Ar4 | tBuPhenyl | tBuPhenyl | Phenyl | Phenyl |
| 328 | NAr1Ar2 | Ph-NAr3Ar4 | tBuPhenyl | tBuPhenyl | Phenyl | 1-Naphthvl |
| 329 | NAr1Ar2 | Ph-NAr3Ar4 | tBuPhenyl | tBuPhenyl | Phenyl | 2-Naphthyl |
| 330 | NAr1Ar2 | Ph-NAr3Ar4 | tBuPhenyl | tBuPhenyl | Phenyl | m-Tolyl |
| 331 | NAr1Ar2 | Ph-NAr3Ar4 | tBuPhenyl | tBuPhenyl | Phenyl | tBuPhenyl |
| 332 | NAr1Ar2 | Ph-NAr3Ar4 | tBuPhenyl | tBuPhenyl | Phenyl | Phenanthrenyl |
| 333 | NAr1Ar2 | Ph-NAr3Ar4 | tBuPhenyl | tBuPhenyl | 1-Naphthyl | 1-Naphthyl |
| 334 | NAr1Ar2 | Ph-NAr3Ar4 | tBuPhenyl | tBuPhenyl | 2-Naphthyl | 2-Naphthyl |
| 335 | NAr1Ar2 | Ph-NAr3Ar4 | tBuPhenyl | tBuPhenyl | m-Tolyl | m-Tolyl |
| 336 | NAr1Ar2 | Ph-NAr3Ar4 | tBuPhenyl | tBuPhenyl | tBuPhenyl | tBuPhenyl |
| 337 | Ph-NAr1 Ar2 | H | Phenyl | Phenyl | - | - |
| 338 | Ph-NAr1Ar2 | Phenyl | Phenyl | Phenyl | - | - |
| 339 | Ph-NAr1 Ar2 | 1-Naphthyl | Phenyl | Phenyl | - | - |
| 340 | Ph-NAr1 Ar2 | 2-Naphthyl | Phenyl | Phenyl | - | - |
| 341 | Ph-NAr1Ar2 | NAr3Ar4 | Phenyl | Phenyl | Phenyl | Phenyl |
| 342 | Ph-NAr1 Ar2 | NAr3Ar4 | Phenyl | Phenyl | Phenyl | 1-Naphthyl |
| 343 | Ph-NAr1Ar2 | NAr3Ar4 | Phenyl | Phenyl | Phenyl | 2-Naphthyl |
| 344 | Ph-NAr1Ar2 | NAr3Ar4 | Phenyl | Phenyl | Phenyl | m-Tolyl |
| 345 | Ph-NAr1Ar2 | NAr3Ar4 | Phenyl | Phenyl | Phenyl | tBuPhenyl |
| 346 | Ph-NAr1Ar2 | NAr3Ar4 | Phenyl | Phenyl | Phenyl | Phenanthrenyl |
| 347 | Ph-NAr1Ar2 | NAr3Ar4 | Phenyl | Phenyl | 1-Naphthyl | 1-Naphthyl |
| 348 | Ph-NAr1Ar2 | NAr3Ar4 | Phenyl | Phenyl | 2-Naphthyl | 2-Naphthyl |
| 349 | Ph-NAr1 Ar2 | NAr3Ar4 | Phenyl | Phenyl | m-Tolyl | m-Tolyl |
| 350 | Ph-NAr1Ar2 | NAr3Ar4 | Phenyl | Phenyl | tBuPhenyl | tBuPhenyl |
| 351 | Ph-NAr1 Ar2 | Ph-NAr3Ar4 | Phenyl | Phenyl | Phenyl | Phenyl |
| 352 | Ph-NAr1Ar2 | Ph-NAr3Ar4 | Phenyl | Phenyl | Phenyl | 1-Naphthyl |
| 353 | Ph-NAr1Ar2 | Ph-NAr3Ar4 | Phenyl | Phenyl | Phenyl | 2-Naphthyl |
| 354 | Ph-NAr1 Ar2 | Ph-NAr3Ar4 | Phenyl | Phenyl | Phenyl | m-Tolyl |
| 355 | Ph-NAr1 Ar2 | Ph-NAr3Ar4 | Phenyl | Phenyl | Phenyl | tBuPhenyl |
| 356 | Ph-NAr1Ar2 | Ph-NAr3Ar4 | Phenyl | Phenyl | Phenyl | Phenanthrenyl |
| 357 | Ph-NAr1 Ar2 | Ph-NAr3Ar4 | Phenyl | Phenyl | 1-Naphthyl | 1-Naphthyl |
| 358 | Ph-NAr1Ar2 | Ph-NAr3Ar4 | Phenyl | Phenyl | 2-Naphthyl | 2-Naphthyl |
| 359 | Ph-NAr1Ar2 | Ph-NAr3Ar4 | Phenyl | Phenyl | m-Tolyl | m-Tolyl |
| 360 | Ph-NAr1 Ar2 | Ph-NAr3Ar4 | Phenyl | Phenyl | tBuPhenyl | tBuPhenyl |
| 361 | Ph-NAr1Ar2 | H | Phenyl | 1-Naphthyl | - | - |
| 362 | Ph-NAr1Ar2 | Phenyl | Phenyl | 1-Naphthyl | - | - |
| 363 | Ph-NAr1 Ar2 | 1-Naphthyl | Phenyl | 1-Naphthyl | - | - |
| 364 | Ph-NAr1 Ar2 | 2-Naphthyl | Phenyl | 1-Naphthyl | - | - |
| 365 | Ph-NAr1 Ar2 | NAr3Ar4 | Phenyl | 1-Naphthyl | Phenyl | Phenyl |
| 366 | Ph-NAr1 Ar2 | NAr3Ar4 | Phenyl | 1-Naphthyl | Phenyl | 1-Naphthyl |
| 367 | Ph-NAr1 Ar2 | NAr3Ar4 | Phenyl | 1-Naphthyl | Phenyl | 2-Naphthyl |
| 368 | Ph-NAr1 Ar2 | NAr3Ar4 | Phenyl | 1-Naphthyl | Phenyl | m-Tolyl |
| 369 | Ph-NAr1 Ar2 | NAr3Ar4 | Phenyl | 1-Naphthyl | Phenyl | tBuPhenyl |
| 370 | Ph-NAr1 Ar2 | NAr3Ar4 | Phenyl | 1-Naphthyl | Phenyl | Phenanthrenyl |
| 371 | Ph-NAr1Ar2 | NAr3Ar4 | Phenyl | 1-Naphthyl | 1-Naphthyl | 1-Naphthyl |
| 372 | Ph-NAr1Ar2 | NAr3Ar4 | Phenyl | 1-Naphthyl | 2-Naphthyl | 2-Naphthyl |
| 373 | Ph-NAr1Ar2 | NAr3Ar4 | Phenyl | 1-Naphthyl | m-Tolyl | m-Tolyl |
| 374 | Ph-NAr1 Ar2 | NAr3Ar4 | Phenyl | 1-Naphthyl | tBuPhenyl | tBuPhenyl |
| 375 | Ph-NAr1 Ar2 | Ph-NAr3Ar4 | Phenyl | 1-Naphthyl | Phenyl | Phenyl |
| 376 | Ph-NAr1Ar2 | Ph-NAr3Ar4 | Phenyl | 1-Naphthyl | Phenyl | 1-Naphthyl |
| 377 | Ph-NAr1 Ar2 | Ph-NAr3Ar4 | Phenyl | 1-Naphthyl | Phenyl | 2-Naphthyl |
| 378 | Ph-NAr1 Ar2 | Ph-NAr3Ar4 | Phenyl | 1-Naphthyl | Phenyl | m-Tolyl |
| 379 | Ph-NAr1 Ar2 | Ph-NAr3Ar4 | Phenyl | 1-Naphthyl | Phenyl | tBuPhenyl |
| 380 | Ph-NAr1Ar2 | Ph-NAr3Ar4 | Phenyl | 1-Naphthyl | Phenyl | Phenanthrenyl |
| 381 | Ph-NAr1Ar2 | Ph-NAr3Ar4 | Phenyl | 1-Naphthyl | 1-Naphthyl | 1-Naphthyl |
| 382 | Ph-NAr1Ar2 | Ph-NAr3Ar4 | Phenyl | 1-Naphthyl | 2-Naphthyl | 2-Naphthyl |
| 383 | Ph-NAr1 Ar2 | Ph-NAr3Ar4 | Phenyl | 1-Naphthyl | m-Tolyl | m-Tolyl |
| 384 | Ph-NAr1Ar2 | Ph-NAr3Ar4 | Phenyl | 1-Naphthyl | tBuPhenyl | tBuPhenyl |
| 385 | Ph-NAr1Ar2 | H | Phenyl | 2-Naphthyl | - | - |
| 386 | Ph-NAr1 Ar2 | Phenyl | Phenyl | 2-Naphthyl | - | - |
| 387 | Ph-NAr1 Ar2 | 1-Naphthyl | Phenyl | 2-Naphthyl | - | - |
| 388 | Ph-NAr1 Ar2 | 2-Naphthyl | Phenyl | 2-Naphthyl | - | - |
| 389 | Ph-NAr1 Ar2 | NAr3Ar4 | Phenyl | 2-Naphthyl | Phenyl | Phenyl |
| 390 | Ph-NAr1 Ar2 | NAr3Ar4 | Phenyl | 2-Naphthyl | Phenyl | 1-Naphthyl |
| 391 | Ph-NAr1 Ar2 | NAr3Ar4 | Phenyl | 2-Naphthyl | Phenyl | 2-Naphthyl |
| 392 | Ph-NAr1 Ar2 | NAr3Ar4 | Phenyl | 2-Naphthyl | Phenyl | m-Tolyl |
| 393 | Ph-NAr1 Ar2 | NAr3Ar4 | Phenyl | 2-Naphthyl | Phenyl | tBuPhenyl |
| 394 | Ph-NAr1Ar2 | NAr3Ar4 | Phenyl | 2-Naphthyl | Phenyl | Phenanthrenyl |
| 395 | Ph-NAr1Ar2 | NAr3Ar4 | Phenyl | 2-Naphthyl | 1-Naphthyl | 1-Naphthyl |
| 396 | Ph-NAr1 Ar2 | NAr3Ar4 | Phenyl | 2-Naphthyl | 2-Naphthyl | 2-Naphthyl |
| 397 | Ph-NAr1Ar2 | NAr3Ar4 | Phenyl | 2-Naphthyl | m-Tolyl | m-Tolyl |
| 398 | Ph-NAr1Ar2 | NAr3Ar4 | Phenyl | 2-Naphthyl | tBuPhenyl | tBuPhenyl |
| 399 | Ph-NAr1 Ar2 | Ph-NAr3Ar4 | Phenyl | 2-Naphthyl | Phenyl | Phenyl |
| 400 | Ph-NAr1Ar2 | Ph-NAr3Ar4 | Phenyl | 2-Naphthyl | Phenyl | 1-Naphthyl |
| 401 | Ph-NAr1Ar2 | Ph-NAr3Ar4 | Phenyl | 2-Naphthyl | Phenyl | 2-Naphthyl |
| 402 | Ph-NAr1 Ar2 | Ph-NAr3Ar4 | Phenyl | 2-Naphthyl | Phenyl | m-Tolyl |
| 403 | Ph-NAr1 Ar2 | Ph-NAr3Ar4 | Phenyl | 2-Naphthyl | Phenyl | tBuPhenyl |
| 404 | Ph-NAr1Ar2 | Ph-NAr3Ar4 | Phenyl | 2-Naphthyl | Phenyl | Phenanthrenyl |
| 405 | Ph-NAr1 Ar2 | Ph-NAr3Ar4 | Phenyl | 2-Naphthyl | 1-Naphthyl | 1-Naphthyl |
| 406 | Ph-NAr1Ar2 | Ph-NAr3Ar4 | Phenyl | 2-Naphthyl | 2-Naphthyl | 2-Naphthyl |
| 407 | Ph-NAr1 Ar2 | Ph-NAr3Ar4 | Phenyl | 2-Naphthyl | m-Tolyl | m-Tolyl |
| 408 | Ph-NAr1Ar2 | Ph-NAr3Ar4 | Phenyl | 2-Naphthyl | tBuPhenyl | tBuPhenyl |
| 409 | Ph-NAr1Ar2 | H | Phenyl | m-Tolyl | - | - |
| 410 | Ph-NAr1 Ar2 | Phenyl | Phenyl | m-Tolyl | - | - |
| 411 | Ph-NAr1Ar2 | 1-Naphthyl | Phenyl | m-Tolyl | - | - |
| 412 | Ph-NAr1Ar2 | 2-Naphthyl | Phenyl | m-Tolyl | - | - |
| 413 | Ph-NAr1 Ar2 | NAr3Ar4 | Phenyl | m-Tolyl | Phenyl | Phenyl |
| 414 | Ph-NAr1Ar2 | NAr3Ar4 | Phenyl | m-Tolyl | Phenyl | 1-Naphthyl |
| 415 | Ph-NAr1 Ar2 | NAr3Ar4 | Phenyl | m-Tolyl | Phenyl | 2-Naphthyl |
| 416 | Ph-NAr1 Ar2 | NAr3Ar4 | Phenyl | m-Tolyl | Phenyl | m-Tolyl |
| 417 | Ph-NAr1 Ar2 | NAr3Ar4 | Phenyl | m-Tolyl | Phenyl | tBuPhenyl |
| 418 | Ph-NAr1 Ar2 | NAr3Ar4 | Phenyl | m-Tolyl | Phenyl | Phenanthrenyl |
| 419 | Ph-NAr1 Ar2 | NAr3Ar4 | Phenyl | m-Tolyl | 1-Naphthyl | 1-Naphthyl |
| 420 | Ph-NAr1 Ar2 | NAr3Ar4 | Phenyl | m-Tolyl | 2-Naphthyl | 2-Naphthyl |
| 421 | Ph-NAr1 Ar2 | NAr3Ar4 | Phenyl | m-Tolyl | m-Tolyl | m-Tolyl |
| 422 | Ph-NAr1 Ar2 | NAr3Ar4 | Phenyl | m-Tolyl | tBuPhenyl | tBuPhenyl |
| 423 | Ph-NAr1 Ar2 | Ph-NAr3Ar4 | Phenyl | m-Tolyl | Phenyl | Phenyl |
| 424 | Ph-NAr1 Ar2 | Ph-NAr3Ar4 | Phenyl | m-Tolyl | Phenyl | 1-Naphthyl |
| 425 | Ph-NAr1Ar2 | Ph-NAr3Ar4 | Phenyl | m-Tolyl | Phenyl | 2-Naphthyl |
| 426 | Ph-NAr1 Ar2 | Ph-NAr3Ar4 | Phenyl | m-Tolyl | Phenyl | m-Tolyl |
| 427 | Ph-NAr1Ar2 | Ph-NAr3Ar4 | Phenyl | m-Tolyl | Phenyl | tBuPhenyl |
| 428 | Ph-NAr1Ar2 | Ph-NAr3Ar4 | Phenyl | m-Totyl | Phenyl | Phenanthrenyl |
| 429 | Ph-NAr1 Ar2 | Ph-NAr3Ar4 | Phenyl | m-Tolyl | 1-Naphthyl | 1-Naphthyl |
| 430 | Ph-NAr1Ar2 | Ph-NAr3Ar4 | Phenyl | m-Tolyl | 2-Naphthyl | 2-Naphthyl |
| 431 | Ph-NAr1 Ar2 | Ph-NAr3Ar4 | Phenyl | m-Tolyl | m-Tolyl | m-Tolyl |
| 432 | Ph-NAr1Ar2 | Ph-NAr3Ar4 | Phenyl | m-Tolyl | tBuPhenyl | tBuPhenyl |
| 433 | Ph-NAr1Ar2 | H | Phenyl | tBuPhenyl | - | - |
| 434 | Ph-NAr1Ar2 | Phenyl | Phenyl | tBuPhenyl | - | - |
| 435 | Ph-NAr1 Ar2 | 1-Naphthyl | Phenyl | tBuPhenyl | - | - |
| 436 | Ph-NAr1 Ar2 | 2-Naphthyl | Phenyl | tBuPhenyl | - | - |
| 437 | Ph-NAr1Ar2 | NAr3Ar4 | Phenyl | tBuPhenyl | Phenyl | Phenyl |
| 438 | Ph-NAr1 Ar2 | NAr3Ar4 | Phenyl | tBuPhenyl | Phenyl | 1-Naphthyl |
| 439 | Ph-NAr1Ar2 | NAr3Ar4 | Phenyl | tBuPhenyl | Phenyl | 2-Naphthyl |
| 440 | Ph-NAr1 Ar2 | NAr3Ar4 | Phenyl | tBuPhenyl | Phenyl | m-Tolyl |
| 441 | Ph-NAr1Ar2 | NAr3Ar4 | Phenyl | tBuPhenyl | Phenyl | tBuPhenyl |
| 442 | Ph-NAr1 Ar2 | NAr3Ar4 | Phenyl | tBuPhenyl | Phenyl | Phenanthrenyl |
| 443 | Ph-NAr1 Ar2 | NAr3Ar4 | Phenyl | tBuPhenyl | 1-Naphthyl | 1-Naphthyl- |
| 444 | Ph-NAr1 Ar2 | NAr3Ar4 | Phenyl | tBuPhenyl | 2-Naphthyl | 2-Naphthyl |
| 445 | Ph-NAr1 Ar2 | NAr3Ar4 | Phenyl | tBuPhenyl | m-Tolyl | m-Tolyl |
| 446 | Ph-NAr1 Ar2 | NAr3Ar4 | Phenyl | tBuPhenyl | tBuPhenyl | tBuPhenyl |
| 447 | Ph-NAr1 Ar2 | Ph-NAr3Ar4 | Phenyl | tBuPhenyl | Phenyl | Phenyl |
| 448 | Ph-NAr1 Ar2 | Ph-NAr3Ar4 | Phenyl | tBuPhenyl | Phenyl | 1-Naphthyl |
| 449 | Ph-NAr1 Ar2 | Ph-NAr3Ar4 | Phenyl | tBuPhenyl | Phenyl | 2-Naphthyl |
| 450 | Ph-NAr1 Ar2 | Ph-NAr3Ar4 | Phenyl | tBuPhenyl | Phenyl | m-Tolyl |
| 451 | Ph-NAr1 Ar2 | Ph-NAr3Ar4 | Phenyl | tBuPhenyl | Phenyl | tBuPhenyl |
| 452 | Ph-NAr1 Ar2 | Ph-NAr3Ar4 | Phenyl | tBuPhenyl | Phenyl | Phenanthrenyl |
| 453 | Ph-NAr1 Ar2 | Ph-NAr3Ar4 | Phenyl | tBuPhenyl | 1-Naphthyl | 1-Naphthyl |
| 454 | Ph-NAr1 Ar2 | Ph-NAr3Ar4 | Phenyl | tBuPhenyl | 2-Naphthyl | 2-Naphthyl |
| 455 | Ph-NAr1 Ar2 | Ph-NAr3Ar4 | Phenyl | tBuPhenyl | m-Tolyl | m-Tolyl |
| 456 | Ph-NAr1 Ar2 | Ph-NAr3Ar4 | Phenyl | tBuPhenyl | tBuPhenyl | tBuPhenyl |
| 457 | Ph-NAr1 Ar2 | H | Phenyl | Phenanthrenyl | - | - |
| 458 | Ph-NAr1Ar2 | Phenyl | Phenyl | Phenanthrenyl | - | - |
| 459 | Ph-NAr1 Ar2 | 1-Naphthyl | Phenyl | Phenanthrenyl | - | - |
| 460 | Ph-NAr1 Ar2 | 2-Naphthyl | Phenyl | Phenanthrenyl | - | - |
| 461 | Ph-NAr1Ar2 | NAr3Ar4 | Phenyl | Phenanthrenyl | Phenyl | Phenyl |
| 462 | Ph-NAr1 Ar2 | NAr3Ar4 | Phenyl | Phenanthrenyl | Phenyl | 1-Naphthyl |
| 463 | Ph-NAr1 Ar2 | NAr3Ar4 | Phenyl | Phenanthrenyl | Phenyl | 2-Naphthyl |
| 464 | Ph-NAr1Ar2 | NAr3Ar4 | Phenyl | Phenanthrenyl | Phenyl | m-Tolyl |
| 465 | Ph-NAr1Ar2 | NAr3Ar4 | Phenyl | Phenanthrenyl | Phenyl | tBuPhenyl |
| 466 | Ph-NAr1 Ar2 | NAr3Ar4 | Phenyl | Phenanthrenyl | Phenyl | Phenanthrenyl |
| 467 | Ph-NAr1Ar2 | NAr3Ar4 | Phenyl | Phenanthrenyl | 1-Naphthyl | 1-Naphthyl |
| 468 | Ph-NAr1 Ar2 | NAr3Ar4 | Phenyl | Phenanthrenyl | 2-Naphthyl | 2-Naphthyl |
| 469 | Ph-NAr1Ar2 | NAr3Ar4 | Phenyl | Phenanthrenyl | m-Tolyl | m-Tolyl |
| 470 | Ph-NAr1Ar2 | NAr3Ar4 | Phenyl | Phenanthrenyl | tBuPhenyl | tBuPhenyl |
| 471 | Ph-NAr1Ar2 | Ph-NAr3Ar4 | Phenyl | Phenanthrenyl | Phenyl | Phenyl |
| 472 | Ph-NAr1Ar2 | Ph-NAr3Ar4 | Phenyl | Phenanthrenyl | Phenyl | 1-Naphthyl |
| 473 | Ph-NAr1Ar2 | Ph-NAr3Ar4 | Phenyl | Phenanthrenyl | Phenyl | 2-Naphthyl |
| 474 | Ph-NAr1 Ar2 | Ph-NAr3Ar4 | Phenyl | Phenanthrenyl | Phenyl | m-Tolyl |
| 475 | Ph-NAr1Ar2 | Ph-NAr3Ar4 | Phenyl | Phenanthrenyl | Phenyl | tBuPhenyl |
| 476 | Ph-NAr1Ar2 | Ph-NAr3Ar4 | Phenyl | Phenanthrenyl | Phenyl | Phenanthrenyl |
| 477 | Ph-NAr1Ar2 | Ph-NAr3Ar4 | Phenyl | Phenanthrenyl | 1-Naphthyl | 1-Naphthyl |
| 478 | Ph-NAr1Ar2 | Ph-NAr3Ar4 | Phenyl | Phenanthrenyl | 2-Naphthyl | 2-Naphthyl |
| 479 | Ph-NAr1 Ar2 | Ph-NAr3Ar4 | Phenyl | Phenanthrenyl | m-Tolyl | m-Tolyl |
| 480 | Ph-NAr1Ar2 | Ph-NAr3Ar4 | Phenyl | Phenanthrenyl | tBuPhenyl | tBuPhenyl |
| 481 | Ph-NAr1 Ar2 | H | 1-Naphthyl | 1-Naphthyl | - | - |
| 482 | Ph-NAr1Ar2 | Phenyl | 1-Naphthyl | 1-Naphthyl | - | - |
| 483 | Ph-NAr1Ar2 | 1-Naphthyl | 1-Naphthyl | 1-Naphthyl | - | - |
| 484 | Ph-NAr1 Ar2 | 2-Naphthyl | 1-Naphthyl | 1-Naphthyl | - | - |
| 485 | Ph-NAr1Ar2 | NAr3Ar4 | 1-Naphthyl | 1-Naphthyl | Phenyl | Phenyl |
| 486 | Ph-NAr1Ar2 | NAr3Ar4 | 1-Naphthyl | 1-Naphthyl | Phenyl | 1-Naphthyl |
| 487 | Ph-NAr1 Ar2 | NAr3Ar4 | 1-Naphthyl | 1-Naphthyl | Phenyl | 2-Naphthyl |
| 488 | Ph-NAr1Ar2 | NAr3Ar4 | 1-Naphthyl | 1-Naphthyl | Phenyl | m-Tolyl |
| 489 | Ph-NAr1 Ar2 | NAr3Ar4 | 1-Naphthyl | 1-Naphthyl | Phenyl | tBuPhenyl |
| 490 | Ph-NAr1Ar2 | NAr3Ar4 | 1-Naphthyl | 1-Naphthyl | Phenyl | Phenanthrenyl |
| 491 | Ph-NAr1Ar2 | NAr3Ar4 | 1-Naphthyl | 1-Naphthyl | 1-Naphthyl | 1-Naphthyl |
| 492 | Ph-NAr1 Ar2 | NAr3Ar4 | 1-Naphthyl | 1-Naphthyl | 2-Naphthyl | 2-Naphthyl |
| 493 | Ph-NAr1Ar2 | NAr3Ar4 | 1-Naphthyl | 1-Naphthyl | m-Tolyl | m-Tolyl |
| 494 | Ph-NAr1Ar2 | NAr3Ar4 | 1-Naphthyl | 1-Naphthyl | tBuPhenyl | tBuPhenyl |
| 495 | Ph-NAr1Ar2 | Ph-NAr3Ar4 | 1-Naphthyl | 1-Naphthyl | Phenyl | Phenyl |
| 496 | Ph-NAr1 Ar2 | Ph-NAr3Ar4 | 1-Naphthyl | 1-Naphthyl | Phenyl | 1-Naphthyl |
| 497 | Ph-NAr1Ar2 | Ph-NAr3Ar4 | 1-Naphthyl | 1-Naphthyl | Phenyl | 2-Naphthyl |
| 498 | Ph-NAr1 Ar2 | Ph-NAr3Ar4 | 1-Naphthyl | 1-Naphthyl | Phenyl | m-Totyl |
| 499 | Ph-NAr1 Ar2 | Ph-NAr3Ar4 | 1-Naphthyl | 1-Naphthyl | Phenyl | tBuPhenyl |
| 500 | Ph-NAr1 Ar2 | Ph-NAr3Ar4 | 1-Naphthyl | 1-Naphthyl | Phenyl | Phenanthrenyl |
| 501 | Ph-NAr1Ar2 | Ph-NAr3Ar4 | 1-Naphthyl | 1-Naphthyl | 1-Naphthyl | 1-Naphthyl |
| 502 | Ph-NAr1Ar2 | Ph-NAr3Ar4 | 1-Naphthyl | 1-Naphthyl | 2-Naphthyl | 2-Naphthyl |
| 503 | Ph-NAr1 Ar2 | Ph-NAr3Ar4 | 1-Naphthyl | 1-Naphthyl | m-Tolyl | m-Tolyl |
| 504 | Ph-NAr1 Ar2 | Ph-NAr3Ar4 | 1-Naphthyl | 1-Naphthyl | tBuPhenyl | tBuPhenyl |
| 505 | Ph-NAr1Ar2 | H | 2-Naphthyl | 2-Naphthyl | - | - |
| 506 | Ph-NAr1Ar2 | Phenyl | 2-Naphthyl | 2-Naphthyl | - | - |
| 507 | Ph-NAr1 Ar2 | 1-Naphthyl | 2-Naphthyl | 2-Naphthyl | - | - |
| 508 | Ph-NAr1 Ar2 | 2-naphthyl | 2-Naphthyl | 2-Naphthyl | - | - |
| 509 | Ph-NAr1 Ar2 | NAr3Ar4 | 2-Naphthyl | 2-Naphthyl | Phenyl | Phenyl |
| 510 | Ph-NAr1 Ar2 | NAr3Ar4 | 2-Naphthyl | 2-Naphthyl | Phenyl | 1-Naphthyl |
| 511 | Ph-NAr1 Ar2 | NAr3Ar4 | 2-Naphthyl | 2-Naphthyl | Phenyl | 2-Naphthyl |
| 512 | Ph-NAr1Ar2 | NAr3Ar4 | 2-Naphthyl | 2-Naphthyl | Phenyl | m-Tolyl |
| 513 | Ph-NAr1 Ar2 | NAr3Ar4 | 2-Naphthyl | 2-Naphthyl | Phenyl | tBuPhenyl |
| 514 | Ph-NAr1Ar2 | NAr3Ar4 | 2-Naphthyl | 2-Naphthyl | Phenyl | Phenanthrenyl |
| 515 | Ph-NAr1 Ar2 | NAr3Ar4 | 2-Naphthyl | 2-Naphthyl | 1-Naphthyl | 1-Naphthyl |
| 516 | Ph-NAr1 Ar2 | NAr3Ar4 | 2-Naphthyl | 2-Naphthyl | 2-Naphthyl | 2-Naphthyl |
| 517 | Ph-NAr1Ar2 | NAr3Ar4 | 2-Naphthyl | 2-Naphthyl | m-Tolyl | m-Tolyl |
| 518 | Ph-NAr1Ar2 | NAr3Ar4 | 2-Naphthyl | 2-Naphthyl | tBuPhenyl | tBuPhenyl |
| 519 | Ph-NAr1 Ar2 | Ph-NAr3Ar4 | 2-Naphthyl | 2-Naphthyl | Phenyl | Phenyl |
| 520 | Ph-NAr1Ar2 | Ph-NAr3Ar4 | 2-Naphthyl | 2-Naphthyl | Phenyl | 1-Naphthyl |
| 521 | Ph-NAr1 Ar2 | Ph-NAr3Ar4 | 2-Naphthyl | 2-Naphthyl | Phenyl | 2-Naphthyl |
| 522 | Ph-NAr1 Ar2 | Ph-NAr3Ar4 | 2-Naphthyl | 2-Naphthyl | Phenyl | m-Tolyl |
| 523 | Ph-NAr1Ar2 | Ph-NAr3Ar4 | 2-Naphthyl | 2-Naphthyl | Phenyl | tBuPhenyl |
| 524 | Ph-NAr1Ar2 | Ph-NAr3Ar4 | 2-Naphthyl | 2-Naphthyl | Phenyl | Phenanthrenyl |
| 525 | Ph-NAr1 Ar2 | Ph-NAr3Ar4 | 2-Naphthyl | 2-Naphthyl | 1-Naphthyl | 1-Naphthyl |
| 526 | Ph-NAr1Ar2 | Ph-NAr3Ar4 | 2-Naphthyl | 2-Naphthyl | 2-Naphthyl | 2-Naphthyl |
| 527 | Ph-NAr1 Ar2 | Ph-NAr3Ar4 | 2-Naphthyl | 2-Naphthyl | m-Tolyl | m-Tolyl |
| 528 | Ph-NAr1 Ar2 | Ph-NAr3Ar4 | 2-Naphthyl | 2-Naphthyl | tBuPhenyl | tBuPhenyl |
| 529 | Ph-NAr1 Ar2 | H | m-Tolyl | m-Tolyl | - | - |
| 530 | Ph-NAr1Ar2 | Phenyl | m-Tolyl | m-Tolyl | - | - |
| 531 | Ph-NAr1Ar2 | 1-Naphthyl | m-Tolyl | m-Tolyl | - | - |
| 532 | Ph-NAr1 Ar2 | 2-Naphthyl | m-Tolyl | m-Tolyl | - | - |
| 533 | Ph-NAr1 Ar2 | NAr3Ar4 | m-Tolyl | m-Tolyl | Phenyl | Phenyl |
| 534 | Ph-NAr1 Ar2 | NAr3Ar4 | m-Tolyl | m-Tolyl | Phenyl | 1-Naphthyl |
| 535 | Ph-NAr1Ar2 | NAr3Ar4 | m-Totyl | m-Tolyl | Phenyl | 2-Naphthyl |
| 536 | Ph-NAr1 Ar2 | NAr3Ar4 | m-Tolyl | m-Tolyl | Phenyl | m-Tolyl |
| 537 | Ph-NAr1 Ar2 | NAr3Ar4 | m-Tolyl | m-Tolyl | Phenyl | tBuPhenyl |
| 538 | Ph-NAr1 Ar2 | NAr3Ar4 | m-Tolyl | m-Tolyl | Phenyl | Phenanthrenyl |
| 539 | Ph-NAr1Ar2 | NAr3Ar4 | m-Tolyl | m-Tolyl | 1-Naphthyl | 1-Naphthyl |
| 540 | Ph-NAr1 Ar2 | NAr3Ar4 | m-Tolyl | m-Tolyl | 2-Naphthyl | 2-Naphthyl |
| 541 | Ph-NAr1 Ar2 | NAr3Ar4 | m-Tolyl | m-Tolyl | m-Tolyl | m-Tolyl |
| 542 | Ph-NAr1 Ar2 | NAr3Ar4 | m-Tolyl | m-Tolyl | tBuPhenyl | tBuPhenyl |
| 543 | Ph-NAr1Ar2 | Ph-NAr3Ar4 | m-Tolyl | m-Tolyl | Phenyl | Phenyl |
| 544 | Ph-NAr1Ar2 | Ph-NAr3Ar4 | m-Tolyl | m-Tolyl | Phenyl | 1-Naphthyl |
| 545 | Ph-NAr1Ar2 | Ph-NAr3Ar4 | m-Tolyl | m-Tolyl | Phenyl | 2-Naphthyl |
| 546 | Ph-NAr1 Ar2 | Ph-NAr3Ar4 | m-Tolyl | m-Tolyl | Phenyl | m-Tolyl |
| 547 | Ph-NAr1Ar2 | Ph-NAr3Ar4 | m-Tolyl | m-Tolyl | Phenyl | tBuPhenyl |
| 548 | Ph-NAr1 Ar2 | Ph-NAr3Ar4 | m-Tolyl | m-Tolyl | Phenyl | Phenanthrenyl |
| 549 | Ph-NAr1Ar2 | Ph-NAr3Ar4 | m-Tolyl | m-Tolyl | 1-Naphthyl | 1-Naphthyl |
| 550 | Ph-NAr1 Ar2 | Ph-NAr3Ar4 | m-Tolyl | m-Tolyl | 2-Naphthyl | 2-Naphthyl |
| 551 | Ph-NAr1Ar2 | Ph-NAr3Ar4 | m-Tolyl | m-Tolyl | m-Tolyl | m-Tolyl |
| 552 | Ph-NAr1Ar2 | Ph-NAr3Ar4 | m-Tolyl | m-Tolyl | tBuPhenyl | tBuPhenyl |
| 553 | Ph-NAr1Ar2 | H | tBuPhenyl | tBuPhenyl | - | - |
| 554 | Ph-NAr1Ar2 | Phenyl | tBuPhenyl | tBuPhenyl | - | - |
| 555 | Ph-NAr1Ar2 | 1-Naphthyl | tBuPhenyl | tBuPhenyl | - | - |
| 556 | Ph-NAr1Ar2 | 2-Naphthyl | tBuPhenyl | tBuPhenyl | - | - |
| 557 | Ph-NAr1Ar2 | NAr3Ar4 | tBuPhenyl | tBuPhenyl | Phenyl | Phenyl |
| 558 | Ph-NAr1Ar2 | NAr3Ar4 | tBuPhenyl | tBuPhenyl | Phenyl | 1-Naphthyl |
| 559 | Ph-NAr1Ar2 | NAr3Ar4 | tBuPhenyl | tBuPhenyl | Phenyl | 2-Naphthyl |
| 560 | Ph-NAr1Ar2 | NAr3Ar4 | tBuPhenyl | tBuPhenyl | Phenyl | m-Tolyl |
| 561 | Ph-NAr1Ar2 | NAr3Ar4 | tBuPhenyl | tBuPhenyl | Phenyl | tBuPhenyl |
| 562 | Ph-NAr1Ar2 | NAr3Ar4 | tBuPhenyl | tBuPhenyl | Phenyl | Phenanthrenyl |
| 563 | Ph-NAr1Ar2 | NAr3Ar4 | tBuPhenyl | tBuPhenyl | 1-Naphthyl | 1-Naphthyl |
| 564 | Ph-NAr1Ar2 | NAr3Ar4 | tBuPhenyl | tBuPhenyl | 2-Naphthyl | 2-Naphthyl |
| 565 | Ph-NAr1Ar2 | NAr3Ar4 | tBuPhenyl | tBuPhenyl | m-Tolyl | m-Tolyl |
| 566 | Ph-NAr1Ar2 | NAr3Ar4 | tBuPhenyl | tBuPhenyl | tBuPhenyl | tBuPhenyl |
| 567 | Ph-NAr1Ar2 | Ph-NAr3Ar4 | tBuPhenyl | tBuPhenyl | Phenyl | Phenyl |
| 568 | Ph-NAr1Ar2 | Ph-NAr3Ar4 | tBuPhenyl | tBuPhenyl | Phenyl | 1-Naphthyl |
| 569 | Ph-NAr1Ar2 | Ph-NAr3Ar4 | tBuPhenyl | tBuPhenyl | Phenyl | 2-Naphthyl |
| 570 | Ph-NAr1Ar2 | Ph-NAr3Ar4 | tBuPhenyl | tBuPhenyl | Phenyl | m-Tolyl |
| 571 | Ph-NAr1Ar2 | Ph-NAr3Ar4 | tBuPhenyl | tBuPhenyl | Phenyl | tBuPhenyl |
| 572 | Ph-NAr1Ar2 | Ph-NAr3Ar4 | tBuPhenyl | tBuPhenyl | Phenyl | Phenanthrenyl |
| 573 | Ph-NAr1Ar2 | Ph-NAr3Ar4 | tBuPhenyl | tBuPhenyl | 1-Naphthyl | 1-Naphthyl |
| 574 | Ph-NAr1Ar2 | Ph-NAr3Ar4 | tBuPhenyl | tBuPhenyl | 2-Naphthyl | 2-Naphthyl |
| 575 | Ph-NAr1Ar2 | Ph-NAr3Ar4 | tBuPhenyl | tBuPhenyl | m-Tolyl | m-Tolyl |
| 576 | Ph-NAr1Ar2 | Ph-NAr3Ar4 | tBuPhenyl | tBuPhenyl | tBuPhenyl | tBuPhenyl |

Die erfindungsgemäßen Verbindungen können nach dem Fachmann bekannten Syntheseschritten dargestellt werden. So lassen sich die verschiedenen Grundgerüste beispielsweise durch Kupplung eines Fluorens mit einem Carbonyl-substituierten Naphthalin, bzw. Phenanthren, Addition eines Alkyl- bzw. Arylmetallderivats und säurekatalysierter Cyclisierung des entsprechenden tertiären Alkohols darstellen, wie in Schema 1 am Beispiel des Naphthalins gezeigt. Heteroanaloga lassen sich entsprechend durch Einsatz der entsprechenden heterocyclischen Verbindung, beispielsweise Carbazol, Dibenzofuran, Dibenzothiophen, etc. synthetisieren. Werden statt Naphthalin oder Phenanthren entsprechend funktionalisierte Heterocyclen eingesetzt, sind die heterocyclischen Grundgerüste zugänglich. Diese Grundgerüste lassen sich nach Standardmethoden funktionalisieren, beispielsweise durch Friedel-Crafts-Alkylierung oder -Acylierung. Weiterhin lassen sich die Grundgerüste nach Standardmethoden der organischen Chemie halogenieren. Je nach den gewählten Halogenierungsbedingungen erhält man selektiv die mono- oder die dihalogenierte Verbindung. So erhält man mit einem Äquivalent NBS selektiv die entsprechende monobromierte Verbindung, während mit zwei Äquivalenten Brom selektiv die entsprechende dibromierte Verbindung erhalten wird. Die bromierten oder iodierten Verbindungen stellen die Basis für weitere Funktionalisierungen dar. So können sie durch Suzuki-Kupplung mit Arylboronsäuren oder Arylboronsäurederivaten oder mit Organozinnverbindungen gemäß Stille zu erweiterten aromatischen Verbindungen umgesetzt werden. Durch Kupplung mit aromatischen oder aliphatischen Aminen gemäß Hartwig-Buchwald erhält man die entsprechenden Amine, wie in Schema 1 gezeigt. Weiterhin können sie via Lithiierung und Reaktion mit Elektrophilen wie Benzonitril und anschließender saurer Hydrolyse zu Ketonen oder mit Chlordiarylphosphinen und anschließender Oxidation zu Phosphinoxiden umgesetzt werden.

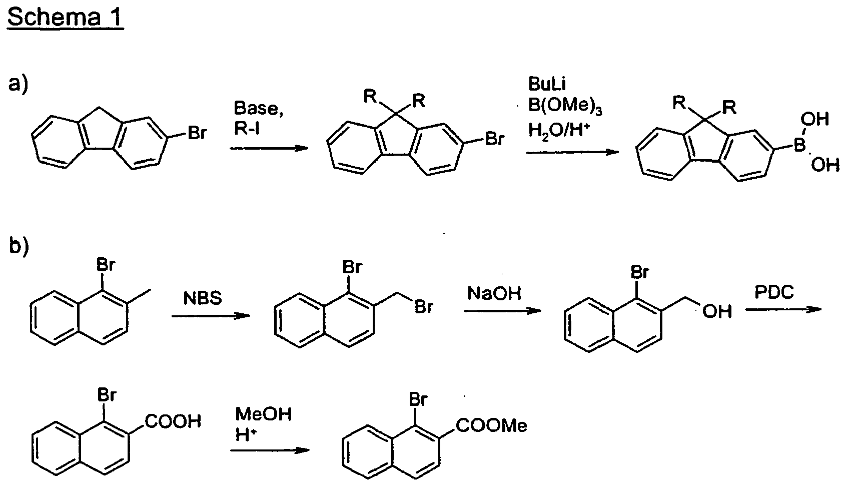

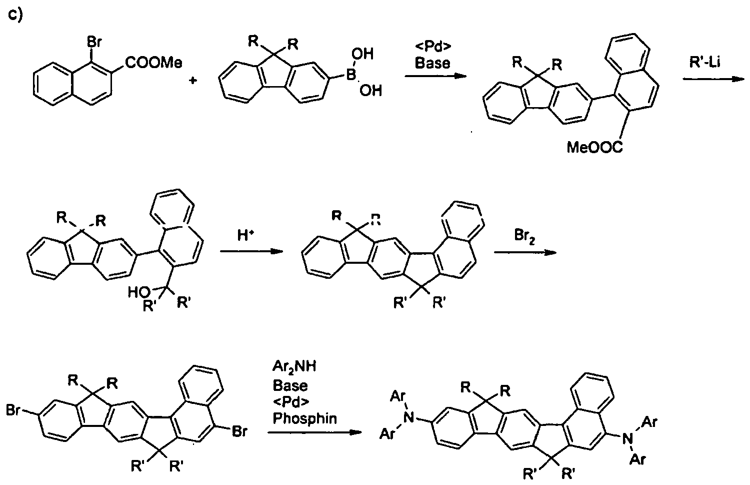

Ein weiterer Gegenstand der Erfindung ist ein Verfahren zur Herstellung der Grundgerüste der Verbindungen durch Kupplung eines Fluorens, welches eine reaktive Gruppe trägt, mit einem Carbonyl-funktionalisierten Naphthalin oder Phenanthren, welches jeweils eine reaktive Gruppe trägt, gefolgt von Addition eines Alkyl- oder Arylmetallreagenz und säurekatalyisierter Cyclisierungsreaktion. Als Kupplungsreaktion zwischen dem Fluoren und dem Naphthalin bzw. Phenanthren eignen sich insbesondere übergangsmetallkatalysierte Kupplungsreaktionen, insbesondere Suzuki-Kupplung, so dass sich hier insbesondere die Kupplung eines Boronsäurederivats, beispielsweise eines Fluorenboronsäurederivats, mit einem Halogenderivat, beispielsweise einem Naphthalin- oder Phenanthrenhalogenderivat anbietet. Die reaktiven Gruppen sind also bevorzugt Halogen, insbesondere Brom, und Boronsäurederivate. Für die Addition des Alkyl- bzw. Arylmetallreagenzes eignen sich insbesondere Alkyl- bzw. Aryllithiumverbindungen und Grignardverbindungen.

Nochmals ein weiterer Gegenstand der Erfindung ist ein Verfahren zur Herstellung funktionalisierter Verbindungen gemäß Formel (1), (2), (4) oder (5) durch Alkylierung oder Acylierung der entsprechenden nicht-funktionalisierten Verbindung oder durch Halogenierung der nicht-funktionalisierten Verbindung, gefolgt von Kupplung mit einem funktionalisierten Aromaten oder mit einem mono- oder disubstituierten Amin oder gefolgt von Metallierung und Reaktion mit Elektrophilen. Die Halogenierung ist bevorzugt eine Bromierung. Als Kupplungsreaktion zwischen dem Grundgerüst gemäß Formel (1), (2), (4) oder (5) und dem Arylsubstituenten eignen sich vor allen übergangsmetallkatalysierte Kupplungsreaktionen, insbesondere Suzuki-Kupplung, so dass sich hier insbesondere die Kupplung eines Boronsäurederivats mit einem Halogenderivat anbietet. Als Kupplungsreaktion mit einem mono- oder disubstituierten Amin eignet sich insbesondere die palladiumkatalysierte Kupplung nach Hartwig-Buchwald.

Bei der Synthese können sowohl die 5-Ring/5-Ring-Derivate wie auch die 5-Ring/6-Ring-Derivate oder Mischungen dieser Verbindungen entstehen. Welche Isomere entstehen und in welchem Verhältnis sie entstehen, hängt von den exakten Synthesebedingungen ab. Falls Mischungen entstehen, können diese entweder getrennt und als Reinverbindungen weiterverarbeitet werden, oder sie können als Mischung eingesetzt werden.

Die oben beschriebenen erfindungsgemäßen Verbindungen, insbesondere Verbindungen, welche mit reaktiven Abgangsgruppen, wie Brom, Iod, Boronsäure oder Boronsäureester, substituiert sind, können als Monomere zur Erzeugung entsprechender Dimere, Trimere, Tetramere, Pentamere, Oligomere, Polymere oder als Kern von Dendrimeren Verwendung finden. Die Oligomerisation bzw. Polymerisation erfolgt dabei bevorzugt über die Halogenfunktionalität bzw. die Boronsäurefunktionalität.

Die erfindungsgemäßen Verbindungen eignen sich für den Einsatz in elektronischen Vorrichtungen, insbesondere in organischen Elektrolumineszenzvorrichtungen (OLEDs, PLEDs). Abhängig von der Substitution werden die Verbindungen in unterschiedlichen Funktionen und Schichten eingesetzt.

Ein weiterer Gegenstand der Erfindung ist daher die Verwendung von erfindungsgemäßen Verbindungen in elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine erfindungsgemäße Verbindung, insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine erfindungsgemäße Verbindung enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Elektronentransportschichten, Elektroneninjektionsschichten und/oder Charge-Generation Layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine organische Schicht mindestens eine erfindungsgemäße Verbindungenthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine erfindungsgemäße Verbindungenthält und wobei die drei Schichten blaue, grüne und orange oder rote Emission zeigen (für den prinzipiellen Aufbau siehe z. B. WO 05/011013), Ebenso eignen sich für weiße Emission Emitter, welche breitbandige Emissionsbanden aufweisen und dadurch weiße Emission zeigen.

In einer Ausführungsform der Erfindung werden die erfindungsgemäßen Verbindungen als Hostmaterial für einen fluoreszierenden Dotanden eingesetzt. In diesem Fall sind bevorzugt ein oder mehrere Substituenten R¹ aus einfachen oder kondensierten Aryl- oder Heteroarylgruppen gewählt, insbesondere Phenyl, o-, m- oder p-Biphenyl, 1- oder 2-Naphthyl, Anthryl, insbesondere Phenylanthryl oder 1- oder 2-Naphthylanthryl, 2-Fluorenyl und 2-Spirobifluorenyl, welche jeweils durch einen oder mehrere Reste R² substituiert sein können. Dies gilt insbesondere für die Reste R¹ an den Strukturen gemäß Formel (7a) bis (14a) und (19a) bis (26a).

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials gemäß Formel (1), (2), (4) oder (5) in der emittierenden Schicht beträgt zwischen 50.0 und 99.9 Gew.-%, bevorzugt zwischen 80 0 und 99.5 Gew.-%, besenders bevorzugt zwischen 90.0 und 99.0 Gew.-%. Entsprechend beträgt der Anteil des Dotanden zwischen 0.1 und 50.0 Gew.-%, bevorzugt zwischen 0.5 und 20.0 Gew.-%, besonders bevorzugt zwischen 1.0 und 10.0 Gew.-%.

Bevorzugte Dotanden in fluoreszierenden Vorrichtungen sind gewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein Amin enthält, welches bevorzugt aromatisch ist. Unter einem Distyrylamin wird eine Verbindung verstanden, die zwei Styrylgruppen und mindestens ein Amin enthält, welches bevorzugt aromatisch ist. Unter einem Tristyrylamin wird eine Verbindung verstanden, die drei Styrylgruppen und mindestens ein Amin enthält, welches bevorzugt aromatisch ist. Unter einem Tetrastyrylamin wird eine Verbindung verstanden, die vier Styrylgruppen und mindestens ein Amin enthält, welches bevorzugt aromatisch ist. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält, davon bevorzugt mindestens ein kondensiertes Ringsystem mit mindestens 14 aromatischen Ringatomen. Die Styrylgruppen sind besonders bevorzugt Stilbene, die auch an der Doppelbindung oder an den Aromaten weiter substituiert sein können. Beispiele für derartige Dotanden sind substituierte oder unsubstituierte Tristilbenamine oder weitere Dotanden, die beispielsweise in WO 06/000388, WO 06/058737, WO 06/000389 und in den nicht offen gelegten Patentanmeldungen DE 102005058543.4 und DE 102006015183.6 beschrieben sind. Weiterhin sind Verbindungen gemäß WO 06/122630 und gemäß der nicht offen gelegten Patentanmeldung DE 102006025846.0 als Dotanden bevorzugt.

In einer weiteren Ausführungsform der Erfindung werden die erfindungsgemäßen Verbindungen als Matrix für phosphoreszierende Dotanden eingesetzt. In diesem Fall enthalten bevorzugt ein oder mehrere Substituenten R¹ und/oder Brücken X mindestens eine Gruppe C=O und/oder SO₂, und/oder ein oder mehrere Substituenten R¹ enthalten mindestens eine Gruppe P(=O). Besonders bevorzugt sind diese Gruppen direkt an die erfindungsgemäße zentrale Einheit gebunden und enthalten weiterhin besonders bevorzugt noch einen bzw. im Faii des Phosphinoxids zwei weitere aromatische Substituenten. Dies gilt insbesondere für die Reste R¹ an den Strukturen gemäß Formel (7a) bis (14a) und (19a) bis (26a).

In phosphoreszierenden Vorrichtungen ist der Dotand bevorzugt ausgewählt aus der Klasse der Metallkomplexe, enthaltend mindestens ein Element der Ordnungszahl größer 20, bevorzugt größer 38 und kleiner 84, besonders bevorzugt größer 56 und kleiner 80. Bevorzugt werden Metallkomplexe verwendet, die Kupfer, Molybdän, Wolfram, Rhenium, Ruthenium, Osmium, Rhodium, Iridium, Palladium, Platin, Silber, Gold oder Europium enthalten, insbesondere Iridium. Generell eignen sich hierfür phosphoreszierende Materialien, wie sie gemäß dem Stand der Technik verwendet werden.

In nochmals einer weiteren Ausführungsform der Erfindung werden die erfindungsgemäßen Verbindungen als emittierende Materialien eingesetzt. Die Verbindungen sind insbesondere dann als emittierende Verbindungen geeignet, wenn mindestens ein Substituent R¹ mindestens eine Vinylaryl-Einheit, mindestens eine Vinylarylamin-Einheit und/oder mindestens eine Arylamino-Einheit enthält. Bevorzugte Arylaminoeinheiten sind die Gruppen der vorne abgebildeten Formeln (29) und (30). Dies gilt insbesondere für die Reste R¹ an den Strukturen gemäß Formel (7a) bis (14a) und (19a) bis (26a). Besonders bevorzugte Dotanden sind solche, in welchen entweder zwei Reste R¹ für Gruppen der Formel (29) oder (30) stehen oder in welchen ein Rest R¹ für eine Gruppe der Formel (29) oder (30) steht und die anderen Reste R¹ für H stehen.

Der Anteil der erfindungsgemäßen Verbindung in der Mischung der emittierenden Schicht beträgt zwischen 0.1 und 50.0 Gew.-%, bevorzugt zwischen 0.5 und 20.0 Gew.-%, besonders bevorzugt zwischen 1.0 und 10.0 Gew.-%. Entsprechend beträgt der Anteil des Hostmaterials zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen hierfür Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268) oder der Boronsäurederivate (z. B. gemäß WO 06/117052). Weiterhin kommen als Hostmaterialien auch die oben beschriebenen erfindungsgemäßen Verbindungen in Frage. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomere dieser Verbindungen, der Oligoarylenvinylene, der Ketone, der Phosphinoxide und der Sulfoxide. Ganz besonders bevorzugte Hostmaterialien sind außer den erfindungsgemäßen Verbindungen ausgewählt aus den Klassen der Oligoarylene, enthaltend Anthracen und/oder Pyren oder Atropisomere dieser Verbindungen, der Phosphinoxide und der Sulfoxide. Unter einem Oligoarylen im Sinne dieser Erfindung soll eine Verbindung verstanden werden, in der mindestens drei Aryl- bzw. Arylengruppen aneinander gebunden sind.

In nochmals einer weiteren Ausführungsform der Erfindung werden die erfindungsgemäßen Verbindungen als Lochtransportmaterial bzw. als Lochinjektionsmaterial eingesetzt. Die Verbindungen sind dann bevorzugt mit mindestens einer Gruppe N(Ar)₂ substituiert, bevorzugt mit mindestens zwei Gruppen N(Ar)₂. Die Gruppen N(Ar)₂ sind bevorzugt ausgewählt aus den oben beschriebenen Formeln (29) oder (30). Dies gilt insbesondere für die Reste R¹ an den Strukturen gemäß Formel (7a) bis (14a) und (19a) bis (26a). Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die erfindungsgemäßen Verbindungen als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP1476881 oder EP 1596445 beschrieben.

In nochmals einer weiteren Ausführungsform der Erfindung werden die erfindungsgemäßen Verbindungen als Elektronentransportmaterial eingesetzt. Hier ist es bevorzugt, wenn ein oder mehrere Substituenten R¹ mindestens eine Einheit C=O, P(=O) und/oder SO₂ enthalten. Weiterhin ist es hier bevorzugt, wenn ein oder mehrere Substituenten R¹ einen elektronenarmen Heterocyclus enthalten, wie beispielsweise Imidazol, Pyrazol, Thiazol, Benzimidazol, Benzothiazol, Triazol, Oxadiazol, Benzothiadiazol, Phenanthrolin, etc. Besonders bevorzugt sind diese Gruppen direkt an die erfindungsgemäße zentrale Einheit gebunden und enthalten weiterhin besonders bevorzugt noch einen bzw. im Fall des Phosphinoxids zwei weitere aromatische Substituenten. Dies gilt insbesondere für die Reste R¹ an den Strukturen gemäß Formel (7a) bis (14a) und (19a) bis (26a). Weiterhin kann es bevorzugt sein, wenn die Verbindung mit Elektronendonorverbindungen dotiert ist.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen weisen ein tieferes LUMO (niedrigstes unbesetztes Molekülorbital) auf als Verbindungen, welche üblicherweise gemäß dem Stand der Technik verwendet werden, und sind dadurch leichter reduzierbar. Daraus resultiert eine verbesserte Elektroneninjektion und damit eine Verringerung der Betriebsspannung.
2. Die erfindungsgemäßen Verbindungen, insbesondere solche, die mit Diarylaminosubstituenten substituiert sind, weisen sehr gute blaue Farbkoordinaten auf und eignen sich daher sehr gut als blaue Emitter.
3. Die mit den erfindungsgemäßen Verbindungen hergestellten OLEDs weisen eine gute Ladungsbalance auf, was in niedrigen Betriebsspannungen resultiert.
4. Die erfindungsgemäßen Verbindungen weisen eine hohe thermische Stabilität auf und lassen sich unzersetzt sublimieren. Außerdem zeigen die Verbindungen eine geringere Verdampfungstemperatur als aromatische Verbindungen im gleichen Molekulargewichtsbereich, welche symmetrisch aufgebaut sind.
5. Die mit den erfindungsgemäßen Verbindungen hergestellten OLEDs weisen eine sehr hohe Lebensdauer auf.
6. Die mit den erfindungsgemäßen Verbindungen hergestellten OLEDs weisen eine sehr hohe Quanteneffizienz auf.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch in anderen elektronischen Vorrichtungen einzusetzen, z. B. in organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

### Beispiele

### Beispiel 1: 1,2-Benzo-3,8-bis-(N,N-diphenylamino)-6,6,12,12-tetramethyl-6,12-dihydro-indeno[1,2.b]fluoren

### a) 1-(9,9-Dimethyl-9H-fluoren-2-yl)-naphthalin-2-carbonsäure-ethylester

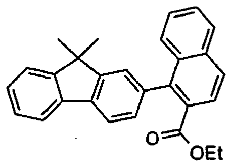

86.3 g (362.55 mmol) 9,9'-Dimethylfluoren-2-boronsäure, 92 g (329.59 mol) 2-Carboxyethyl-bromnaphthalin und 159.4 g (692 mmol) Tri-Kaliumphosphat Monohydrat werden in 450 mL Toluol, 230 mL Dioxan und 700 mL Wasser suspendiert, 6.0 g (19.8 mmol) Tris-o-tolylphosphin, gefolgt von 740 mg (3.3 mmol) Palladiumacetat zugegeben und das Gemisch 4 h zum Sieden erhitzt. Die organische Phase wird abgetrennt, über Kieselgel filtriert und im Vakuum eingeengt. Der Rückstand wird aus Heptan umkristallisiert. Ausbeute: 100.6 g (78 %) farbloser Feststoff.

### b) 2-[1-(9,9-Dimethyl-9H-fluoren-2-yl)-naphthalin-2-yl]-propan-2-ol

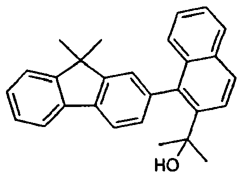

100.6 g 1-(9,9-Dimethyl-9H-fluoren-2-yl)naphthalin-2-carbonsäureethylester (256 mmol) werden in 1000 mL getrocknetem THF vorgelegt, 510 mL einer 1.5 M Methyllithium-Lösung in Diethylether bei -70 °C zugetropft und 2 h bei dieser Temperatur gerührt. Zur Aufarbeitung werden 100 mL Eiswasser gefolgt von 300 mL 50%iger Essigsäure zugegeben. Die organische Phase wird abgetrennt, zweimal mit Wasser gewaschen, getrocknet und im Vakuum eingeengt. Der verbliebene farblose Feststoff wird zweimal aus Heptan/Toluol umkristallisiert. Ausbeute: 82 g (85 %) farblose Kristalle.

### c) 1,2-Benzo-6,6,12,12-tetramethyl-6,12-dihydro-indeno[1,2-b]fluoren

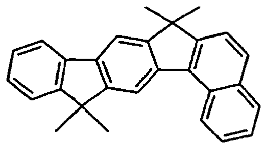

97 g 2-[1-(9,9-Dimethyl-9H-fluoren-2-yl)-naphthalin-2-yl]-propan-2-ol (256 mmol) werden in 750 mL Dichlormethan gelöst, auf 5 °C gekühlt und bei dieser Temperatur eine Mischung von 75 mL Methansulfonsäure und 100 g Polyphosphorsäure zugegeben. Nach 2 h bei 5 °C werden 300 mL Ethanol zugetropft und das Reaktionsgemisch 10 min zum Sieden erhitzt. Nach Abkühlen auf Raumtemperatur wird der Niederschlag abgesaugt, mit Ethanol gewaschen und aus Toluol umkristallisiert. Man erhält isomerenreines Benzindenofluoren als farblose Kristalle (70 g, 76 %).

### d) 1,2-Benzo-3,8-dibrom-6,6,12,12-tetramethyl-6,12-dihydro-indeno-[1,2-b]fluoren

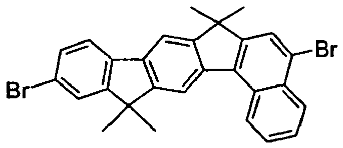

20.9 g (58 mmol) 1,2-Benzo-6,6,12,12-tetramethyl-6,12-dihydro-indeno-[1,2-b]fluoren werden in 600 mL Dichlormethan suspendiert, auf 5 °C abgekühlt und bei dieser Temperatur 6.5 mL (128 mmol) Brom in 50 mL Dichlormethan zugetropft. Nach 6 h werden 200 mL Ethanol zugegeben, 1 h bei Raumtemperatur gerührt und der Niederschlag abgesaugt, mit Ethanol gewaschen und getrocknet. Man erhält 27 g (90 %) des Dibromids mit einer Reinheit von > 99 % (RP-HPLC).

### e) 1,2-Benzo-3,8-bis-(N,N-diphenylamino)-6,6,12,12-tetramethyl-6,12-dihydro-indeno[1,2-b]fluoren

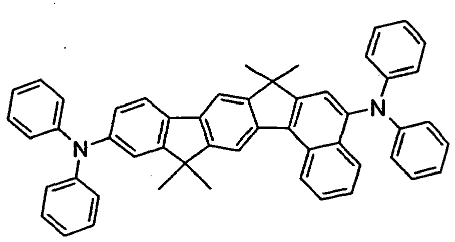

26.4 g (51 mmol)1,2-Benzo-3,8-dibrom-6,6,12,12-tetramethyl-6,12-dihydro-indeno[1,2-b]fluoren und 22.6 g (133 mmol) Diphenylamin werden in 200 mL getrocknetem Toluol gelöst, mit N₂ gesättigt, anschließend 1 mL (4 mmol) Tri-tert-butylphosphin, gefolgt von 450 mg (2 mmol) Palladiumacetat sowie 14.6 g (153 mmol) Natrium-tert butylat zugegeben. Das Gemisch wird 3 h zum Sieden erhitzt, die organische Phase abgetrennt, zweimal mit Wasser gewaschen, filtriert und einrotiert. Nach sechsmaliger Umkristallisation aus Toluol und zweimaliger Sublimation (2 x 10⁻⁵ mbar/340 °C) erhält man 12 g (68 %) des Diamins in Form eines gelben Pulvers mit einer Reinheit von > 99.9 % (RP-HPLC). Die Verbindung weist ausgezeichnete thermische Stabilität auf. Bei der Sublimation lässt sich keine Zersetzung beobachten.

In Analogie zum oben beschriebenem Verfahren werden folgende Verbindungen synthetisiert (alle Reinheiten > 99.9 %, Ausbeuten nach zweimaliger Sublimation):

| Beispiel Nr. | Struktur | Ausbeute (%) |
|---|---|---|
| 2 | 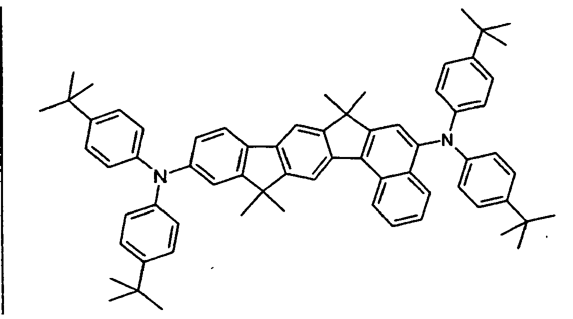 | 69 |
| 3 | 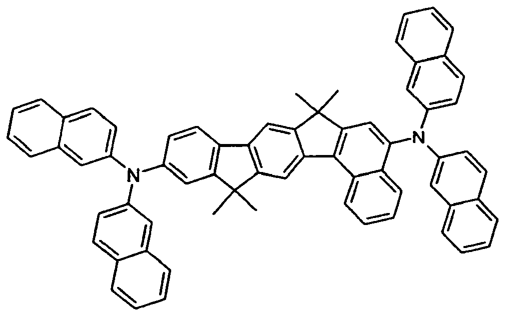 | 54 |
| 4 | 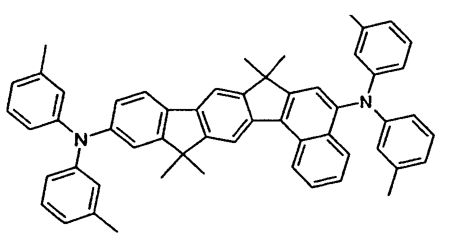 | 77 |
| 5 | 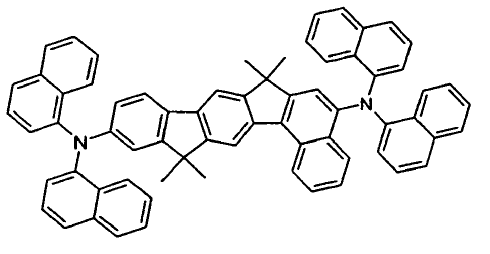 | 79 |
| 6 | 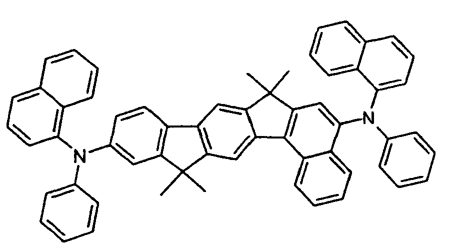 | 84 |
| 7 | 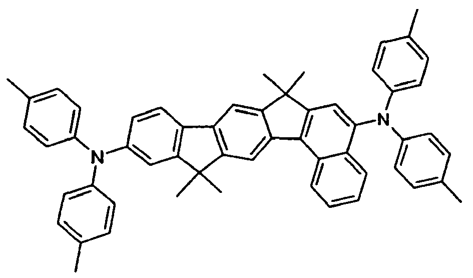 | 82 |

### Beispiel 8: 1,2-Benzo-3-[-9-{-10-(2-naphthyl)}-anthryl]-6,6,12,12-tetramethyl-6,12-dihydro-indeno[1,2-b]fluoren

### a) 1,2-Benzo-3-brom-6,6,12,12-tetramethyl-6,12-dihydro-indeno(1,2-b]fluoren

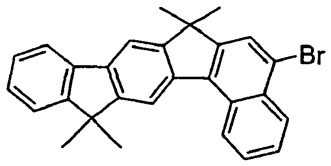

15.5 g (43 mmol) 1,2-Benzo-6,6,12,12-tetramethyl-6,12-dihydro-indeno-[1,2-b]fluoren werden in 350 mL THF gelöst, 8.4 g (47.3 mmol) NBS zugegeben und 4 h zum Sieden erhitzt. Nach Entfernen des Lösungsmittels im Vakuum wird der Rückstand in Ethanol/Wasser (1 : 1) ausgekocht, der Feststoff abgesaugt, mit Ethanol gewaschen und getrocknet. Es verbleiben 15.4 g (82 %) Monobromid als farbloses Pulver.

### b) 1,2-Benzo-6,6,12,12-tetramethyl-6,12-dihydro-indeno[1,2-b]fluoren-3-boronsäure

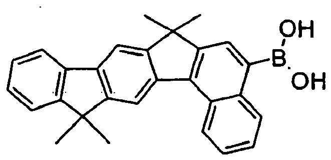

14 g (32 mmol) 1,2-Benzo-3-brom-6,6,12,12-tetramethyl-6,12-dihydro-indeno[1,2-b]fluoren werden in 150 mL trockenem Diethylether suspendiert, bei -70 °C 21 mL (42 mmol) einer 2 M Lösung von *n*-Butyllithium in Cyclohexan zugegeben, 1 h bei dieser Temperatur gerührt, 8.8 mL (79 mmol) Trimethylborat zugetropft und auf Raumtemperatur kommen gelassen. Nach wässriger Aufarbeitung, Trocknen der organischen Phase und Entfernen des Lösungsmittels im Vakuum erhält man 12.5 g (96 %) der Boronsäure als farblosen Schaum, der ohne weitere Reinigung umgesetzt wird.

### c) 1,2-Benzo-3-[-9-(10-(2-naphthyl)}-anthryl]-6,6,12,12-tetramethyl-6,12-dihydro-indeno[1,2-b]fluoren

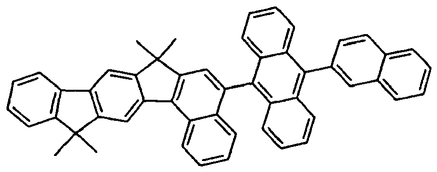

Eine Mischung aus 11.3 g (30 mmol) 9-Brom-10-(2-naphthyl)-anthracen, 12.5 g (31 mmol) 1,2-Benzo-6,6,12,12-tetramethyl-6,12-dihydro-indeno-[1,2-b]fluoren-3-boronsäure und 13.1 g (62 mmol) Tri-Kaliumphosphat Trihydrat in 40 mL Toluol, 20 mL Dioxan und 60 mL Wasser wird 30 min mit N₂ gesättigt, 1.1 g (4 mmol) Tris-o-tolylphosphin, sowie 132 mg (1 mmol) Palladiumacetat zugegeben und das Gemisch 4 h zum Sieden erhitzt. Die organische Phase wird abgetrennt, mehrmals mit Wasser gewaschen, filtriert und im Vakuum das Lösungsmittel entfernt. Nach sechsmaliger Umkristallisation aus Dioxan und zweimaliger Sublimation (2 x 10⁻⁵ mbar/380 °C) erhält man 12 g (62 %) der Zielverbindung in Form eines blassgelben Pulvers mit einer Reinheit von > 99.9 % (RP-HPLC). Die Verbindung weist ausgezeichnete thermische Stabilität auf. Bei der Sublimation lässt sich keine Zersetzung beobachten.

In Analogie zum oben beschriebenem Verfahren werden folgende Verbindungen synthetisiert (alle Reinheiten > 99.9 %, Ausbeuten nach zweimaliger Sublimation):

| Beispiel Nr. | Struktur | Ausbeute (%) |
|---|---|---|
| 9 | 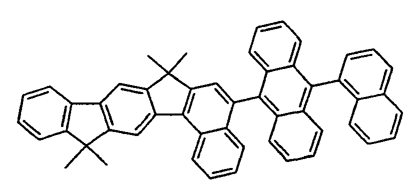 | 58 |
| 10 | 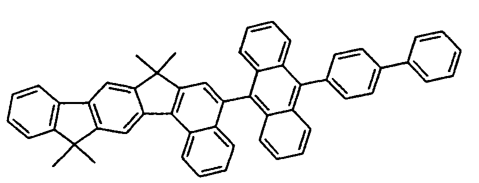 | 62 |
| 11 | 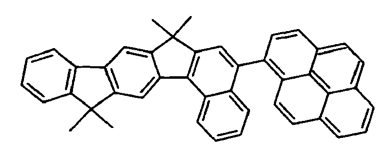 | 71 |
| 12 | 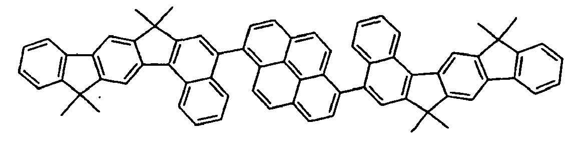 | 66 |

### Beispiel 13: 1,2-Benzo-3-(N,N-bis-4-tert-butylphenylamino)-6,6,12,12-tetramethyl-6,12-dihydro-indeno[1,2-b]fluoren

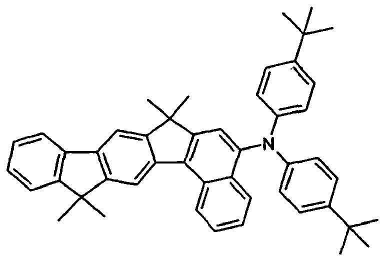

23.1 g (53 mmol) 1,2-Benzo-3-brom-6,6,12,12-tetramethyl-6,12-dihydro-indeno[1,2-b]fluoren und 19.2 g (68 mmol) Di-*tert*-butylphenylamin werden in 100 mL getrocknetem Toluol gelöst, mit N₂ gesättigt, anschließend 0.5 mL (2 mmol) Tri-*tert*-butylphosphin, gefolgt von 240 mg (1 mmol) Palladiumacetat sowie 8.6 g (89 mmol) Natrium-*tert*-butylat zugegeben. Das Gemisch wird 3 h zum Sieden erhitzt, die organische Phase abgetrennt, zweimal mit Wasser gewaschen, filtriert und einrotiert. Nach viermaliger Umkristallisation aus *i*-PrOH erhält man 24 g (71 %) des Amins in Form eines gelben Pulvers mit einer Reinheit von > 99.9 % (RP-HPLC). Die Verbindung weist ausgezeichnete thermische Stabilität auf. Bei der Sublimation lässt-sich keine Zersetzung beobachten.

In Analogie zum oben beschriebenen Verfahren werden folgende Verbindungen synthetisiert (alle Reinheiten > 99.9 %, Ausbeuten nach zweimaliger Sublimation):

| Beispiel Nr. | Struktur | Ausbeute (%) |
|---|---|---|
| 14 | 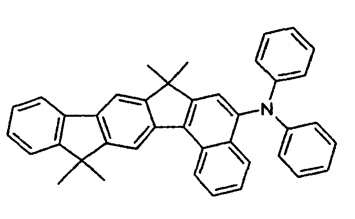 | 82 |
| 15 | 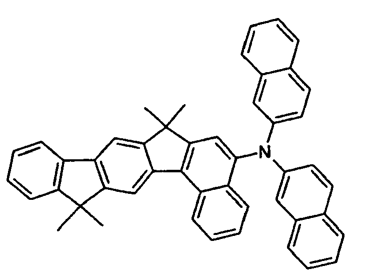 | 74 |
| 16 | 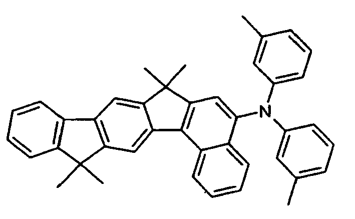 | 78 |
| 17 | 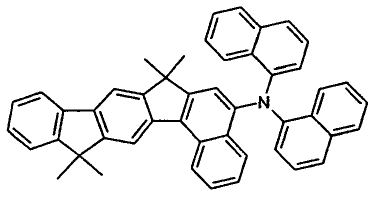 | 66 |
| 18 | 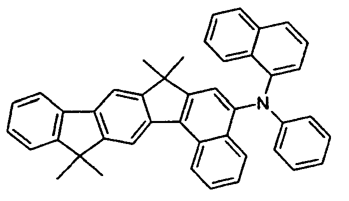 | 63 |
| 19 | 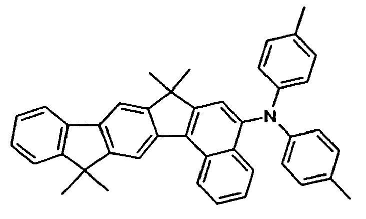 | 74 |

### Beispiel 20: 3',8-Bis(diphenylamino)-3,4-benzo-6,6,12,12-tetramethyl-6,12-dihydro-indeno[1,2-b]fluoren

### a) 3,4-Benzo-6,6,12,12-tetramethyl-6,12-dihydro-indeno[1,2-b]fluoren

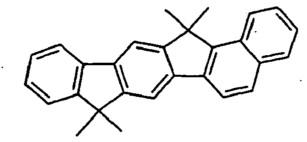

Diese Verbindung wird analog zur Synthese von 1,2-Benzo-6,6,12,12-tetramethyl-6,12-dihydro-indeno[1,2-b]fluoren (Beispiel 1c) aus 9,9'-Dimethylfluoren-2-boronsäure und 1-Carboxyethyl-2-bromnaphthalin hergestellt.

### b) 3',8-Dibrom-3,4-benzo-6,6,12,12-tetramethyl-6,12-dihydro-indeno[1,2-b]fluoren

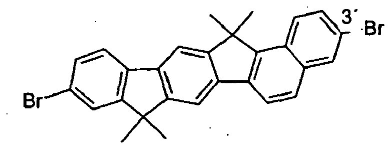

Die Verbindung wird analog zur Synthese von 1,2-Benzo-3,8-dibrom-6,6,12,12-tetramethyl-6,12-dihydro-indeno[1,2-b]fluoren (Beispiel 1d), ausgehend von 3,4-Benzo-6,6,12,12-tetramethyl-6,12-dihydro-indeno[1,2-b]fluoren hergestellt.

### c) 3',8-Bis(diphenylamino)-3,4-benzo-6,6,12,12-tetramethyl-6,12-dihydro-indeno[1,2-b]fluoren

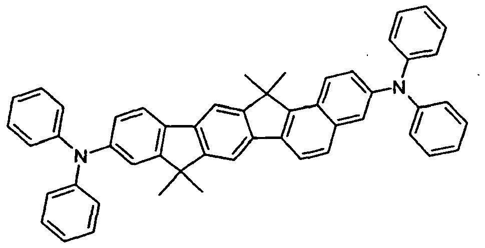

Die Verbindung wird in Analogie zu dem im Beispiel 13 beschriebenen Verfahren synthetisert. Ausbeute: 77 % in einer Reinheit von > 99.9 % nach zweimaliger Sublimation. Die Verbindung weist ausgezeichnete thermische Stabilität auf. Bei der Sublimation lässt sich keine Zersetzung beobachten.

In Analogie zu Beispiel 20 werden folgende Verbindungen synthetisiert (alle Reinheiten > 99.9 %, Ausbeuten nach zweimaliger Sublimation):

| Beispiel Nr. | Struktur | Ausbeute (%) |
|---|---|---|
| 21 | 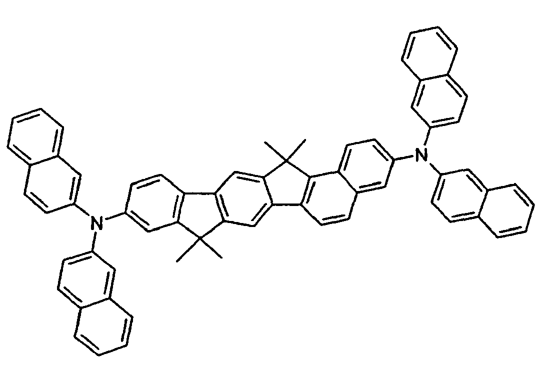 | 75 |
| 22 | 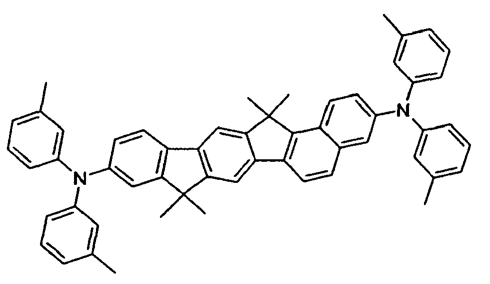 | 73 |
| 23 | 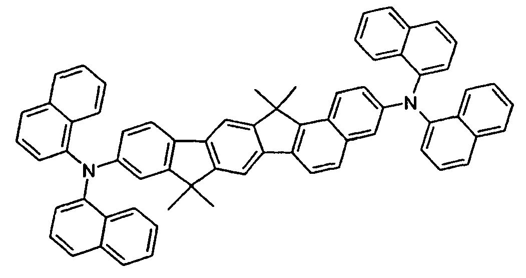 | 54 |
| 24 | 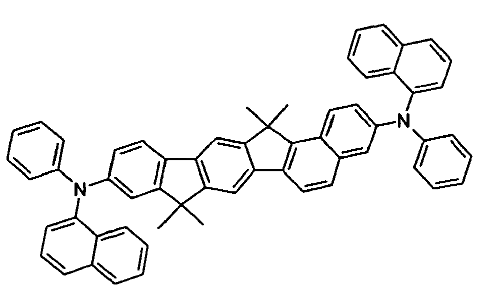 | 44 |
| 25 | 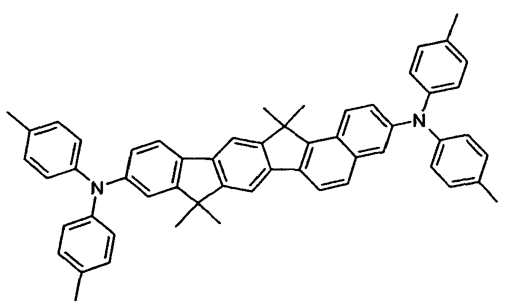 | 55 |

### Beispiel 26: 2',8-Bis(diphenylamino)-2,3-Benzo-6,6,12,12-tetramethyl-6,12-dihydro-indeno[1,2-b]fluoren

### a) 2,3-Benzo-6,6,12,12-tetramethyl-6,12-dihydro-indeno[1,2-b]fluoren

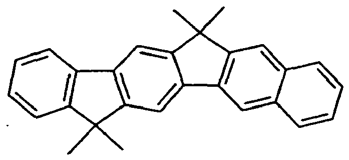

Diese Verbindung wird analog zur Synthese von 1,2-Benzo-6,6,12,12-tetramethyl-6,12-dihydro-indeno[1,2-b]fluoren (Beispiel 1c) aus 9,9'-Dimethylfluoren-2-boronsäure und 3-Carboxyethyl-2-bromnaphthalin hergestellt.

### b) 2',8-Dibrom-2,3-Benzo-6,6,12,12-tetramethyl-6,12-dihydro-indeno[1,2-b]fluoren

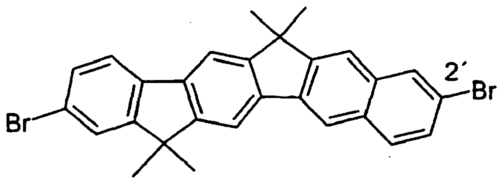

Die Verbindung wird analog zur Synthese von 1,2-Benzo-3,8-dibrom-6,6,12,12-tetramethyl-6,12-dihydro-indeno[1,2-b]fluoren (Beispiel 1d), ausgehend von 2,3-Benzo-6,6,12,12-tetramethyl-6,12-dihydro-indeno[1,2-b]fluoren hergestellt.

### c) 2',8-Bis(diphenylamino)-2,3-Benzo-6,6,12,12-tetramethyl-6,12-dihydro-indeno[1,2-b]fluoren

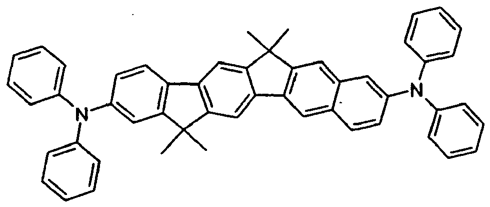

Diese Verbindung wird in Analogie zum im Beispiel 13 beschriebenen Verfahren synthetisiert. Ausbeute: 74 % in einer Reinheit von > 99.9 % nach zweimaliger Sublimation. Die Verbindung weist ausgezeichnete thermische Stabilität auf. Bei der Sublimation lässt sich keine Zersetzung beobachten.

In Analogie zum im Beispiel 25 beschriebenem Verfahren werden folgende Verbindungen synthetisiert (alle Reinheiten > 99.9 %, Ausbeuten nach zweimaliger Sublimation):

| Beispiel Nr. | Struktur | Ausbeute (%) |
|---|---|---|
| 27 | 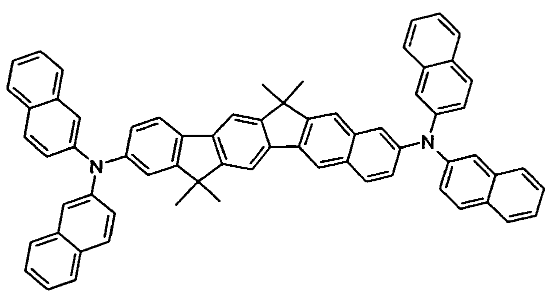 | 68 |
| 28 | 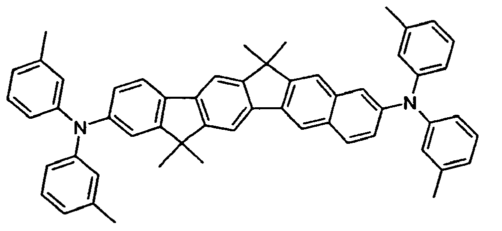 | 54 |
| 29 | 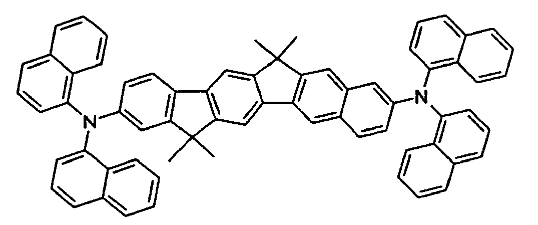 | 57 |
| 30 | 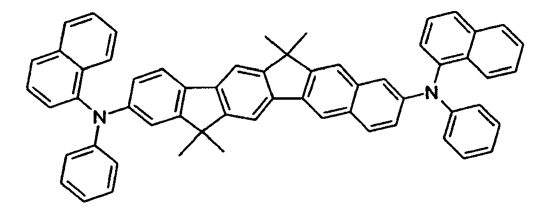 | 79 |
| 31 | 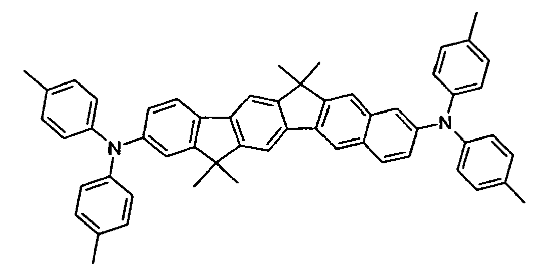 | 81 |

### Beispiel 32: 1,2-Benzo-3-(N,N-bis-4-tert-butylphenylamino)-6,6,dimethyl-12,12-oxa-6,12-dihydro-indeno[1,2-b]fluoren

### a) 1-(Dibenzofuran-2-yl)-naphthalin-2-carbonsäureethylester

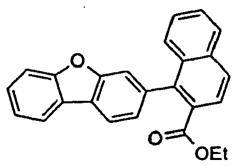

Durchführung analog Beispiel 1a). Anstelle von 86.3 g (362 mmol) 9,9'-Dimethylfluoren-2-boronsäure werden 42.4 g (200 mmol) Dibenzofuran-2-boronsäure eingesetzt, die Molmenge der anderen Reagenzien wird entsprechend angepasst. Ausbeute: 60.8 g (83 %) farbloser Feststoff.

### b) 2-[1-(Dibenzofuran-2-yl)-naphthalin-2-yl]-propan-2-ol

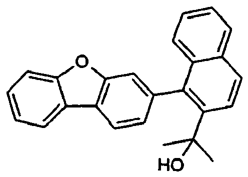

Durchführung analog Beispiel 1b). Anstelle von 100.6 g (256 mmol) 1-(9,9-Dimethyl-9H-fluoren-2-yl)-naphthalin-2-carbonsäureethylester werden 55.0 g (150 mmol) 1-(Dibenzofuran-2-yl)-naphthalin-2-carbonsäureethylester eingesetzt, die Molmenge der anderen Reagenzien wird entsprechend angepasst. Ausbeute: 41.8 g (79 %) farbloser Feststoff.

### c) 1,2-Benzo-6,6,dimethyl-12,12-oxa-6,12-dihydro-indeno[1,2-b]fluoren

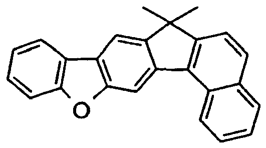

Durchführung analog Beispiel 1c). Anstelle von 97 g (256 mmol) 2-[1-(9,9-Dimethyl-9H-fluoren-2-yl)-naphthalin-2-yl]-propan-2-ol werden 39.8 g (113 mmol) 2-[1-(Dibenzofuran-2-yl)-naphthalin-2-yl]-propan-2-ol eingesetzt, die Molmenge der anderen Reagenzien wird entsprechend angepasst. Ausbeute: 29.1 g (77 %) farblose Kristalle.

### d) 1,2-Benzo-3-brom-6,6-dimethyl-12,12-oxa-6,12-dihydro-indeno[1,2b]fluoren

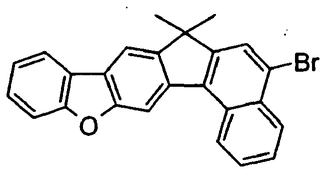

Durchführung analog Beispiel 8a). An Stelle von 15.5 g (43 mmol) 1,2-Benzo-6,6,12,12-tetramethyl-6,12-dihydro-indeno[1,2-b]fluoren werden 20.1 g (60 mmol) 1,2-Benzo-6,6,dimethyl-12,12-oxa-6,12-dihydro-indeno[1,2-b]fluoren eingesetzt, die Molmenge der anderen Reagenzien wird entsprechend angepasst. Die Reaktion wird in DMF bei 40 °C durchgeführt. Nach Entfernen des Lösungsmittels im Vakuum wird der Rückstand in Ethanol/Wasser (1 : 1) ausgekocht, der Feststoff abgesaugt, mit Ethanol gewaschen und getrocknet und anschließend zweimal aus DMF umkristallisiert. Es verbleiben 13.9 g (56 %) Monobromid als farblose Kristalle.

### e) 1,2-Benzo-3-(N,N-bis-4-tert-butylphenylamino)-6,6,dimethyl-12,12-oxa-6,12-dihydro-indeno[11,2-b]fluoren

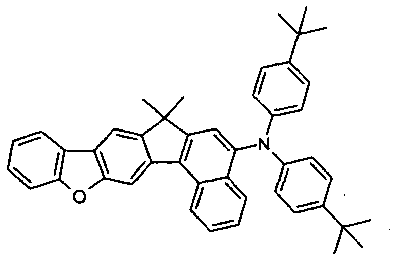

Durchführung analog Beispiel 13. An Stelle von 23.1 g (53 mmol) 1,2-Benzo-3-brom-6,6,12,12-tetramethyl-6,12-dihydro-indeno[1,2-b]fluoren werden 10.3 g (25 mmol) 1,2-Benzo-3-brom-6,6,dimethyl-12,12-oxa-6,12-dihydro-indeno[1,2-b]fluoren eingesetzt, die Molmenge der anderen Reagenzien wird entsprechend angepasst. Reinigung durch fünfmalige Umkristallisation aus DMF und zweimalige Sublimation (T = 315 °C, p = 5 x 10⁻⁵ mbar) ergibt 9,8 g (64 %) eines gelben Pulvers mit einer Reinheit von > 99.8 % (RP-HPLC). Die Verbindung weist ausgezeichnete thermische Stabilität auf. Bei der Sublimation lässt sich keine Zersetzung beobachten.

In Analogie zum oben beschriebenen Verfahren werden folgende Verbindungen synthetisiert (alle Reinheiten > 99.9 %, Ausbeuten nach zweimaliger Sublimation). Dabei kommen anstelle von Dibenzofuran-3-boronsäure die Dibenzothiophen-2-boronsäure bzw. die N-Phenylcarbazol-2-boronsäure zum Einsatz.

| Beispiel Nr. | Struktur | Ausbeute letzte Stufe (%) |
|---|---|---|
| 33 | 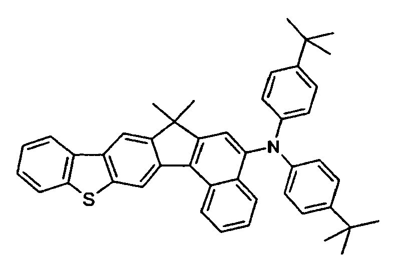 | 78 |
| 34 | 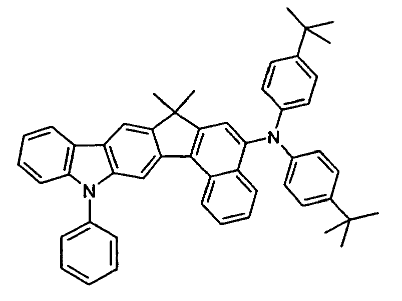 | 51 |

### Beispiel 35 (Referenzbeispiel): 1,2-Benzo-3-(1-napohthyl)-6,6,dimethyl-12,12-mesitylboranyl-6,12-dihydro-indeno[1,2-b]fluoren

### a) 1-Biphenyl-4-yl-naphthalin-2-carbonsäureethylester

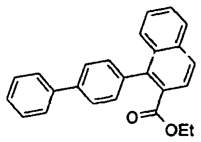

Durchführung analog zu Beispiel 1a). Anstelle von 86.3 g (362 mmol) 9,9'-Dimethylfluoren-2-boronsäure werden 39.6 g (200 mmol) Biphenyl-4-boronsäure eingesetzt, die Molmenge der anderen Reagenzien wird entsprechend angepasst. Ausbeute: 67.2 g (95 %) farbloser Feststoff.

### b) 2-[1-Biphenyl-4-yl-naphthalin-2-yl]-propan-2-ol

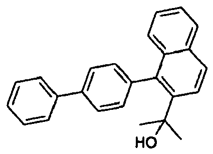

Durchführung analog zu Beispiel 1b). Anstelle von 100.6 g (256 mmol) 1-(9,9-Dimethyl-9H-fluoren-2-yl)-naphthalin-2-carbonsäureethylester werden 52,9 g (150 mmol) 1-Biphenyl-4-yl-naphthalin-2-carbonsäureethylester eingesetzt, die Molmenge der anderen Reagenzien wird entsprechend angepasst. Ausbeute: 36.0 g (71 %) farbloser Feststoff.

### c) 7,7-Dimethyl-9-phenyl-7H-benzo[c]fluoren

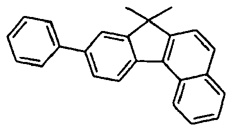

Durchführung analog Beispiel 1c). Anstelle von 97 g (256 mmol) 2-[1-(9,9-Dimethyl-9H-fluoren-2-yl)-naphthalin-2-yl]-propan-2-ol werden 35.0 g (103 mmol) 2-[1-Biphenyl-4-yl-naphthalin-2-yl]-propan-2-ol eingesetzt, die Molmenge der anderen Reagenzien wird entsprechend angepasst. Ausbeute: 26.5 g (80 %) farblose Kristalle.

### d) 1,2-Benzo-6,6-dimethyl-12,12-mesitylboranyl-6,12-dihydro-indeno[1,2-b]fluoren

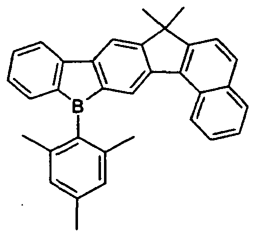

Eine Lösung von 16.0 g (50 mmol) 7,7-Dimethyl-9-phenyl-7H-benzo[c]-fluoren in 300 ml Cyclohexan wird unter guten Rühren mit 30.0 ml (200 mmol) N,N,N',N'-Tetramethylethylendiamin und dann mit 44.0 ml (110 mmol) n-Butyllithium (2.5 molar in n-Hexan) versetzt und 36 h bei Raumtemperatur gerührt. Die Reaktionsmischung wird auf -78 °C abgekühlt und tropfenweise mit einer Lösung von 21.8 g (130 mmol) Difluormesitylboran in 50 ml Toluol versetzt. Nach Erwärmen der Reaktionsmischung auf Raumtemperatur gibt man 500 ml entgastes Wasser zu, trennt die organische Phase ab, trocknet über Magnesiumsulfat und engt die organische Phase zur Trockene ein. Der Feststoff wird dreimal aus Toluol / n-Heptan umkristallisiert. Ausbeute: 19.7 g (88 %) farbloser Feststoff.

### e) 1,2-Benzo-3-brom-6,6-dimethyl-12,12-mesitylboranyl-6,12-dihydro-indeno[1,2-b]fluoren

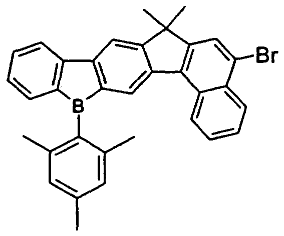

Durchführung analog zu Beispiel 32d). Anstelle von 20.0 g (60 mmol) 1,2-Benzo-6,6,dimethyl-12,12-oxa-6,12-dihydro-indeno[1,2-bjfluoren werden 11.2 g (25 mmol) 1,2-Benzo-6,6-dimethyl-12,12-mesitylboranyl-6,12-dihydro-indeno[1,2-b]fluoren eingesetzt, die Molmenge der anderen Reagenzien wird entsprechend angepasst. Ausbeute: 9.8 g (74 %) farblose Kristalle.

### f) 1,2-Benzo-3-(naphth-1-yl)-fi,6-dimethyl-12,12-mesitylboranyl-6,12-dihydro-indeno[1,2-b]fluoren

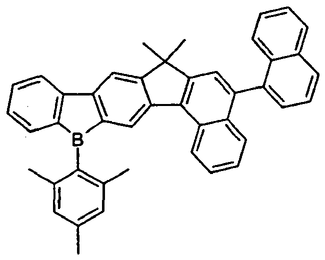

Durchführung analog zu Beispiel 8c). Anstelle von 11.3 g (30 mmol) 9-Brom-(2-naphthyl)-anthracen werden 5.3 g (10 mmol) 1,2-Benzo-3-brom-6,6-dimethyl-12,12-mesitylboranyl-6,12-dihydro-indeno[1,2-b]fluoren und an Stelle von 12.5 g (31 mmol) 1,2-Benzo-6,6,12,12-tetramethyl-6,12-dihydro-indeno[1,2-b]fluoren-3-boronsäure werden 2.1 g (12 mmol) Naphthalin-1-boronsäure eingesetzt, die Molmenge der anderen Reagenzien wird bezogen aus das Bromid entsprechend angepasst. Viermalige Umkristallisation aus DMF und anschließende zweimalige Sublimation (T = 280 °C, p = 2 x 10⁻⁵ mbar) ergibt 3.1 g (54 %) eines farblosen Pulvers mit einer Reinheit von > 99.9 % nach (RP-HPLC). Die Verbindung weist ausgezeichnete thermische Stabilität auf. Bei der Sublimation lässt sich keine Zersetzung beobachten.

### Beispiel 36: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 37 bis 49 werden die Ergebnisse verschiedener OLEDs vorgestellt. Glasplatten, die mit strukturiertem ITO (Indium Zinn Oxid) beschichtet sind, bilden die Substrate der OLEDs. Zur verbesserten Prozessierung wird 20 nm PEDOT (aus Wasser aufgeschleudert; bezogen von H. C. Starck, Goslar, Deutschland; Poly(3,4-ethylendioxy-2,5-thiophen)) auf das Substrat aufgebracht. Die OLEDs bestehen aus folgender Schichtenfolge: Substrat / PEDOT / Lochtransportschicht (HTM1) 60 nm / Emissionschicht (EML) 30 nm / Elektronentransportschicht (ETM) 20 nm und abschließend eine Kathode. Die Materialien bis auf PEDOT werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dünne LiF-Schicht und einer darauf abgeschiedenen 150 nm Al-Schicht gebildet. Die Tabelle 2 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in lm/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 4000 cd/m² auf die Hälfte gesunken ist.

In Tabelle 3 sind die Ergebnisse einiger OLEDs (Beispiele 37 bis 49) zusammengefasst. Als erfindungsgemäße Dotanden bzw. Hostmaterialien werden die Verbindungen der Beispiele 1, 4, 6, 7, 8, 13 und 16 verwendet. Als Vergleichsbeispiele wird der Dotand D1 und das Hostmaterial H1 gemäß dem Stand der Technik verwendet.

**Tabelle 2: Chemische Strukturen der verwendeten Materialien**

| | | |
|---|---|---|
| 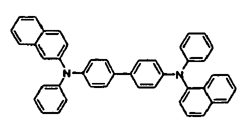 | 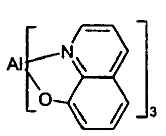 | 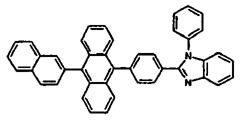 |
| HTM1 | ETM1 | ETM2 |
| 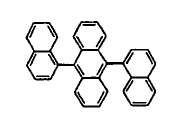 | 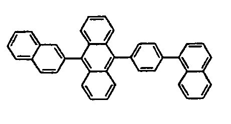 | 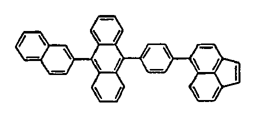 |
| H1 | H2 | H3 |
| 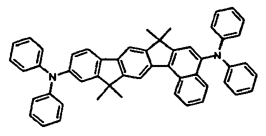 | 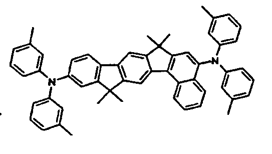 | 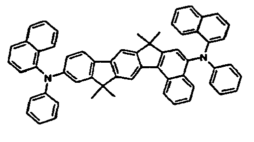 |
| Bsp. 1 | Bsp. 4 | Bsp. 6 |
| 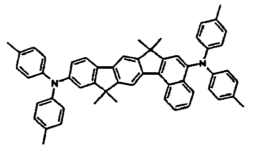 | 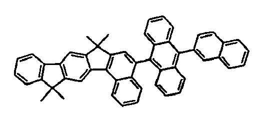 | 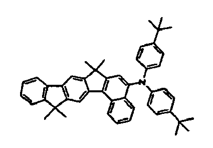 |
| Bsp. 7 | Bsp. 8 | Bsp. 13 |
| 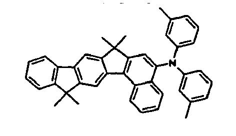 | 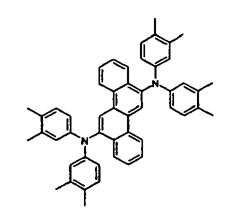 | |
| Bsp. 16 | D1 | |

Wie man aus den in Tabelle 3 zusammengefassten Ergebnissen erkennen kann, führen erfindungsgemäße OLEDs zu einer deutlich verbesserten Lebensdauer im Vergleich zu OLEDs gemäß dem Stand der Technik. Weiterhin erhält man bei tiefer blauen Farbkoordinaten eine vergleichbare oder höhere Effizienz im Vergleich zum Stand der Technik.

**Tabelle 3: Ergebnisse der OLEDs**

| **Beispiel** | **EML** | **ETM** | **Max. Effizienz (cd/A)** | **Spannung (V) bei 1000cd/m²** | **CIE** | **Lebensdauer (h) bei 4000 cd/m²** |
|---|---|---|---|---|---|---|
| 37 (Vergleich) | H1 + 5% D1 | ETM1 | 6.5 | 5.8 | x=0.14/ y=0.19 | 450 |
| 38 | H1 + 5% Bsp. 1 | ETM1 | 6.8 | 5.8 | x=0.15/ y=0.16 | 660 |
| 39 | H2 + 5% Bsp. 1 | ETM1 | 6.9 | 5.7 | x=0.16/ y=0.17 | 700 |
| 40 | H3 + 5% Bsp. 1 | ETM1 | 6.8 | 5.7 | x=0.16/ y=0.17 | 770 |
| 41 | H3+5% Bsp. 1 | ETM2 | 6.7 | 5.3 | x=0.16/ y=0.17 | 850 |
| 42 | Bsp. 8+ 5% D1 | ETM1 | 6.7 | 5.7 | x=0.15/ y=0.17 | 580 |
| 43 | H2 + 5% Bsp. 4 | ETM 1 | 7.0 | 5.6 | x=0.16/ y=0.18 | 930 |
| 44 | H2+5% Bsp. 4 | ETM2 | 6.9 | 5.2 | x=0.16/ y=0.18 | 1050 |
| 45 | H2 + 5% Bsp. 6 | ETM1 | 5.5 | 5.7 | x=0.15/ y=0.13 | 730 |
| 46 | H2 + 5% Bsp. 7 | ETM1 | 5.5 | 5.7 | x=0.17/ y=0.21 | 1500 |
| 47 | H2 + 5% Bsp. 13 | ETM1 | 6.7 | 5.8 | x=0.14/ y=0.18 | 690 |
| 48 | H2 + 5% Bsp. 16 | ETM1 | 5.2 | 5.9 | x=0.15/ y=0.10 | 690 |
| 49 | H2 + 5% Bsp. 16 | ETM2 | 5.0 | 5.6 | x=0.15/ y=0.10 | 670 |

## Patentansprüche

1. Verbindungen gemäß den Formeln (1), (2), (4) und (5), 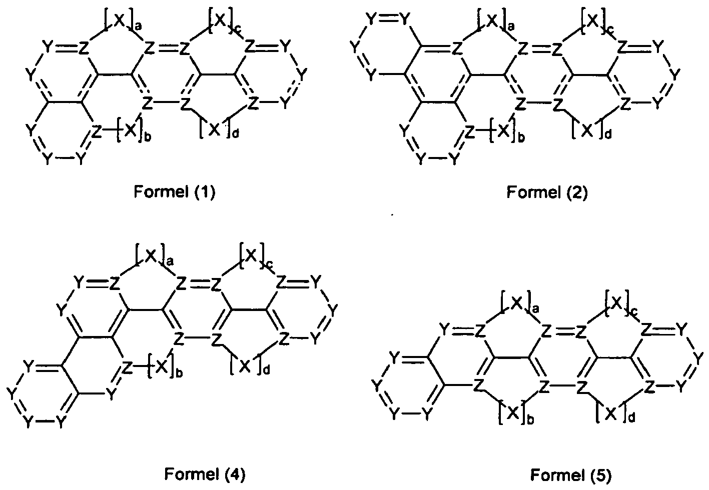 wobei für die Symbole und Indizes gilt:
Y ist gleich CR¹;
Z ist gleich C, falls an die Gruppe Z eine Brücke X gebunden ist, und ist gleich CR¹, falls an die Gruppe Z keine Brücke X gebunden ist;
X ist bei jedem Auftreten gleich oder verschieden eine bivalente Brücke, ausgewählt aus C(R¹)₂, C=O, C=NR¹, O, S, S=O, SO₂ oder N(R¹);
R¹ ist bei jedem Auftreten gleich oder verschieden H, F, Br, C(=O)Ar, P(=O)Ar₂, CR²=C²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²-, -C=C- oder -O- ersetzt sein können und wobei ein oder mehrere H-Atome durch F ersetzt sein können, oder eine Triarylamingruppe mit 18 bis 30 C-Atomen, die mit einem oder mehreren Resten R² substituiert sein kann, oder eine Arylgruppe mit 6 bis 16 C-Atomen oder Heteroarylgruppe mit 2 bis 16 C-Atomen oder eine Soirobiftuorengruppe; die jeweils mit einem oder mehreren Resten R² substituiert sein kann, oder eine Kombination aus zwei oder drei dieser Systeme, und/oder mindestens ein Symbol R¹ steht für eine Gruppe N(Ar)₂ der Formel (29) oder (30), 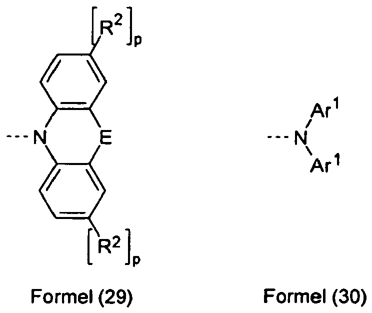 wobei R² die in Anspruch 1 aufgeführte Bedeutung hat und weiterhin gilt:
E steht für eine Einfachbindung, O, S, N(R²) oder C(R²)₂;
Ar¹ ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 30 aromatischen Ringatomen, welche jeweils mit einem oder mehreren Resten R² substituiert sein kann;
p ist bei jedem Auftreten gleich oder verschieden 0 oder 1; ,
Ar ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können auch zwei Reste Ar, welche an dasselbe Stickstoff- oder Phosphoratom binden, durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verknüpft sein;
R² ist bei jedem Auftreten gleich oder verschieden H oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches aliphatisches oder aromatisches Ringsystem bilden;
a, b, c, d ist bei jedem Auftreten gleich oder verschieden 0 oder 1, mit der Maßgabe, dass a + b = 1 und c + d = 1 ist, wobei a = 0 bzw. b = 0 bzw. c = 0 bzw. d = 0 jeweils bedeutet, dass die entsprechende Brücke X nicht vorhanden ist.

2. Verbindungen nach Anspruch 1, gewählt aus den Formeln (7) bis (14) und (19) bis (26), 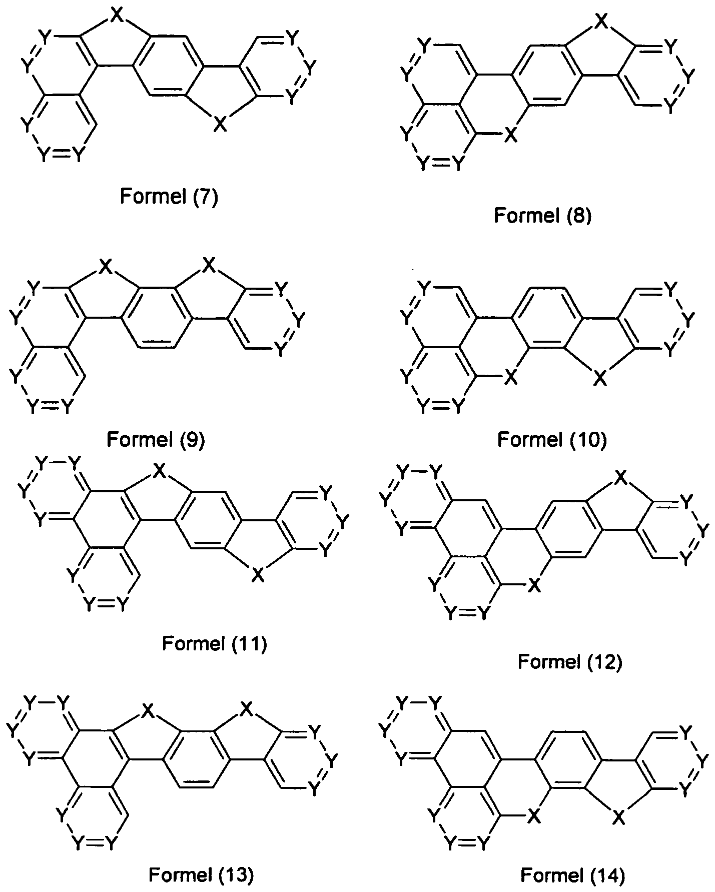 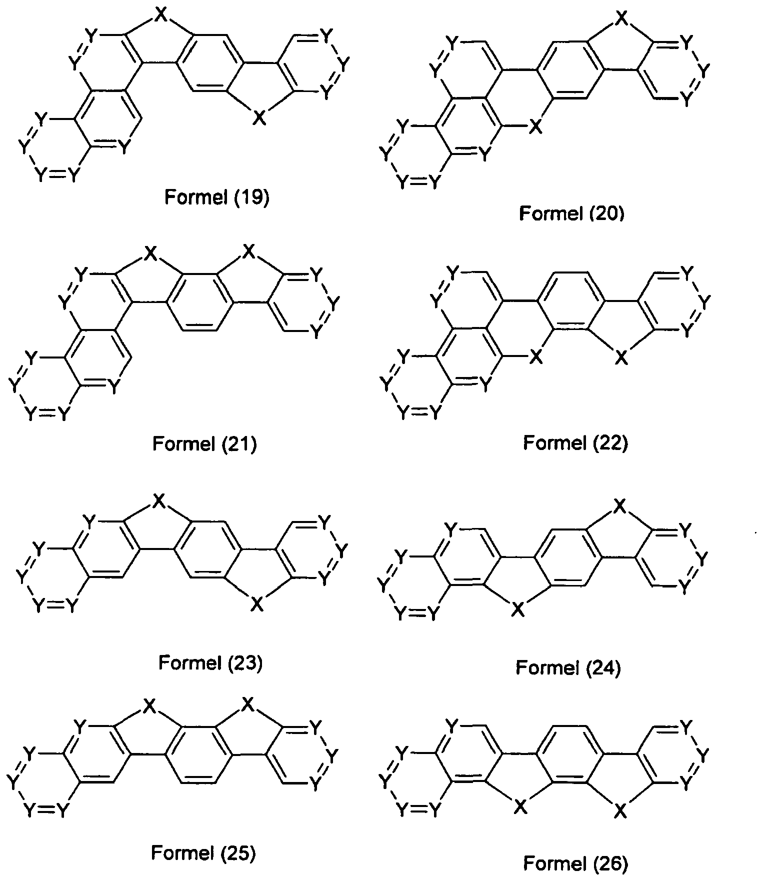 wobei die Symbole X und Y dieselbe Bedeutung haben, wie in Anspruch 1 beschrieben.

3. Verbindungen nach Anspruch 1 und/oder 2, gewählt aus den Formeln (7a) bis (14a) und (19a) bis (26a), 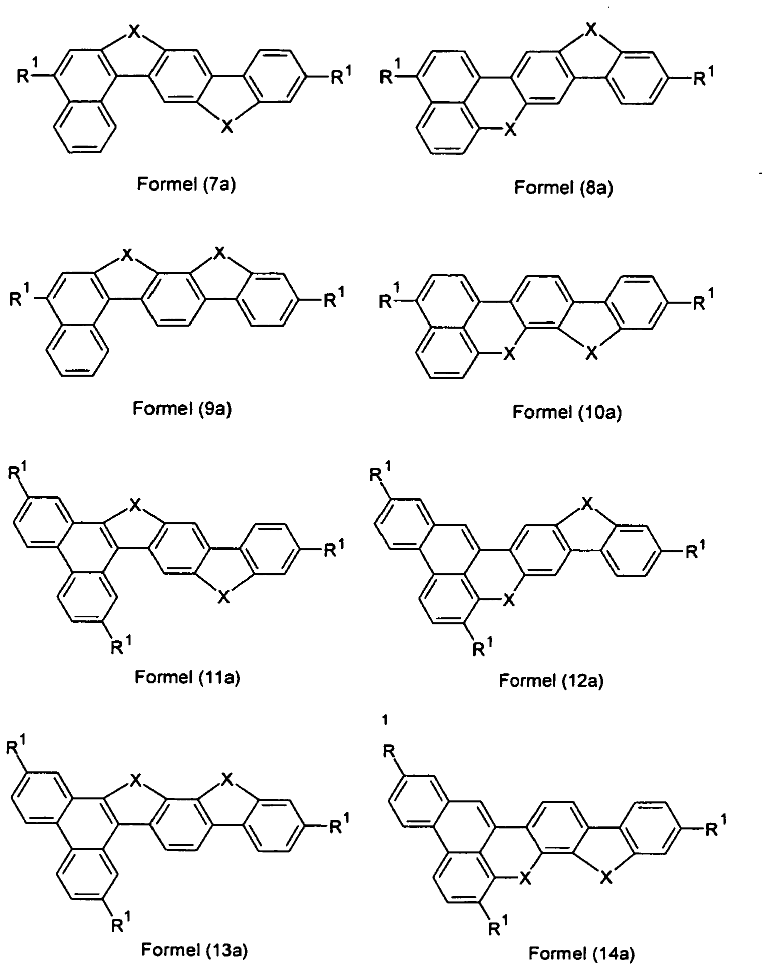 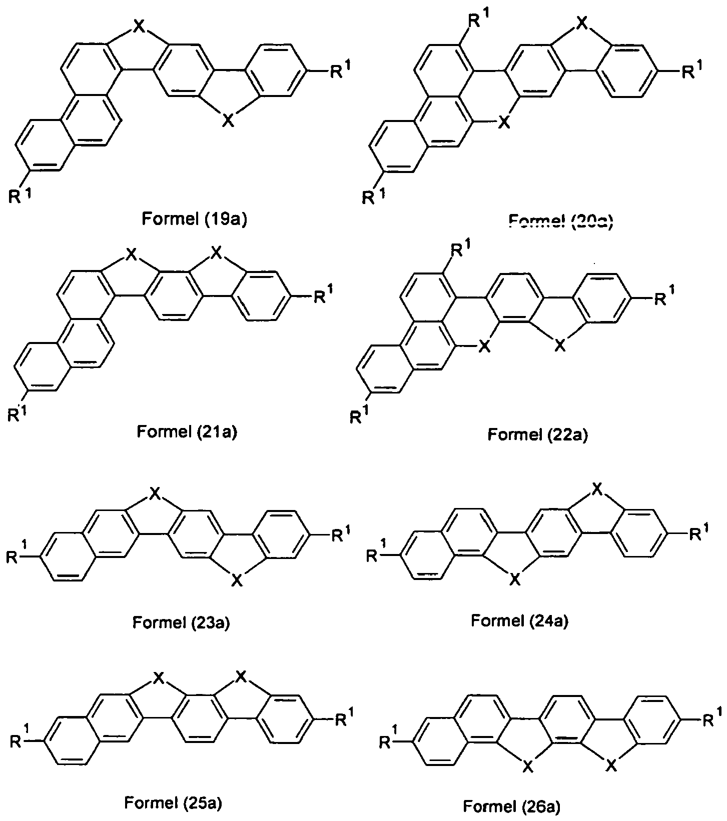 wobei die Symbole X und R¹ dieselbe Bedeutung haben, wie in Anspruch 1 beschrieben.

4. Verbindungen nach einem oder mehreren der Ansprüche 1 bis 3, **dadurch gekennzeichnet, dass** Reste R¹, die an die Brücken X gebunden sind, gleich oder verschieden sind und ausgewählt sind aus H, geradkettigen Alkylgruppen mit 1 bis 5 C-Atomen oder verzweigten Alkylgruppen mit 3 bis 5 C-Atomen, wobei eine oder mehrere nicht benachbarte CH₂-Gruppen durch -R²C=CR²-, -C=C- oder -O- ersetzt sein können und wobei ein oder mehrere H-Atome durch F ersetzt sein können, oder Arylgruppen mit 6 bis 16 C-Atomen oder Heteroarylgruppen mit 2 bis 16 C-Atomen, die jeweils mit einem oder mehreren Resten R² substituiert sein können, oder eine Kombination aus zwei oder drei dieser Systeme; dabei können zwei der Reste R¹, die an dasselbe Brückenatom gebunden sind, auch miteinander ein Ringsystem bilden: oder dass die Reste R¹ Alkylgruppen mit bis zu 10 C-Atomen darstellen, wenn die Verbindung gemäß Formel (1), (2), (4) oder (5) als Lösung verarbeitet wird.

5. Verfahren zur Herstellung von Verbindungen nach einem oder mehreren der Ansprüche 1 bis 4 durch Kupplung eines Fluorens, welches eine reaktive Gruppe trägt, mit einem Carbonyl-funktionalisierten Naphthalin oder Phenanthren, welches jeweils eine reaktive Gruppe trägt, gefolgt von Addition eines Alkyl- oder Arylmetallreagenzes und säurekatalysierter Cyclisierungsreaktion.

6. Verfahren zur Herstellung funktionalisierter Verbindungen nach einem oder mehreren der Ansprüche 1 bis 4 durch Alkylierung oder Acylierung der entsprechenden nicht-funktionalisierten Verbindung oder durch Halogenierung der nicht-funktionalisierten Verbindung, gefolgt von Kupplung mit einem reaktiven Aromaten oder mit einem mono- oder disubstituierten Amin, oder durch Halogenierung der nicht-funktionalisierten Verbindung, gefolgt von Metallierung und Reaktion mit einem Elektrophil.

7. Verwendung von Verbindungen nach einem oder mehreren der Ansprüche 1 bis 4 in elektronischen Vorrichtungen.

8. Vorrichtung, ausgewählt aus organischen Elektrolumineszenzvorrichtungen (OLED, PLED), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) und organischen Photorezeptoren, enthaltend Anode, Kathode und mindestens eine organische Schicht, enthaltend mindestens eine Verbindung nach einem oder mehreren der Ansprüche 1 bis 4.

9. Organische Elektrolumineszenzvorrichtung nach Anspruch 8, enthaltend Kathode, Anode, eine oder mehrere emittierende Schichten und gegebenenfalls weitere Schichten, gewählt aus jeweils einer oder mehreren Lochinjektionsschichten, Lochtransportschichten, Elektronentransportschichten, Elektroneninjektionsschichten und/oder Charge-Generation Layers.

10. Organische Elektrolumineszenzvorrichtung nach Anspruch 8 und/oder 9, **dadurch gekennzeichnet, dass** ein oder mehrere Substituenten R¹ aus einfachen oder kondensierten Aryl- oder Heteroarylgruppen gewählt sind und dass die Verbindung nach einem oder mehreren der Ansprüche 1 bis 4 als Host für einen fluoreszierenden Dotanden eingesetzt wird und/oder dass ein oder mehrere Substituenten R¹ und/oder Brücken X mindestens eine Gruppe C=O und/oder SO₂ enthalten und/oder ein oder mehrere Substituenten R¹ mindestens eine Gruppe P(=O) enthalten und dass die Verbindung nach einem oder mehreren der Ansprüche 1 bis 4 als Matrix für phosphoreszierende Dotanden eingesetzt wird und/oder dass ein oder mehrere Substituenten R¹ mindestens eine Vinylaryl-Einheit, mindestens eine Vinylarylamin-Einheit und/oder mindestens eine Arylamino-Einheit enthalten und die Verbindungen nach einem oder mehreren der Ansprüche 1 bis 4 als emittierende Materialien eingesetzt werden und/oder dass ein oder mehrere Substituenten R¹ für eine Gruppe N(Ar)₂ stehen und dass die Verbindung nach einem oder mehreren der Ansprüche 1 bis 4 gegebenenfalls mit Elektronenakzeptor-Verbindungen dotiert sein kann und dass sie als Lochtransportmaterial oder als Lochinjektionsmaterial eingesetzt wird, bevorzugt in einer Lochtransport- oder in einer Lochinjektionsschicht, und/oder dass ein oder mehrere Substituenten R¹ mindestens eine Einheit C=O, P(=O) und/oder SO₂ enthalten und dass die Verbindungen nach einem oder mehreren der Ansprüche 1 bis 4 gegebenenfalls mit einer Elektronendonator-Verbindung dotiert sein kann und dass sie als Elektronentransportmaterial eingesetzt wird.

## Claims

1. Compounds of the formulae (1), (2), (4) and (5), 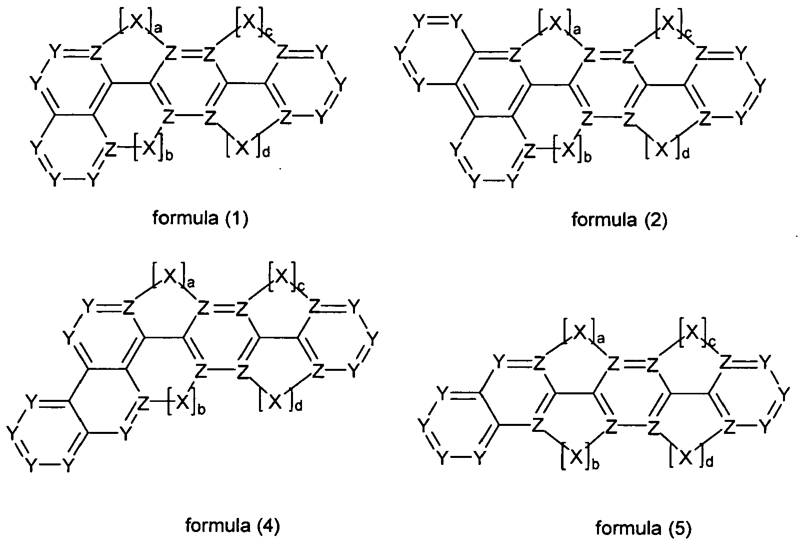 where the following applies to the symbols and indices:
Y is equal to CR¹;
Z is equal to C if a bridge X is bonded to the group Z and is equal to CR¹ if no bridge X is bonded to the group Z;
X is on each occurrence, identically or differently, a divalent bridge selected from C(R¹)₂, C=O, C=NR¹, O**,** S, S=O, SO₂ or N(R');
R¹ is on each occurrence, identically or differently, H, F, Br, C(=O)Ar, P(=O)Ar₂, CR²=CR²Ar, a straight-chain alkyl group having 1 to 5 C atoms or a branched alkyl group having 3 to 5 C atoms, where one or more non-adjacent CH₂ groups may be replaced by -R²C=CR²-, -C=C- or -O- and where one or more H atoms may be replaced by F, or a triarylamine group having 18 to 30 C atoms, which may be substituted by one or more radicals R², or an aryl group having 6 to 16 C atoms or a heteroaryl group having 2 to 16 C atoms or a spirobifluorene group, each of which may be substituted by one or more radicals R², or a combination of two or three of these systems, and/or at least one symbol R¹ stands for a group N(Ar)₂ of the formula (29) or (30), 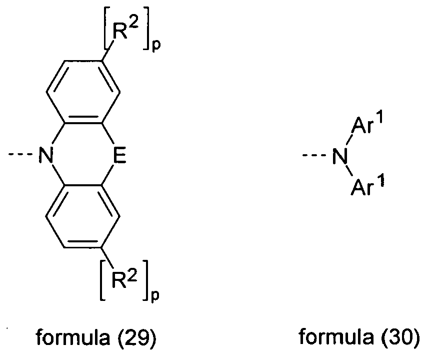 where R² has the meaning shown in Claim 1, and furthermore:
E stands for a single bond, O, S, N(R²) or C(R²)₂;
Ar¹ is, identically or differently on each occurrence, an aryl or heteroaryl group having 5 to 20 aromatic ring atoms or a triarylamine group having 15 to 30 aromatic ring atoms, each of which may be substituted by one or more radicals R², preferably an aryl or heteroaryl group having 6 to 14 aromatic ring atoms or a triarylamine groups having 18 to 30 aromatic ring atoms, each of which may be substituted by one or more radicals R²;
p is on each occurrence, identically or differently, 0 or 1;
Ar is on each occurrence, identically or differently, an aromatic or heteroaromatic ring system having 5 to 30 aromatic ring atoms, which may be substituted by one or more non-aromatic radicals R¹; two radicals Ar which are bonded to the same nitrogen or phosphorus atom may also be linked to one another here by a single bond or a bridge selected from B(R²), C(R²)₂, Si(R²)₂, C=O, C=NR², C=C(R²)₂, O, S, S=O, SO₂, N(R²), P(R²) and P(=O)R²;
R² is on each occurrence, identically or differently, H or an aliphatic, aromatic and/or heteroaromatic hydrocarbon radical having 1 to 20 C atoms, in which, in addition, H atoms may be replaced by F; two or more adjacent substituents R² here may also form a mono- or polycyclic aliphatic or aromatic ring system with one another;
a, b, c, d are on each occurrence, identically or differently, 0 or 1, with the proviso that a + b = 1 and c + d = 1, where a = 0 or b = 0 or c = 0 or d = 0 in each case means that the corresponding bridge X is not present.

2. Compounds according to Claim 1, selected from the formulae (7) to (14) and (19) to (26), 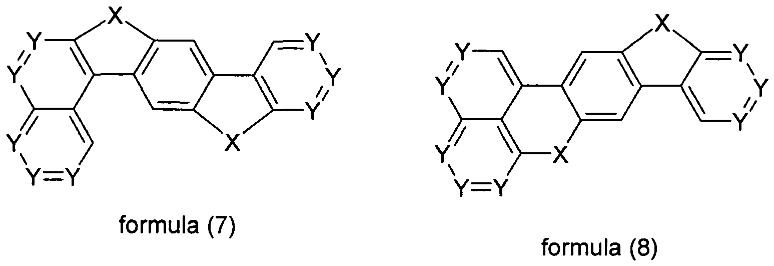 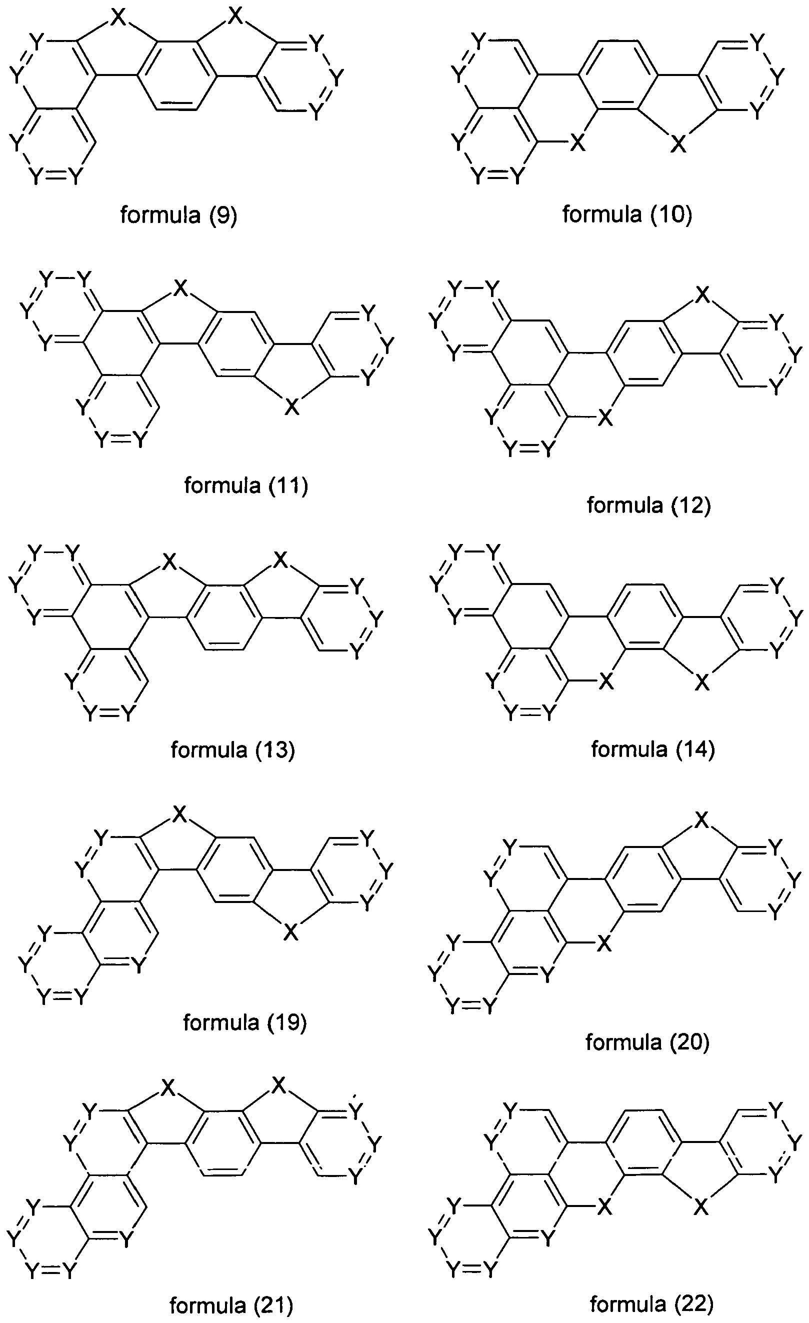 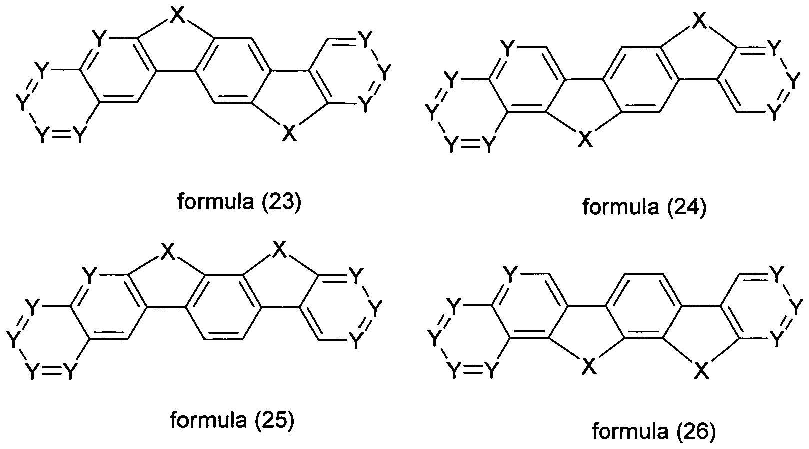 where the symbols X and Y have the same meaning as described in Claim 1.

3. Compounds according to Claim 1 and/or 2, selected from the formulae (7a) to (14a) and (19a) to (26a), 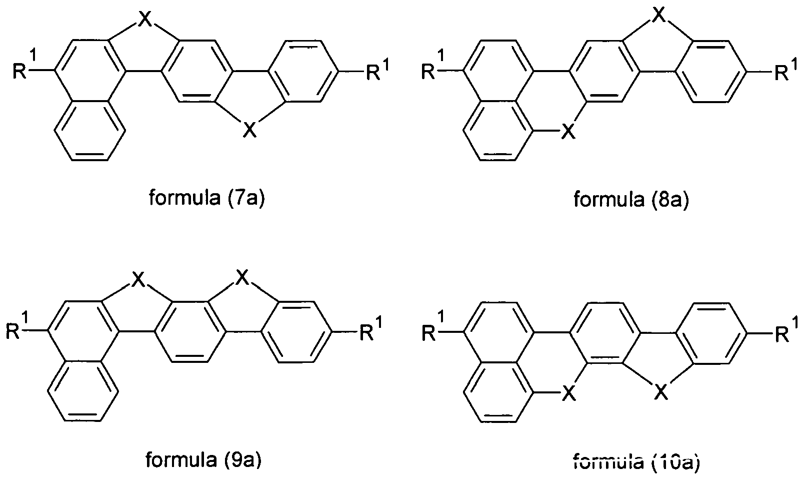 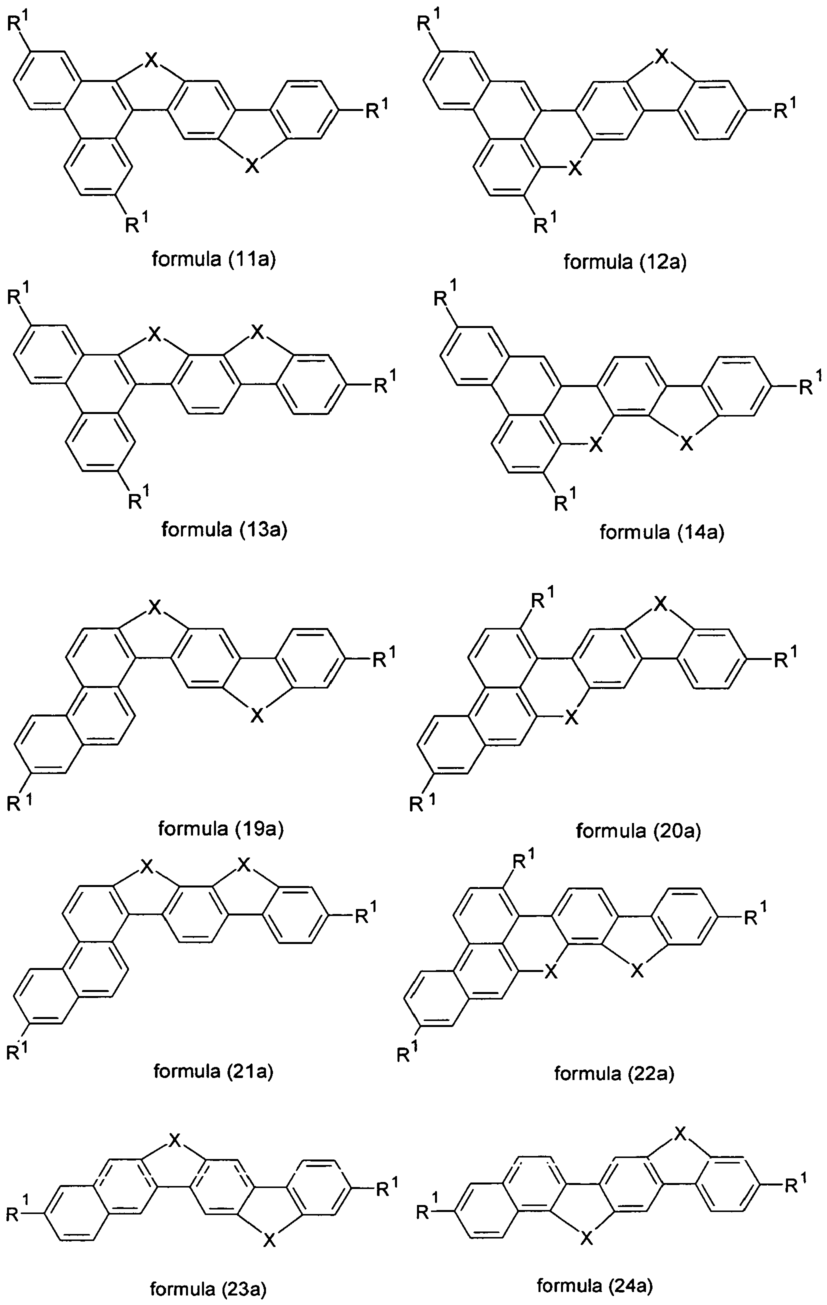 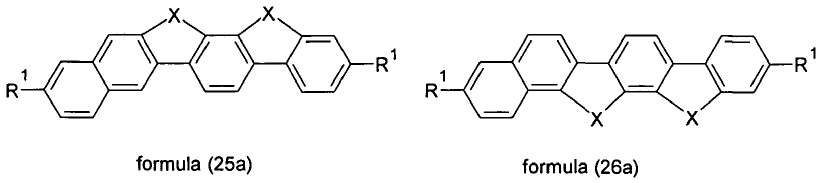 where the symbols X and R¹ have the same meaning as described in Claim 1.

4. Compounds according to one or more of Claims 1 to 3, **characterised in that** radicals R¹ which are bonded to the bridges X are identical or different and are selected from H, straight-chain alkyl groups having 1 to 5 C atoms or branched alkyl groups having 3 to 5 C atoms, where one or more non-adjacent CH₂ groups may be replaced by -R²C=CR²-, -C=C- or -O- and where one or more H atoms may be replaced by F, or aryl groups having 6 to 16 C atoms or heteroaryl groups having 2 to 16 C atoms, each of which may be substituted by one or more radicals R², or a combination of two or three of these systems; two of the radicals R¹ which are bonded to the same bridge atom may also form a ring system with one another here; or **in that** the radicals R¹ represent alkyl groups having up to 10 C atoms if the compound of the formula (1), (2), (4) or (5) is processed as a solution.

5. Process for the preparation of compounds according to one or more of Claims 1 to 4 by coupling a fluorene carrying a reactive group to a carbonyl-functionalised naphthalene or phenanthrene, each carrying a reactive group, followed by the addition of an alkyl- or arylmetal reagent and an acid-catalysed cyclisation reaction.

6. Process for the preparation of functionalised compounds according to one or more of Claims 1 to 4 by alkylation or acylation of the corresponding unfunctionalised compound or by halogenation of the unfunctionalised compound, followed by coupling to a reactive aromatic compound or to a mono- or disubstituted amine, or by halogenation of the unfunctionalised compound, followed by metallation and reaction with an electrophile.

7. Use of compounds according to one or more of Claims 1 to 4 in electronic devices.

8. Device, selected from organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) and organic photoreceptors, comprising anode, cathode and at least one organic layer comprising at least one compound according to one or more of Claims 1 to 4.

9. Organic electroluminescent device according to Claim 8, comprising cathode, anode, one or more emitting layers and optionally further layers selected from in each case one or more hole-injection layers, hole-transport layers, electron-transport layers, electron-injection layers and/or charge-generation layers.

10. Organic electroluminescent device according to Claim 8 and/or 9, **characterised in that** one or more substituents R¹ are selected from simple or condensed aryl or heteroaryl groups and **in that** the compound according to one or more of Claims 1 to 4 is employed as host for a fluorescent dopant and/or **in that** one or more substituents R¹ and/or bridges X contain at least one group C=O and/or SO₂ and/or one or more substituents R1 contain at least one group P(=O) and **in that** the compound according to one or more of Claims 1 to 4 is employed as matrix for phosphorescent dopants and/or **in that** one or more substituents R¹ contain at least one vinylaryl unit, at least one vinylarylamine unit and/or at least one arylamino unit and the compound according to one or more of Claims 1 to 4 is employed as emitting material and/or **in that** one or more substituents R¹ stand for a group N(Ar)₂ and **in that** the compound according to one or more of Claims 1 to 4 may optionally be doped with electron-acceptor compounds and **in that** it is employed as hole-transport material or hole-injection material, preferably in a hole-transport or hole-injection layer, and/or **in that** one or more substituents R¹ contain at least one unit C=O, P(=O) and/or SO₂ and **in that** the compound according to one or more of Claims 1 to 4 may optionally be doped with an electron-donor compound and **in that** it is employed as electron-transport material.

## Revendications

1. Composés des formules (1), (2), (4) et (5), 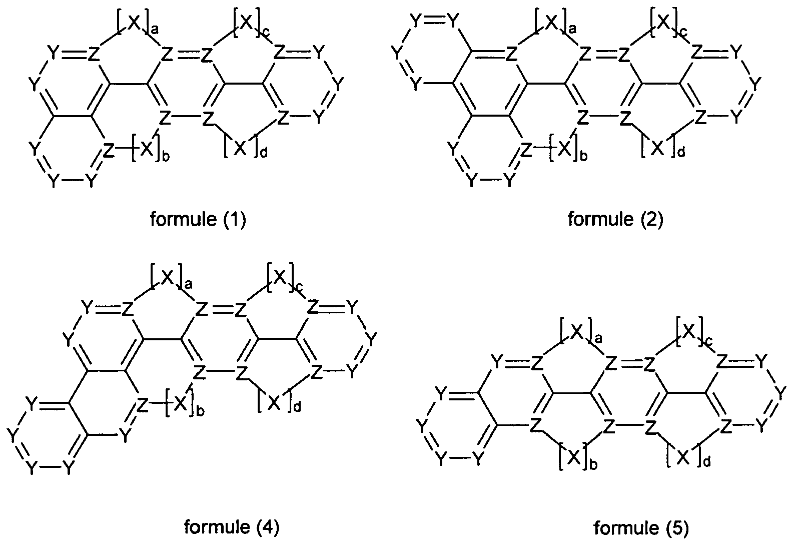 dans lesquelles ce qui suit s'applique aux symboles et indices :
Y est égal à CR¹ ;
Z est égal à C si un pont X est lié au groupe Z et est égal à CR¹ si aucun pont X n'est lié au groupe Z ;
X est, pour chaque occurrence, de manière identique ou différente, un pont divalent choisi parmi C(R¹)₂, C=O, C=NR¹, O, S, S=O, SO₂ ou N(R¹) ;
R¹ est, pour chaque occurrence, de manière identique ou différente, H, F, Br, C(=O)Ar, P(=O)Ar₂, CR²=CR²Ar, un groupe alkyle en chaîne droite comportant de 1 à 5 atomes de C ou un groupe alkyle ramifié comportant de 3 à 5 atomes de C, où un ou plusieurs groupes CH₂ non adjacents peut/peuvent être remplacé(s) par -R²C=CR²-, -C=C- ou -O- et où un ou plusieurs atomes de H peut/peuvent être remplacé(s) par F, ou un groupe triarylamine comportant de 18 à 30 atomes de C, lesquels peuvent être substitués par un ou plusieurs radicaux R², ou un groupe aryle comportant de 6 à 16 atomes de C ou un groupe hétéroaryle comportant de 2 à 16 atomes de C ou un groupe spirobifluorène, dont chacun peut être substitué par un ou plusieurs radicaux R², ou une combinaison de deux ou trois de ces systèmes, et/ou au moins un symbole R¹ représente un groupe N(Ar)₂ de la formule (29) ou (30), 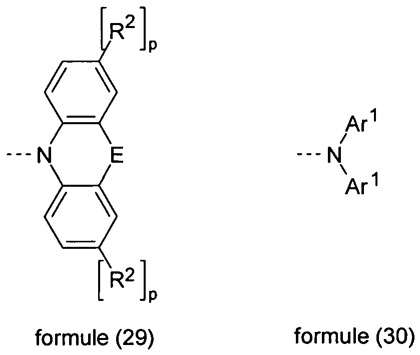 dans lesquelles R² présente la signification présentée selon la revendication 1, et en outre :
E représente une liaison simple, O, S, N(R²) ou C(R²)₂ ;
Ar¹ est, de manière identique ou différente pour chaque occurrence, un groupe aryle ou hétéroaryle comportant de 5 à 20 atomes de cycle aromatique ou un groupe triaryiamine comportant de 15 à 30 atomes de cycle aromatique, dont chacun peut être substitué par un ou plusieurs radicaux R², de préférence un groupe aryle ou hétéroaryle comportant de 6 à 14 atomes de cycle aromatique ou un groupe triarylamine comportant de 18 à 30 atomes de cycle aromatique, dont chacun peut être substitué par un ou plusieurs radicaux R² ;
p est, pour chaque occurrence, de manière identique ou différente, 0 ou 1 ;
Ar est, pour chaque occurrence, de manière identique ou différente, un système de cycle aromatique ou hétéroaromatique comportant de 5 à 30 atomes de cycle aromatique, lesquels peuvent être substitués par un ou plusieurs radicaux non aromatiques R¹ ; deux radicaux Ar qui sont liés au même atome d'azote ou de phosphore peuvent également être liés l'un à l'autre ici au moyen d'une liaison simple ou d'un pont choisi parmi B(R²), C(R²)₂, Si(R²)₂, C=O, C=NR², C=C(R²)₂, O, S, S=O, SO₂, N(R²), P(R²) et P(=O)R² ;
R² est, pour chaque occurrence, de manière identique ou différente, H ou un radical hydrocarbone aliphatique, aromatique et/ou hétéroaromatique comportant de 1 à 20 atomes de C, où, en outre, des atomes de H peuvent être remplacés par F ; deux substituents R² adjacents ou plus peuvent ici également former un système de cycle aliphatique ou aromatique mono- ou polycyclique l'un avec l'autre/les uns avec les autres ;
a, b, c, d sont, pour chaque occurrence, de manière identique ou différente, 0 ou 1, étant entendu que a + b = 1 et c + d = 1, où a = 0 ou b = 0 ou c = 0 ou d = 0 dans chaque cas signifie que le pont correspondant X n'est pas présent.

2. Composés selon la revendication 1, choisis parmi les formules (7) à (14) et (19) à (26), 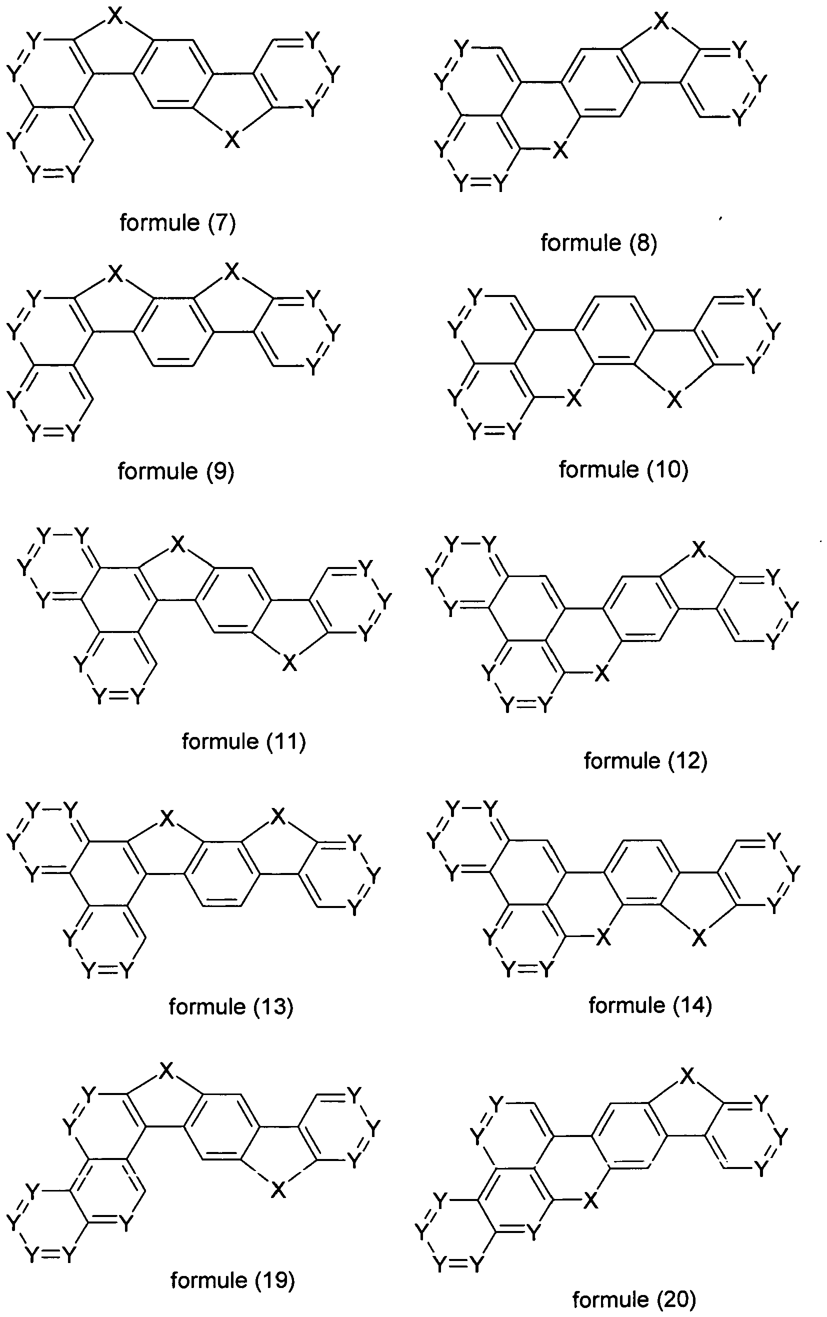 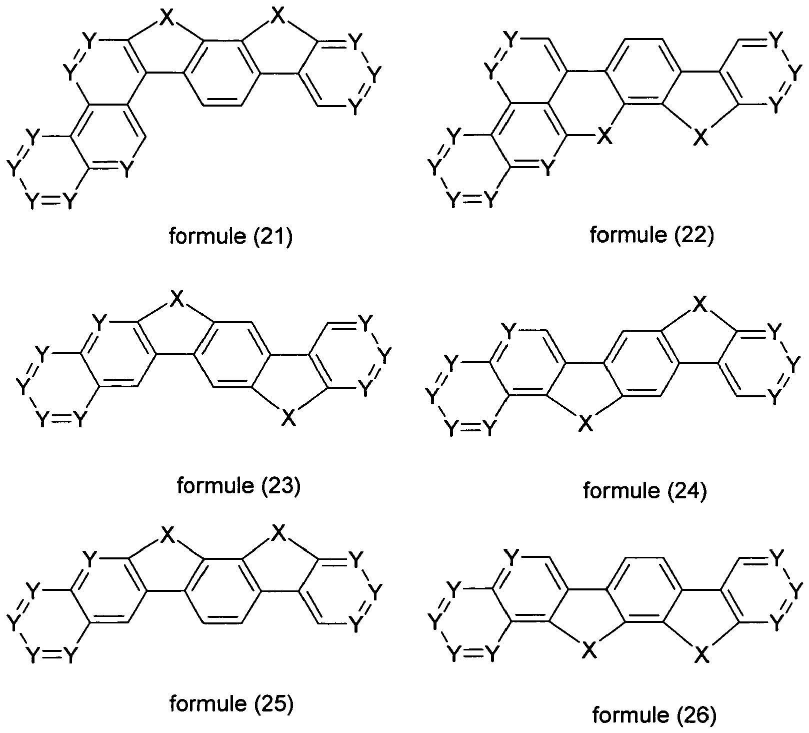 dans lesquelles les symboles X et Y présentent la même signification que décrit selon la revendication 1.

3. Composés selon la revendication 1 et/ou 2, choisis parmi les formules (7a) à (14a) et (19a) à (26a), 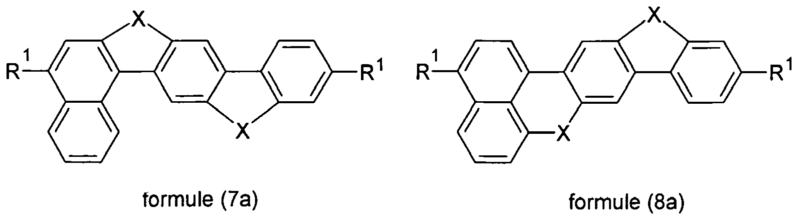 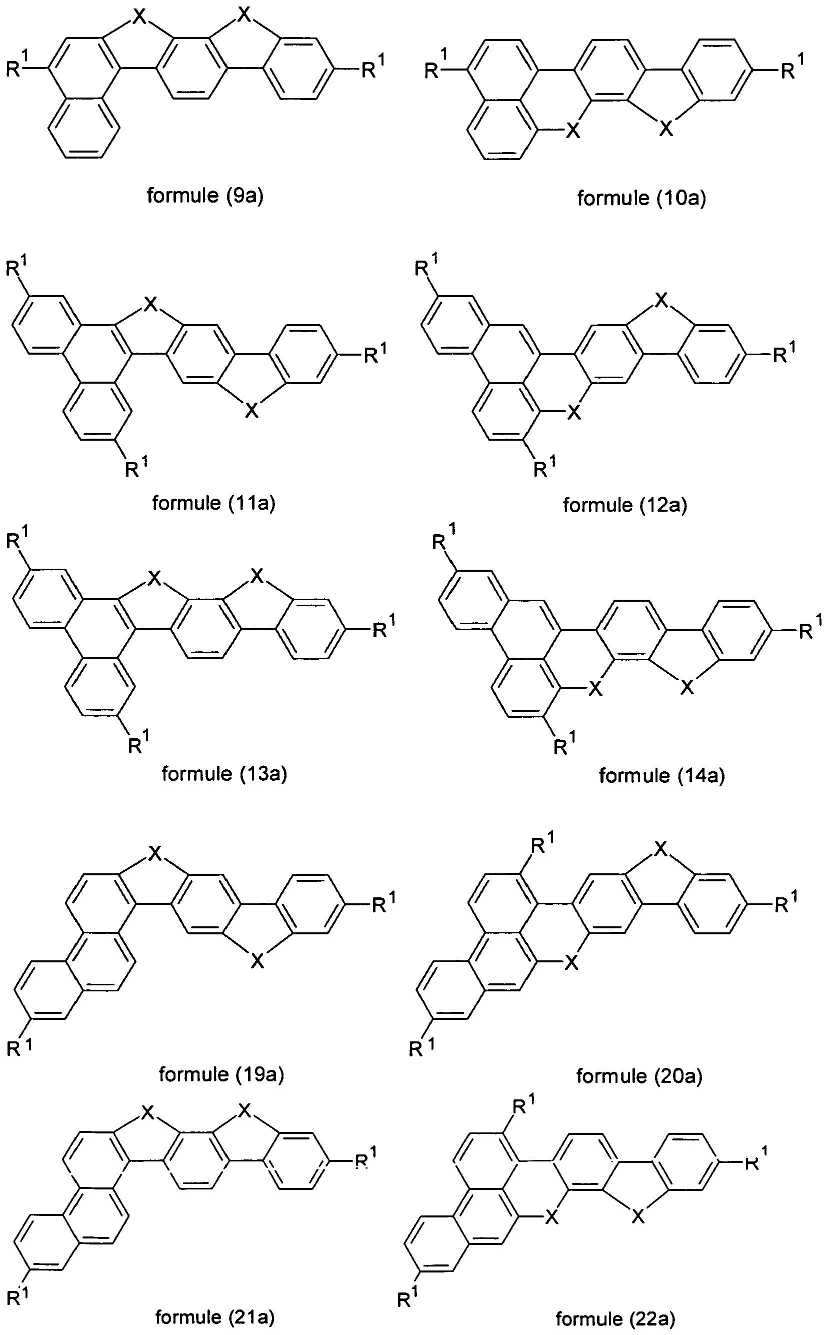 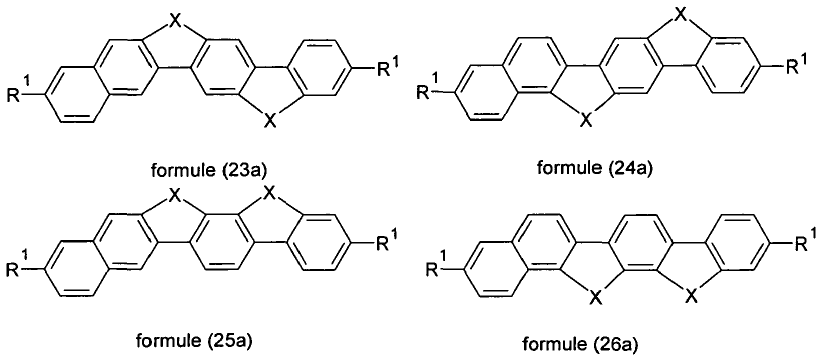 dans lesquelles les symboles X et R¹ présentent la même signification que décrit selon la revendication 1.

4. Composés selon une ou plusieurs des revendications 1 à 3, **caractérisés en ce que** des radicaux R¹ qui sont liés aux ponts X sont identiques ou différents et sont choisis parmi H, des groupes alkyle en chaîne droite comportant de 1 à 5 atomes de C ou des groupes alkyle ramifiés comportant de 3 à 5 atomes de C, où un ou plusieurs groupes CH₂ non adjacents peut/peuvent être remplacé(s) par -R²C=CR²-, -C=C- ou -O- et où un ou plusieurs atomes de H peut/peuvent être remplacé(s) par F, ou des groupes aryle comportant de 6 à 16 atomes de C ou des groupes hétéroaryle comportant de 2 à 16 atomes de C, dont chacun peut être substitué par un ou plusieurs radicaux R², ou une combinaison de deux ou trois de ces systèmes ; deux des radicaux R¹ qui sont liés au même atome de pont peuvent également former un système de cycle l'un avec l'autre ici ; ou **en ce que** les radicaux R¹ représentent des groupes alkyle comportant jusqu'à 10 atomes de C si le composé de la formule (1), (2), (4) ou (5) est traité en tant que solution.

5. Procédé pour la préparation de composés selon une ou plusieurs des revendications 1 à 4 en couplant un fluorène porteur d'un groupe réactif à un naphtalène ou phénanthrène fonctionnalisé carbonyle, chacun étant porteur d'un groupe réactif, ce couplage étant suivi par l'ajout d'un réactif alkylmétal ou arylmétal et par une réaction de cyclisation catalysée par acide.

6. Procédé pour la préparation de composés fonctionnalisés selon une ou plusieurs des revendications 1 à 4 par alkylation ou acylation du composé non fonctionnalisé correspondant ou par halogénisation du composé non fonctionnalisé, cette réaction étant suivie par un couplage sur un composé aromatique réactif ou sur un amine mono- ou disubstitué, ou par halogénisation du composé non fonctionnalisé, cette réaction étant suivie par une métallation et par une réaction avec un électrophile.

7. Utilisation de composés selon une ou plusieurs des revendications 1 à 4 dans des dispositifs électroniques.

8. Dispositif, choisi parmi des dispositifs électroluminescents organiques (OLED, PLED), des transistors à effet de champ organiques (O-FET), des transistors à film mince organiques (O-TFT), des transistors émetteurs de lumière organiques (O-LET), des circuits intégrés organiques (O-IC), des cellules solaires organiques (O-SC), des dispositifs à extinction de champ organiques (O-FQD), des cellules électrochimiques émettrices de lumière (LEC), des diodes laser organiques (O-laser) et des photorécepteurs organiques, comprenant une anode, une cathode et au moins une couche organique comprenant au moins un composé selon une ou plusieurs des revendications 1 à 4.

9. Dispositif électroluminescent organique selon la revendication 8, comprenant une cathode, une anode, une ou plusieurs couches émettrices et en option, d'autres couches choisies parmi, dans chaque cas, une ou plusieurs couches d'injection de trous, une ou plusieurs couches de transport de trous, une ou plusieurs couches de transport d'électrons, une ou plusieurs couches d'injection d'électrons et/ou une ou plusieurs couches de génération de charges.

10. Dispositif électroluminescent organique selon la revendication 8 et/ou 9, **caractérisé en ce qu'**un ou plusieurs substituents R¹ est/sont choisi(s) parmi des groupes aryle ou hétéroaryle simples ou condensés et **en ce que** le composé selon une ou plusieurs des revendications 1 à 4 est utilisé en tant qu'hôte pour un dopant fluorescent et/ou **en ce qu'**un ou plusieurs substituents R¹ et/ou ponts X contient/contiennent au moins un groupe C=O et/ou SO₂ et/ou un ou plusieurs substituents R¹ contient/contiennent au moins un groupe P(=O) et **en ce que** le composé selon une ou plusieurs des revendications 1 à 4 est utilisé en tant que matrice pour des dopants phosphorescents et/ou **en ce qu'**un ou plusieurs substituents R¹ contient/contiennent au moins une unité de vinylaryle, au moins une unité de vinylarylamine et/ou au moins une unité d'arylamino et le composé selon une ou plusieurs des revendications 1 à 4 est utilisé en tant que matériau d'émission et/ou **en ce qu'**un ou plusieurs substituents R¹ représente/représentent un groupe N(Ar)₂ et **en ce que** le composé selon une ou plusieurs des revendications 1 à 4 peut en option être dopé avec des composés accepteurs d'électrons et **en ce qu'**il est utilisé en tant que matériau de transport de trous ou matériau d'injection de trous, de préférence dans une couche de transport de trous ou une couche d'injection de trous, et/ou **en ce qu'**un ou plusieurs substituents R¹ contient/contiennent au moins une unité C=O, P(=O) et/ou SO₂ et **en ce que** le composé selon une ou plusieurs des revendications 1 à 4 peut en option être dopé avec un composé donneur d'électrons et **en ce qu'**il est utilisé en tant que matériau de transport d'électrons.
